# EUROPEAN PATENT APPLICATION

(11) **EP 4 060 027 A1**
(43) Date of publication of application: **21.09.2022**
(21) Application number: 20888079.9
(22) Date of filing: 12.11.2020
(51) Int. Cl.: C12N 5/10

(54) **TMEM59 PROTEIN DIMER OR CHIMERIC EXPRESSION RECEPTOR IMPROVING T CELL FUNCTION**

(30) Priority: 13.11.2019 CN 201911107366
(71) Applicant: Shanghai Pharmaceuticals Holding Co., Ltd., Shanghai 201203 (CN)
(72) Inventor: LIAO, Xuemei, Shanghai 201203 (CN); HUANG, Jingwei, Shanghai 201203 (CN); CHEN, Zhuo, Shanghai 201203 (CN); LIU, Yang, Shanghai 201203 (CN); CHENG, Xiaocui, Shanghai 201203 (CN); XIE, Manman, Shanghai 201203 (CN); SUN, Rongfang, Shanghai 201203 (CN); LI, Yingying, Shanghai 201203 (CN); XIA, Guangxin, Shanghai 201203 (CN); KE, Ying, Shanghai 201203 (CN); LIU, Yanjun, Shanghai 201203 (CN)
(74) Representative: Böhm, Brigitte
(86) International application number: PCT/CN2020/128440
(87) International publication number: WO 2021/093818

(57) **Abstract**

The present invention relates to an immune cell, which contains a TMEM59 protein and/or a functional fragment thereof, a chimeric antigen receptor (CAR) and/or a coding element thereof, as well as a use of the immune cell in the preparation of a drug for treating tumors. Further provided are a method for promoting the proliferation of immune cells and a method for promoting the production of memory cells.

## Description

### Field of the Invention

The present application relates to the field of biomedicine, and specifically to a use of a chimeric antigen receptor T lymphocyte in the preparation of a composition for treating tumors and a method therefor.

### Background of the Invention

Malignant tumor is a major disease that seriously threatens human life. Currently, there are no effective cures for most malignant tumors, which have disease recurrence and high mortality rates, thus seriously threatening the health of patients and significantly reducing the quality of life.

The chimeric antigen receptor T lymphocyte therapy is an adoptive immunotherapy emerged in recent years. A single-chain antibody variable region derived from a monoclonal antibody targeting a tumor specific antigen, together with a co-stimulatory molecule (e.g., 4-1BB, CD28 protein, etc.) and an intracellular segment CD3zeta of a T lymphocyte signaling region, forms a chimeric antigen receptor protein, which is expressed across the cell membrane on the surface of the T lymphocyte. When the single-chain antibody variable region recognizes the specific antigen on the surface of tumor cells, it activates the co-stimulatory molecule and CD3zeta, transmits the activation signals of the T lymphocyte, directly activates the first and second signals on which the activation of T lymphocyte depends, promotes T lymphocyte activation, t lymphocyte proliferation and enhances its immune killing function so as to achieve the immunomodulation and killing on the tumor cells, thereby realizing the purpose of removing the tumor.

Increasing the survival time of effector T lymphocytes in a patient is an important approach to optimize the therapeutic efficacy and prolong the effective time of the chimeric antigen receptor T therapy. It is demonstrated from clinical trials that the long-term disease remission obtained from treatment with the chimeric antigen receptor T lymphocyte therapy is closely related to the long-term survival and persistence of chimeric antigen receptor T lymphocytes in the patient. Although an effector T lymphocyte has a potent ability to lyse tumors, its survival time in the patient is relatively short. However, memory T lymphocytes, including effector memory T lymphocytes and central memory T lymphocytes, are able to expand for a long time in vivo and possess a potent proliferative ability, at the same time, they have the potential to differentiate into effector T lymphocytes, so that they can provide effector T lymphocytes continuously.

Therefore, chimeric antigen receptor T lymphocytes with extended survival time are urgently needed to maintain their efficacy.

### Summary of the Invention

The present application provides an immune cell, the immune cell includes a TMEM59 protein and/or a functional fragment thereof and a chimeric antigen receptor (CAR) and/or a coding element thereof, as well as a use of the immune cell in the preparation of a drug for treating tumors. The present application further provides a method for promoting the proliferation of immune cells and a method for promoting the production of memory cells. The immune cell of the present application has one of the following beneficial effects: (1) capable of increasing the number of memory lymphocytes, for example, compared with CAR T cells not including the TMEM59 protein and/or the functional fragment thereof, by at least 50%, 60%, 70%, 80%, 90%, 100%, 150% or 200% above; (2) capable of enhancing the proliferative ability of lymphocytes, for example, compared with CAR T cells not including the TMEM59 protein and/or the functional fragment thereof, by at least 10%, 20%, 30%, 40% or 50% above; (3) enhancing the killing rate of lymphocytes against target cells, for example, compared with CAR T cells not including the TMEM59 protein and/or the functional fragment thereof, by at least 10%, 20%, 30% or higher; and, (4) inhibiting the growth of tumor cells, and/or improving the survival rate of subjects with tumors.

In one aspect, the present application provides an immune cell, which includes a TMEM59 protein and/or a functional fragment thereof located on the cell membrane, wherein the functional fragment includes at least a transmembrane region and a hinge region of the TMEM59 protein.

In some embodiments, the immune cell is modified to include the TMEM59 protein and/or the functional fragment thereof on the cell membrane.

In some embodiments, the TMEM59 protein and/or the functional fragment thereof is a human TMEM59 protein and/or a functional fragment thereof.

In some embodiments, the transmembrane region of the TMEM59 protein includes an amino acid sequence as set forth in SEQ ID NO. 1.

In some embodiments, the hinge region of the TMEM59 protein includes an amino acid sequence as set forth in SEQ ID NO. 2.

In some embodiments, the TMEM59 protein and/or the functional fragment thereof includes an amino acid sequence as set forth in SEQ ID NO. 3.

In some embodiments, the immune cell includes a chimeric antigen receptor (CAR) and/or a coding element thereof.

In some embodiments, the CAR includes a transmembrane region.

In some embodiments, the transmembrane region of the CAR is selected from: a transmembrane region of the TMEM59 protein and a transmembrane region of CD8.

In some embodiments, the transmembrane region of the CAR includes an amino acid sequence as set forth in any one of SEQ ID NOs. 1 and 37.

In some embodiments, the CAR includes a hinge region.

In some embodiments, the hinge region of the CAR is selected from: a hinge region of the TMEM59 protein and a hinge region of CD8.

In some embodiments, the hinge region of the CAR includes an amino acid sequence as set forth in any one of SEQ ID NOs. 2 and 38.

In some embodiments, the CAR includes an intracellular domain, the intracellular domain of the CAR includes a signaling domain and/or a costimulatory domain.

In some embodiments, the signaling domain includes a signaling domain of CD3zeta.

In some embodiments, the costimulatory domain includes a costimulatory domain of 4-1BB.

In some embodiments, the CAR includes a targeting moiety.

In some embodiments, the targeting moiety includes ScFv.

In some embodiments, the targeting moiety specifically binds to and/or recognizes a tumor antigen.

In some embodiments, the targeting moiety specifically binds to and/or recognizes a target selected from a group consisting of: CD19, CD22 and GPC3.

In some embodiments, the immune cell includes the CAR as well as the TMEM59 protein and/or the functional fragment thereof, and wherein the CAR does not include the TMEM59 protein and/or the functional fragment thereof.

In some embodiments, the CAR includes the targeting moiety, the hinge region of CD8, the transmembrane region of CD8, the signaling domain of CD3zeta and the costimulatory domain of 4-1BB, wherein the TMEM59 protein and/or the functional fragment thereof include the transmembrane region and the hinge region of the TMEM59 protein.

In some embodiments, the CAR includes an amino acid sequence as set forth in any one of SEQ ID NOs. 49, 51, 55 and 56.

In some embodiments, the immune cell includes the CAR as well as the TMEM59 protein and/or the functional fragment thereof, and wherein said CAR includes the TMEM59 protein and/or the functional fragment thereof.

In some embodiments, the CAR includes the targeting moiety, the hinge region of the TMEM59 protein, the transmembrane region of the TMEM59 protein, the signaling domain of CD3zeta and the costimulatory domain of 4-1BB, wherein said TMEM59 protein and/or the functional fragment thereof includes the transmembrane region and the hinge region of the TMEM59 protein.

In some embodiments, the CAR includes an amino acid sequence as set forth in any one of SEQ ID NOs. 43, 57 and 44.

In some embodiments, the immune cell includes a T lymphocyte.

In another aspect, the present application provides a pharmaceutical composition, which includes the immune cell and optionally a pharmaceutically acceptable carrier.

In another aspect, the present application provides a use of the immune cell or the pharmaceutical composition in the preparation of a drug for treating tumors.

In some embodiments, the tumor includes a solid tumor and/or a non-solid tumor.

In some embodiments, the tumor includes B lymphocytic leukemia and/or liver cancer.

In another aspect, the present application provides a method for promoting the proliferation of an immune cell, which includes: allowing the immune cell to express a TMEM59 protein and/or a functional fragment thereof on its cell membrane, wherein the functional fragment includes at least a transmembrane region and a hinge region of the TMEM59 protein.

In another aspect, the present application provides a method for promoting the production of a memory immune cell, which includes: allowing the immune cell to express a TMEM59 protein and/or a functional fragment thereof on its cell membrane, wherein the functional fragment includes at least a transmembrane region and a hinge region of the TMEM59 protein.

In another aspect, the present application provides a method for enhancing the killing ability of an immune cell against a tumor, which includes: allowing the immune cell to express a TMEM59 protein and/or a functional fragment thereof on its cell membrane, wherein the functional fragment includes at least a transmembrane region and a hinge region of the TMEM59 protein.

In some embodiments, the expression includes allowing the immune cell to include a nucleic acid encoding the TMEM59 protein and/or the functional fragment thereof.

In some embodiments, the expression includes transfecting the immune cell with a plasmid including a nucleic acid encoding the TMEM59 protein and/or the functional fragment thereof.

In some embodiments, the immune cell includes a T lymphocyte.

In some embodiments, the immune cell includes a nucleic acid encoding the TMEM59 protein and/or the functional fragment thereof.

In some embodiments, the nucleic acid is in a viral vector.

In another aspect, the present application provides a method for inhibiting the proliferation of tumor cells and/or killing tumor cells, which includes: contacting the immune cell with the tumor cells.

In some embodiments, the method includes an in vitro method or an ex vivo method.

In some embodiments, the tumor includes a solid tumor and/or a non-solid tumor.

In some embodiments, the tumor includes B lymphocytic leukemia and/or liver cancer.

Those skilled in the art can easily perceive other aspects and advantages of the present application from the detailed description below. In the following detailed description, only exemplary embodiments of the present application are shown and described. As those skilled in the art will recognize, the content of the present application enables those skilled in the art to make changes to the disclosed specific embodiments without departing from the spirit and scope of the invention involved in the present application. Correspondingly, the drawings and descriptions in the specification of the present application are merely exemplary, rather than restrictive.

### Brief Description of the Drawings

The specific features of the invention involved in the present application are shown in the appended claims. The characteristics and advantages of the invention involved in the present application can be better understood by referring to the exemplary embodiments and the accompanying drawings described in detail below. A brief description of the drawings is as follows:
Fig. 1 shows the number of memory cells in the H63 CAR-T lymphocyte;
Fig. 2 shows the number of memory cells in the H63 CAR-2A-TMEM59 T lymphocyte;
Fig. 3 shows the number of memory cells in the 21D4 CAR-2A-GFP T lymphocyte;
Fig. 4 shows the number of memory cells in the 21D4 CAR-2A-TMEM59 T lymphocyte;
Fig. 5 shows the proliferation number of the T lymphocyte of the present application;
Fig. 6 shows the killing rate of the T lymphocyte of the present application against a target cell;
Fig. 7 shows the trend of the biofluorescence intensity in tumor-bearing mice;
Fig. 8 shows the trend of the survival rate of tumor-bearing mice over time;
Fig. 9 shows an in vivo imaging map of the tumor-bearing mice.

### Detailed Description of the Embodiments

The implementation of the present application will be illustrated in the following specific examples, and other advantages and effects of the present application will be easily known by those familiar with this technology from the content disclosed in the specification.

In the present application, the term "chimeric antigen receptor (CAR)" may refer to, for example, an artificial T cell receptor, a chimeric T cell receptor, or a chimeric immune receptor, and includes a genetically engineered receptor obtained by implanting artificial specificity onto a particular immune effector cell. The CAR can be used to confer the specificity of a monoclonal antibody to T cells, thus allowing the production of a large number of specific T cells, for example, so as to be used in the adoptive cellular immunotherapy. In some cases, the CAR can direct the cellular specificity to tumor-associated antigens. The CAR can include an intracellular activation domain, a transmembrane region, and an extracellular domain including a tumor-associated antigen binding region. For example, the CAR can include a fusion of a single-chain variable fragment (scFv) derived from a monoclonal antibody fused to the transmembrane region and the intracellular domain of CD3zeta. In some other cases, the CAR can also include additional costimulatory signaling domains, such as CD3zeta, FcR, CD27, CD28, CD137, DAP10, and/or OX40. In some cases, the CAR can be co-expressed with a molecule, which may include a costimulatory molecule, a reporter gene for imaging (e.g., for positron emission tomography), a gene product that conditionally ablates T cells upon the addition of a prodrug, a homing receptor, a chemokine, a chemokine receptor, a cytokine, and a cytokine receptor.

In the present application, the term "antibody" generally refers to a protein or polypeptide sequence that specifically binds to an antigen, and that is derived from an immunoglobulin molecule. The antibody may be a polyclonal or monoclonal, a multi-chain or single-chain, or a complete immunoglobulin, and it can be derived from a natural source or a recombinant source. The antibody may be a tetramer of immunoglobulin molecules. The term "antibody fragment" generally refers to at least a part of a complete antibody or a recombinant variant thereof, and can also refer to an antigen-binding domain of a complete antibody, for example, an antigenic determining variable region, which is sufficient to confer recognition and specific binding of the antibody fragment to a target (e.g., an antigen). Examples of the antibody fragments include, but not limited to, antigen-binding fragments (Fab, Fab', F(ab)₂, Fv fragments, F(ab')₂, scFv, di-scFv and/or dAb), immunoconjugates, multi-specific antibodies (e.g., bispecific antibodies), antibody fragments, antibody derivatives, antibody analogues or fusion proteins, VHH domains, and multi-specific antibodies formed from the antibody fragments. The term "scFv" generally refers to a fusion protein including at least one antibody fragment containing a light chain variable region and at least one antibody fragment containing a heavy chain variable region, wherein the light chain variable region and the heavy chain variable region can be joined continuously by short flexible peptide linkers so as to be expressed as a single-chain polypeptide, and wherein the scFv maintains the specificity of the complete antibody from which it is derived. Unless otherwise specified, the scFv in the present application may have VL and VH variable regions in any order. For example, relative to the N-terminus and C-terminus of the polypeptide, scFv may include VL-linker-VH or may include VH-linker-VL. The part including an antibody or an antibody fragment thereof in the CAR of the present application may exist in various forms, including, e.g., single-domain antibody fragment (sdAb), single-chain antibody (scFv) and humanized antibody (Harlow et, al, 1999, In: Using Antibodies: A Laboratory Manual, Cold Spring Harbor Laboratory Press, NY; Harlow et, al, 1989, In: Antibodies: A Laboratory Manual, Cold Spring Harbor, New York; Houston et, al, 1988, Proc. Natl. Acad. Sci. USA 85:5879-5883; Bird et, al, 1988, Science 242:423-426), wherein the antigen-binding domain is expressed as a part of a continuous polypeptide chain. In some cases, the antigen-binding domain of CAR of the present application includes an antibody fragment. In some other cases, CAR may include an antibody fragment containing scFv.

In the present application, the term "scFv" generally refers to a fusion protein including at least one antibody fragment containing a light chain variable region and at least one antibody fragment containing a heavy chain variable region, wherein the light chain variable region and the heavy chain variable region are contiguous (for example, via synthetic linkers, e.g., short flexible polypeptide linkers), and can be expressed in a form of a single-chain polypeptide, and wherein the scFv maintains the specificity of the complete antibody from which it is derived. In the present application, the scFv may have the VL and VH variable regions in any order (for example, relative to the N-terminus and C-terminus of the polypeptide). The scFv molecule has a general structure of NH₂-VL-linker-VH-COOH or NH₂-VH-linker-VL-COOH.

In the present application, the term "TMEM59" generally refers to the transmembrane protein 59, its functional variant and/or its functional fragment. The sequence of TMEM59 is known in the art. For example, the nucleotide sequence encoding the human TMEM59 protein and/or functional fragment thereof of the present application may include the nucleotide sequence of SEQ ID NO. 4. The human TMEM59 protein and/or functional fragment thereof of the present application may include the amino acid sequence of SEQ ID NO. 3.

In the present application, the term "co-stimulatory molecules" generally refers to correlation binding partner on the T cells, which specifically bind to co-stimulatory ligands, thereby mediating the co-stimulatory response of T cells, for example, but not limited to, proliferation. The co-stimulatory molecules are cell surface molecules or their ligands that are required for an effective immune response and are not antigenic receptors. The co-stimulatory molecules include, but not limited to, MHC class I molecules, BTLA and Toll-ligand receptors, as well as OX40, CD2, CD27, CD28, CDS, ICAM-1, LFA-I (CD18) and 4-1BB (CD137).

In the present application, the term "costimulatory domain" generally refers to any sequences of 4-1BB (CD137), which include, for example, stimulatory signaling domains of 4-1BB. They may include homologues, variants, isomers or functional fragments of 4-1BB.

In the present application, the term "signaling domain" refers to functional parts of a protein. The signaling domains can produce signals that promote the immune effector functions of CAR-containing cells, e.g., CAR-expressing cells, such as T cells or NK cells. For example, in CAR-expressing cells, examples of immune effector functions may include cytolytic activity and auxiliary activity, where the latter may include the secretion of cytokines. In some cases, signaling domains transduce effector functional signals and direct cells to perform specialized functions. Although the whole intracellular signaling domain can be used, in many instances, it is not necessary to use the intact chain of the whole domain. To the extent of using the truncated portions of the intracellular signaling domain, such truncated portions can be used in place of the intact chains as long as they transduce the effector functional signals. Therefore, the term "signaling domain" may include any truncated portions of the signaling domain that are sufficient to transduce the required functional signals.

In the present application, the term "transmembrane region" generally refers to a domain of a peptide, a polypeptide or a protein capable of spanning the cytoplasmic membrane. These domains can be used to anchor an antibody or its fragments on the cell membrane.

In the present application, the term "encoding" generally refers to the inherent property of a particular sequence of nucleotides in a polynucleotide such as a gene, cDNA or mRNA to act as a template for the synthesis of other multimers and macromolecules in a biological process, said multimers and macromolecules having either a defined sequence of nucleotides (i.e., rRNA, tRNA and mRNA) or a defined sequence of amino acids and the biological properties arising therefrom. Thus, if transcription and translation of an mRNA corresponding to a gene produces a protein in a cell or other biological system, the gene encodes the protein. Both the coding strand whose nucleotide sequence is identical to the mRNA sequence and is usually provided in the sequence listing, and the non-coding strand used as a template for the transcription of a gene or cDNA may be referred to as the protein or other product encoding the gene or cDNA. In the present application, the term "coding element" generally refers to a nucleic acid (an RNA or DNA molecule) including a nucleotide sequence encoding a protein (e.g., a chimeric antigen receptor (CAR)).

In the present application, the term "tumor antigen" generally refers to any molecules expressed on tumor cells (or related to the development of tumor cells) that are known or believed to have a role in the tumorigenic properties of tumor cells. These antigens generally have extracellular parts which can be present on the cell surface, and also have transmembrane parts and cytoplasmic parts that are joined together with the extracellular parts. These antigens can sometimes be present only on the surface of tumor cells but not on the surface of normal cells. Tumor antigens can be expressed specifically on tumor cells, or have tumor-specific mutations compared to normal cells. In such cases, they are referred to as tumor-specific antigens (TSAs). TSAs are unique to tumor cells, and do not occur on other cells of the body. Tumor antigens can be expressed not only in tumor cells, and can also be expressed on normal cells in conditions that cannot induce an immune tolerance state to the antigens, where they are referred to as tumor-associated antigens (TAAs). Compared to normal cells, TAAs can be overexpressed on tumor cells, or are prone to bind to antibodies in tumor cells due to the less compact structure of tumor tissues compared to normal tissues.

In the present application, the term "specifically bind to" generally refers to an antibody or ligand that recognizes and binds to a homologous binding ligand protein present in a sample, but substantially does not recognize and bind to other molecules in the sample.

In the present application, the term "CD19" generally refers to Cluster of Differentiation 19 protein, an antigenic determinant that can be detected in precursor cells of leukemia. The amino acid and nucleic acid sequences of human and mouse CD19 can be found in public databases, e.g., GenBank, UniProt and Swiss-Prot. For example, the amino acid sequence of human CD19 can be found in UniProt/Swiss-Prot Accession No. P15391, and the nucleotide sequence encoding human CD19 can be found in NCBI Accession No. NM_001178098. CD19 is expressed on most B-lineage cancers, which is also an early marker of B-cell progenitor cells. In the present application, "CD19" may include proteins containing mutations, for example, point mutations, fragments, insertions, deletions and spliced variants of full-length wild-type CD19.

In the present application, the term "does not include" generally refers to the exclusion of the possibility of a certain behavior, structure or structure. For example, "A does not include B" generally means to exclude the possibility of B occurring in A.

In the present application, the terms "peptide", "polypeptide" and "protein" can be used interchangeably and generally refer to compounds composed of amino acid residues covalently linked by peptide bonds. The protein or peptide must contain at least two amino acids, and there is no limitation on the maximum number of amino acids that can be included in the protein or peptide sequence. The polypeptide may include any peptides or proteins that contain two or more amino acids linked to each other through peptide bonds. In the present application, this term refers to two short chains, which are also commonly known as peptides, oligopeptides and oligomers in the art, for example longs chains, which are commonly known as proteins in the art, of which there are many types. "Polypeptides" include, for example, bioactive fragments, substantially homologous polypeptides, oligopeptides, homodimers, heterodimers, variants of polypeptides, modified polypeptides, derivatives, analogues, fusion proteins, etc. Polypeptides include native peptides, recombinant peptides or combinations thereof.

In addition to particular proteins and nucleotides mentioned herein, the present application may also include their functional variants, derivatives, analogues, homologues and fragments thereof.

The term "functional variant" refers to a polypeptide having substantially the same amino acid sequence or encoded by substantially the same nucleotide sequence as the naturally occurring sequence and capable of having one or more activities of the naturally occurring sequence. In the context of the present application, the variant of any given sequence refers to a sequence in which a particular sequence of residues (either amino acid or nucleotide residues) has been modified so that the polypeptide or polynucleotide remains substantially at least one endogenous function. The variant sequences can be obtained through the addition, deletion, substitution, modification, replacement and/or variation of at least one amino acid residue and/or nucleotide residue present in a naturally occurring protein and/or polynucleotide, as long as the original functional activity is maintained. In the present application, the term "derivative" generally refers to a polypeptide or polynucleotide of the present application including any substitution, variation, modification, replacement, deletion and/or addition from/to one (or more) amino acid residues of the sequence, provided that the resulting polypeptide or polynucleotide substantially maintains at least one of its endogenous functions.

In the present application, the term "analogue" generally, with respect to a polypeptide or polynucleotide, includes any mimetic of the polypeptide or polynucleotide, that is, a chemical compound having at least one endogenous function of the polypeptide or polynucleotide that the mimetic mimics. In general, amino acids can be substituted, for example, at least 1 (e.g., 1, 2, 3, 4, 5, 6, 7, 8, 9, 10 or 20 or above) amino acids can be substituted, provided that the modified sequence substantially maintains the required activity or capability. Amino acid substitution may include the use of non-naturally occurring analogues. The protein or polypeptide used in the present application may also have deletion, insertion or substitution of amino acid residues, where the amino acid residues undergo silent changes and result in functionally equivalent proteins. Intentional amino acid substitutions can be made based on the similarity of the polarity, charge, solubility, hydrophobicity, hydrophilicity, and/or the amphoteric properties of the residues, as long as the endogenous function is retained. For example, negatively charged amino acids include aspartic acid and glutamic acid; positively charged amino acids include lysine and arginine; and amino acids containing uncharged polar head-groups with a similar hydrophilic value include asparagine, glutamine, serine, threonine and tyrosine.

In the present application, the term "homologue" generally refers to an amino acid sequence or a nucleotide sequence having a certain homology with a wild-type amino acid sequence and a wild-type nucleotide sequence. The term "homology" may be equivalent to the "identity" of sequences. Homologous sequences may include amino acid sequences that are at least 80%, 85%, 90%, 99.1%, 99.2%, 99.3%, 99.4%, 99.5%, 99.6%, 99.7%, 99.8% or 99.9% the same as the subject sequence. In general, homologues will contain the same active sites as the subject amino acid sequence, and the like. Homology may be considered on the basis of similarity (i.e., amino acid residues having similar chemical properties/functions), or homology can be expressed in terms of the sequence identity. In the present application, a sequence having a percentage identity in either of the SEQ ID NOs of the mentioned amino acid sequence or nucleotide sequence refers to a sequence having the percentage identity over the whole length of the mentioned SEQ ID NOs. In order to determine the sequence identity, alignment of sequences can be performed by a variety of ways known to those skilled in the art, for example, by using BLAST, BLAST-2, ALIGN, NEEDLE or Megalign (DNASTAR) software, etc. The persons skilled in the art are able to determine the suitable parameters suitable for alignment, including any algorithms required to achieve an optimal alignment in the full-length sequence being compared.

In the present application, the term "vector" generally refers to a vector containing a recombinant polynucleotide, where the recombinant polynucleotide includes an expression control sequence efficiently linked to a nucleotide sequence to be expressed. The vector includes cis-acting elements sufficient for expression; other elements for expression may be provided by the host cell or may be provided in an in-vitro expression system. The vector may include all expression vectors known in the art that can be incorporated into the recombinant polynucleotide, including cosmid, plasmid (e.g., naked or encapsulated in liposomes) and viruses (e.g., lentiviruses, retroviruses, adenoviruses, and adeno-associated viruses). The term "lentivirus" refers to a genus of Lentiviridae. Lentiviruses are unique in retroviruses and capable of infecting non-dividing cells; they can deliver a significant amount of genetic information into DNA of host cells, therefore, they are one of the most efficient gene delivery vector methods. HIV, SIV and FIV are all examples of lentiviruses. The term "lentiviral vector" generally refers to vectors derived from at least one part of a lentivirus genome, including particularly those self-inactivated lentiviral vectors provided in Milone et al., Mol. Ther.17(8):1453-1464 (2009). Other examples of lentiviral vectors that can be used clinically include, but not limited to, e.g., LENTIVECTOR^{®} gene delivery technology of Oxford BioMedica, LENTIMAX^{™} vector system of Lentigen, etc. Non-clinical lentiviral vectors are also available and are known to those skilled in the art.

In the present application, the term "subject" generally refers to living organisms that can trigger immune responses (e.g., mammals, human).

In the present application, the term "treat" generally refers to slowing or improving the progression, severity, and/or duration of a proliferative condition, or improving one or more symptoms (e.g., one or more distinguishable symptoms) of a proliferative condition as a result of the administration of one or more therapies (for example, one or more therapeutic agents, such as the CAR of the present application). In the present application, the term "treat" may also refer to improving at least one measurable physical parameter of a proliferative condition, such as tumor growth, which physical parameter does not have to be identifiable to the patient. The term "treatment" in the present application may also refer to inhibiting the progression of a proliferative condition by for example stabilizing discernible symptoms in a physical manner, by for example stabilizing physical parameters in a physiological manner, or both. In some cases, the term "treatment" may refer to reducing or stabilizing the tumor size or the count of cancer cells.

In the present application, the term "include" generally refers to the inclusion of explicitly specified features, but not excluding other elements.

In the present application, the term "about" generally refers to varying in a range of 0.5%-10% above or below a specified value, for example, varying in a range of 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, or 10% above or below a specified value.

In one aspect, the present application provides an immune cell, which may include a chimeric antigen receptor (CAR) and/or a coding element thereof.

### Targeting moiety

The CAR of the immune cell of the present application may include a targeting moiety. The targeting moiety may be an antigen-binding domain, and the selection of the targeting moiety depends on the type and number of ligands defining the surface of the target cells. In one aspect, CAR-mediated T cell responses can be directed to target antigens by engineering an antigen-binding domain that specifically binds the desired antigens into the CAR. In some cases, the targeting moiety may specifically bind to and/or recognize tumor antigens. The tumor antigens may be tumor specific antigens (TSAs) or tumor-associated antigens (TAAs). According to the desired tumor antigen to be targeted, the CAR of the present application can be engineered to include an appropriate antigen-binding portion specific to the desired antigen. The targeting moiety may be: including, but not limited to, monoclonal antibodies, polyclonal antibodies, recombinant antibodies, human antibodies, humanized antibodies and functional fragments thereof, including, but not limited to, single-domain antibodies, such as heavy chain variable domains (VH), light chain variable domains (VL), and variable domains (VHH) of camelid-derived nanobodies. In some cases, the targeting moiety of the present application may be antibody single-chain variable regions (scFv). Generally, for ease of practical applications, the targeting moiety of CAR may include human or humanized residues for the antigen-binding domain of an antibody or an antibody fragment.

In some cases, the targeting moiety of the CAR of the present application may target a CD19 target. For example, the targeting moiety of the CAR of the present application may be the single-chain variable region of a humanized CD19 monoclonal antibody. For example, the single-chain variable region of the humanized CD19 monoclonal antibody may include an amino acid sequence as set forth in SEQ ID NO. 41. Further for example, the single-chain variable region of the humanized CD 19 monoclonal antibody can be encoded by a nucleotide sequence as set forth in SEQ ID NO. 11. Alternatively, the targeting moiety may also be the single-chain variable region of a full humanized CD19 monoclonal antibody. For example, the single-chain variable region of the full humanized CD19 monoclonal antibody may include an amino acid sequence as set forth in SEQ ID NO. 42. Further for example, the single-chain variable region of the full humanized CD19 monoclonal antibody can be encoded by a nucleotide sequence as set forth in SEQ ID NO. 12.

In some other cases, the targeting moiety of the CAR of the present application may target a CD22 target. For example, the targeting moiety targeting CD22 can be encoded by an amino acid sequence as set forth in SEQ ID NO. 48. For example, the targeting moiety targeting CD22 can be encoded by a nucleotide sequence as set forth in SEQ ID NO. 35.

In some other cases, the targeting moiety of the CAR of the present application may target a GPC3 target. For example, the targeting moiety targeting GPC3 may include a nucleotide sequence as set forth in SEQ ID NO. 54. For example, the targeting moiety targeting GPC3 may include a nucleotide sequence as set forth in SEQ ID NO. 36.

In the present application, the targeting moiety of the CAR may specifically bind to and/or recognize a target selected from a group consisting of: CD19, CD22 and GPC3.

The targeting moiety of the CAR of the present application may specifically bind to and/or recognize the targeting moieties of one or more (for example, two or more) targets. In some cases, the CAR of the present application may include a targeting moiety with binding and/or recognition specificity for one target, where the target may be selected from a group consisting of: CD19, CD22 and GPC3. In some other cases, the targeting moiety of the CAR of the present application may include a first targeting moiety with binding and/or recognition specificity for a first target, and a second targeting moiety with binding and/or recognition specificity for a second target, where the target is selected from a group consisting of: CD19 and CD22, CD19 and GPC3 as well as CD22 and GPC3.

### Transmembrane region

The CAR of the immune cell of the present application may include a transmembrane region. In some cases, the hinge region may be a transmembrane region naturally associated with one domain in the CAR. In some other cases, the transmembrane region can be modified by amino acids to avoid the binding of such a domain with transmembrane domains of the same or different surface membrane proteins, thereby minimizing the interaction with other members of the receptor complex. The transmembrane domains may be naturally-derived or synthetically-derived. In the case of naturally-derived, the domains may be derived from any membrane binding or transmembrane proteins. The transmembrane domains for particular uses in the present application may be or may be derived from those feasible in the art. The transmembrane domains may also be synthesized, which, in such cases, may mainly include primarily hydrophobic amino acid residues. In some cases, the transmembrane regions may be a transmembrane region of a TMEM59 protein. For example, the transmembrane region of the TMEM59 protein may include an amino acid sequence as set forth in SEQ ID NO. 1. Further for example, the transmembrane region of the TMEM59 protein can be encoded by a nucleotide sequence as set forth in SEQ ID NO. 5. In some other cases, the transmembrane region may be a transmembrane region of CD8. For example, the transmembrane region of CD8 may include an amino acid sequence as set forth in SEQ ID NO. 37. Further for example, the transmembrane region of CD8 can be encoded by a nucleotide sequence as set forth in SEQ ID NO. 34.

### Hinge region

In some cases, the transmembrane region can be linked to an extracellular region of CAR through a hinge, for example, the targeting moiety of the CAR. The CAR of the immune cell of the present application may include a hinge region. In some cases, the hinge region may be a hinge region of a TMEM59 protein. For example, the hinge region of the TMEM59 protein may include an amino acid sequence as set forth in SEQ ID NO. 2. Further for example, the TMEM59 protein can be encoded by a nucleotide sequence as set forth in SEQ ID NO. 6. In some other cases, the hinge region may be a hinge region of CD8. For example, the hinge region of CD8 may include an amino acid sequence as set forth in SEQ ID NO. 38. Further for example, the hinge region of CD8 can be encoded by a nucleotide sequence as set forth in SEQ ID NO. 8.

### Intracellular domain

In the present application, the CAR of the immune cell may also include an intracellular domain. The intracellular domain may include a signaling domain. Examples of intracellular signaling domains that can be used in the CAR of the present application may generally include cytoplasmic sequences of T cell receptors (TCRs) and co-receptors that act together in order to trigger signal transduction after the adaption of antigen receptors, as well as any derivatives or variants of these sequences and any one recombinant sequence with the same functional capacity.

It is known that signals generated through the TCRs alone are insufficient to completely activate T cells, and secondary or co-stimulatory signals are also required. Therefore, the activation of T cells can be considered to be mediated by two different kinds of cytoplasmic signaling sequences: antigen-dependent primarily activated signaling sequences initiated with TCR (primary cytoplasmic signaling sequences) and signaling sequences providing secondary or co-stimulatory signals by acting in an antigen-independent manner (secondary cytoplasmic signaling sequences, e.g., costimulatory domains).

Primary cytoplasmic signaling sequences regulate the primary activation of TCR complexes in a stimulatory manner or in an inhibitory manner. The primary cytoplasmic signaling sequences that act in a stimulatory manner may include signaling motifs, e.g., immune receptor tyrosine-based activation motifs (ITAMs). The ITAMs that can be used as the primary cytoplasmic signaling sequences in the present application may include any signaling domains feasible in the art. In some cases, the signaling domains of the present application may be a signaling domain of CD3zeta, which may include a nucleotide sequence as set forth in SEQ ID NO. 9.

In some cases, the intracellular domain of the CAR of the present application may include a signaling domain of CD3zeta. Alternatively, the signaling domain can also combine with any other feasible one or more intracellular domains if it is useful for the functioning of the CAR of the present application. For example, the intracellular domain of the CAR may include the chain portion and the costimulatory domain of CD3zeta. The costimulatory domain generally refers to the portion in the CAR which includes the intracellular domain of the co-stimulatory molecule. The co-stimulatory molecule generally refers to cell surface molecules other than antigen receptors or their ligands that are required for the effective response of lymphocytes to an antigen. Examples of co-stimulatory molecules suitable for the present application may include those feasible in the art. In some cases, the costimulatory domain of the present application may include a costimulatory domain of 4-1BB. For example, the costimulatory domain of 4-1BB may include a nucleotide sequence as set forth in SEQ ID NO. 10.

### TMEM59 protein and/or functional fragment thereof

The immune cell of the present application may include a TMEM59 protein and/or a functional fragment thereof located on the cell membrane.

In some cases, the immune cell of the present application may include a transmembrane region and a hinge region of the TMEM59 protein. In some cases, the TMEM59 protein may be a human TMEM59 protein. For example, the transmembrane region of the TMEM59 protein may include an amino acid sequence as set forth in SEQ ID NO. 1. For example, the hinge region of the TMEM59 protein may include an amino acid sequence as set forth in SEQ ID NO. 2.

In some cases, the immune cell may include a TMEM59 protein and/or a functional fragment thereof, wherein the functional fragment includes at least a transmembrane region and a hinge region of the TMEM59 protein. In some cases, the functional fragment may be a human TMEM59 protein and/or a functional fragment thereof. For example, the transmembrane region of the TMEM59 protein may include an amino acid sequence as set forth in SEQ ID NO. 1. For example, the hinge region of the TMEM59 protein may include an amino acid sequence as set forth in SEQ ID NO. 2. For example, the TMEM59 protein and/or the functional fragment thereof may include an amino acid sequence as set forth in SEQ ID NO. 3.

In some cases, the TMEM59 protein and/or the functional fragment thereof can be located on the cell membrane of the immune cell. In some other cases, the immune cell can be modified to include the TMEM59 protein and/or the functional fragment thereof on the cell membrane.

The CAR of the present application may also include a signal peptide. In some cases, the signal peptide may include a CD8 signal peptide. For example, the CD8 signal peptide may include an amino acid sequence as set forth in SEQ ID NO. 46, for example, the CD8 signal peptide can be encoded by a nucleotide sequence as set forth in SEQ ID NO. 16.

The immune cell of the present application may include the CAR as well as the TMEM59 protein and/or the functional fragment thereof. In some cases, the TMEM59 protein and/or the functional fragment thereof can be located on the cell membrane of the immune cell.

### TMEM59 co-expressed by chimeric antigen receptors

In some cases, the CAR may not include the TMEM59 protein and/or the functional fragment thereof. In some specific cases, the CAR may include the targeting moiety, the hinge region, the transmembrane region, the signaling domain and the costimulatory domain. The CAR may include the targeting moiety, the hinge region of CD8, the transmembrane region of CD8, the signaling domain of CD3zeta and the costimulatory domain of 4-1BB. In some cases, the CAR can co-express the TMEM59 protein and/or the functional fragment thereof. In some cases, the intracellular domain of the CAR can be directly or indirectly linked to the TMEM59 protein and/or the functional fragment thereof. For example, the CAR may include an amino acid sequence as set forth in any one of SEQ ID NOs. 49, 51, 55 and 56. For example, the CAR can be encoded by a nucleotide sequence as set forth in any one of SEQ ID NOs. 19, 21, 25 and 28. For example, the TMEM59 protein and/or the functional fragment thereof may include an amino acid sequence as set forth in any one of SEQ ID NOs. 1-3. For example, the TMEM59 protein and/or the functional fragment thereof can be encoded by a nucleotide sequence as set forth in any one of SEQ ID NOs. 4-6.

For example, the CAR may include the targeting moiety of the single-chain variable region of the humanized CD19 monoclonal antibody, the hinge region of CD8, the transmembrane region of CD8, the signaling domain of CD3zeta and the costimulatory domain of 4-1BB. For example, the CAR may include an amino acid sequence as set forth in SEQ ID NO. 49. Further for example, the CAR can be encoded by a nucleotide sequence as set forth in SEQ ID NO. 19. In some cases, the CAR can co-express the TMEM59 protein and/or the functional fragment thereof. For example, the TMEM59 protein and/or the functional fragment thereof may include an amino acid sequence as set forth in SEQ ID NO. 3. Further for example, the TMEM59 protein and/or the functional fragment thereof can be encoded by an amino acid sequence as set forth in SEQ ID NO. 4. The protein in the co-expression of the TMEM59 protein and/or the functional fragment thereof by the CAR of the present application may include an amino acid sequence as set forth in SEQ ID NO. 50 or 52. Further for example, the protein in the co-expression of the TMEM59 protein and/or the functional fragment thereof by the CAR of the present application can be encoded by a nucleotide sequence as set forth in any one of SEQ ID NOs. 20, 32, 36 and 29.

For example, the CAR may include the targeting moiety of the single-chain variable region of the full humanized CD19 monoclonal antibody, the hinge region of CD8, the transmembrane region of CD8, the signaling domain of CD3zeta and the costimulatory domain of 4-1BB. For example, the CAR may include an amino acid sequence as set forth in SEQ ID NO. 51. For example, the CAR can be encoded by a nucleotide sequence as set forth in SEQ ID NO. 21. In some cases, the CAR can co-express the TMEM59 protein and/or the functional fragment thereof. For example, the TMEM59 protein and/or the functional fragment thereof may include a nucleotide sequence as set forth in SEQ ID NO. 3. For example, the TMEM59 protein and/or the functional fragment thereof can be encoded by a nucleotide sequence as set forth in SEQ ID NO. 4.

For example, the CAR may include the GPC3 targeting moiety, the hinge region of CD8, the transmembrane region of CD8, the signaling domain of CD3zeta and the costimulatory domain of 4-1BB. For example, the CAR may include an amino acid sequence as set forth in SEQ ID NO. 55. For example, the CAR can be encoded by a nucleotide sequence as set forth in SEQ ID NO. 25. In some cases, the CAR can co-express the TMEM59 protein and/or the functional fragment thereof. For example, the TMEM59 protein and/or the functional fragment thereof may include a nucleotide sequence as set forth in SEQ ID NO. 3. For example, the TMEM59 protein and/or the functional fragment thereof can be encoded by a nucleotide sequence as set forth in SEQ ID NO. 4. For example, the CAR may include CD19 and CD22 targeting moieties, the hinge region of CD8, the transmembrane region of CD8, the signaling domain of CD3zeta and the costimulatory domain of 4-1BB. For example, the CAR may include a nucleotide sequence as set forth in SEQ ID NO.28. In some cases, the CAR can co-express the TMEM59 protein and/or the functional fragment thereof. For example, the TMEM59 protein and/or the functional fragment thereof may include an amino acid sequence as set forth in SEQ ID NO. 3. For example, the TMEM59 protein and/or the functional fragment thereof can be encoded by a nucleotide sequence as set forth in SEQ ID NO. 4.

In the present application, the CAR and the TMEM59 protein and/or the functional fragment thereof can be co-expressed through a shear peptide. For example, the shear peptide may be a 2A sequence. Further for example, the 2A sequence may include an amino acid sequence as set forth in SEQ ID NO. 47. Further for example, the 2A sequence can be encoded by a nucleotide sequence as set forth in SEQ ID NO. 17. In the present application, the CAR and the TMEM59 protein and/or the functional fragment thereof can be co-expressed through an IRES sequence. Further for example, the IRES sequence may include a nucleotide sequence as set forth in SEQ ID NO. 18.

### TMEM59 modified chimeric antigen receptors

In some other cases, the CAR may include the TMEM59 protein and/or the functional fragment thereof. In some specific cases, the CAR may include the targeting moiety, the hinge region, the transmembrane region, the signaling domain and the costimulatory domain. In some cases, the CAR may include the targeting moiety, the hinge region of the TMEM59 protein, the transmembrane region of the TMEM59 protein, the signaling domain and the costimulatory domain. For example, the CAR may include the targeting moiety, the hinge region of the TMEM59 protein, the transmembrane region of the TMEM59 protein, the signaling domain of CD3zeta and the costimulatory domain of 4-1BB.

For example, the CAR may include an amino acid sequence as set forth in any one of SEQ ID NOs. 43, 57 and 44. For example. The CAR can be encoded by a nucleotide sequence as set forth in anyone of SEQ ID NOs. 13, 14, 31, 32 and 33.

For example, the CAR may include the targeting moiety of the single-chain variable region of the full humanized CD19 monoclonal antibody, the hinge region of the TMEM59 protein, the transmembrane region of the TMEM59 protein, the signaling domain of CD3zeta and the costimulatory domain of 4-1BB. For example, the CAR may include an amino acid sequence as set forth in SEQ ID NO. 44. For example, the CAR can be encoded by a nucleotide sequence as set forth in SEQ ID NO. 7 or 33.

For example, the CAR may include CD19 and CD22 targeting moieties, the hinge region of the TMEM59 protein, the transmembrane region of the TMEM59 protein, the signaling domain of CD3zeta and the costimulatory domain of 4-1BB. For example, the CAR may include an amino acid sequence as set forth in SEQ ID NO. 57. For example, the CAR can be encoded by a nucleotide sequence as set forth in SEQ ID NO. 31 or 32.

For example, the CAR may include GPC3 targeting moiety, the hinge region of the TMEM59 protein, the transmembrane region of the TMEM59 protein, the signaling domain of CD3zeta and the costimulatory domain of 4-1BB. For example, the CAR may include a nucleotide sequence as set forth in SEQ ID NO. 43. For example, the CAR can be encoded by a nucleotide sequence as set forth in SEQ ID NO. 13 or 14.

### Vectors, cells and preparation methods

In one aspect, the present application provides one or more isolated nucleic acids, which can encode the CAR polypeptide or portions thereof. Generally, the expression of a natural or synthetic nucleic acid encoding a CAR is achieved by operatively linking a nucleic acid encoding a CAR polypeptide or a portion thereof to a promoter and incorporating the construct into an expression vector. The vector may be suitable for replicating and integrating eukaryotic cells. Typical cloning vectors include transcriptional and translational terminators, initiation sequences and promoters that can be used for regulating the expression of desired nucleic acid sequences.

The nucleic acid sequences encoding the desired molecules can be obtained by using recombinant methods known in the art, such as, for example using standard techniques, by screening libraries from cells expressing the gene, or by obtaining the gene from vectors known to include the gene, or by direct isolation from cells and tissues including the gene. Nucleic acids can be cloned into the vectors, for example, nucleic acids can be cloned into vectors including, but not limited to, plasmids, phagemids, phage derivatives, animal viruses and cosmids. In particular, the target vectors may include expression vectors, replication vectors, probe-producing vectors, and sequencing vectors.

In another aspect, the present application provides cells including the nucleic acids or vectors of the present application. In some cases, the cells may be in vitro cells, which includes nucleic acids encoding the CAR of the present application. In other cases, the cells of the present application, e.g., in vitro cells, may include polypeptides encoded by the CAR of the present application.

For the nucleic acids, vectors or polypeptides of the present application, any cells can be used as host cells. In some embodiments, cells may be prokaryotic cells, fungal cells, yeast cells or higher eukaryotic cells, e.g., mammal cells. Suitable prokaryotic cells include, but not limited to, eubacteria, e.g., Gram-negative or Gram-positive organisms. In some cases, cells may be human cells. In some other cases, cells may be immune cells. The immune cells of the present application are selected from a group consisting of: T lymphocytes, B lymphocytes, tumor infiltrating lymphocytes (TILs), TCR expression cells, natural killer (NK) cells, dendritic cells, granulocytes, innate lymphoid cells, megakaryocytes, monocytes, macrophages, platelets, thymocytes and myeloid cells. In some cases, the immune cells may include T lymphocytes. In some other cases, T lymphocytes may be tumor infiltrating lymphocytes (TILs), autologous T lymphocytes, engineered autologous T lymphocytes (eACTTMs), allogeneic T cells, heterologous T cells, or any combinations thereof. In some cases, T lymphocytes can be obtained from donor subjects. The cells of the present application can be obtained from any sources known in the art. For example, T lymphocytes can be differentiated from hematopoietic stem cell populations in vitro, or T lymphocytes can be obtained from subjects. T lymphocytes can be obtained from, e.g., peripheral blood mononuclear cells, bone marrow, lymph node tissues, umbilical cord blood, thymus tissues, tissues from infection sites, ascitic fluid, pleural effusion, spleen tissues as well as tumors. In addition, T lymphocytes can be derived from one or more T lymphocyte lines available in the art. T cells can also be obtained from blood samples collected from subjects by any number of technologies (e.g., FICOLL^{™} isolation and/or apheresis) known to a person skilled in the art.

Methods for introducing a gene into cells and expressing a gene into cells are known in the art. Any methods in the art can be used to introduce a vector into host cells, e.g., mammalian, bacterial, yeast or insect cells. For example, expression vectors can be transferred into the host cells by physical, chemical or biological means.

Physical methods for introducing nucleic acids into host cells include calcium phosphate precipitation, liposomal transfection, particle bombardment, microinjection, electroporation, etc. The method for producing cells including vectors and/or exogenous nucleic acids are well known in the art (see, for example, Sambrook et al., 2001, Molecular Cloning: A Laboratory Manual, Cold Spring HarborLaboratory, New York and other manuals of Virology and Molecular Biology). Chemical means for introducing nucleic acids into host cells include colloidal dispersion systems, such as macromolecular complexes, nanocapsules, microspheres, beads; and lipid-based systems, including oil-in-water emulsions, micelles, mixed micelles and liposomes. Biological methods for introducing nucleic acids of interest into host cells include the use of DNA and RNA vectors. Currently widely used methods for inserting a gene into mammals (e.g., human cells) can utilize viral vectors. The viruses used as vectors include, but are not limited to, retroviruses, adenoviruses, adeno-associated viruses, herpes viruses, and lentiviruses. Generally, suitable vectors contain origins of replication, promoter sequences, convenient restriction endonuclease sites and one or more optional markers that act in at least one organism.

### Pharmaceutical composition

The present application provides a pharmaceutical composition, which includes the immune cell and optionally a pharmaceutically acceptable carrier. In some cases, the pharmaceutical composition may include a pharmaceutically acceptable carrier, diluent, solubilizer, emulsifier, preservative and/or adjuvant. In some other cases, the pharmaceutical composition may include an excipient. In some other cases, the pharmaceutical composition may include a polynucleotide encoding the CAR of the present application. In some other cases, the pharmaceutical composition may include the CAR encoded by the polynucleotide of the present application. In some other cases, the composition may include T cells containing CAR

The pharmaceutical composition of the present application can be prepared for parenteral delivery, for inhalation or for digestive tract delivery, e.g., oral administration. The preparation of such a pharmaceutically acceptable composition is within the capability of those skilled in the art. In some cases, a buffer solution is used to maintain the pharmaceutical composition at physiological pH or below, usually in a pH range of about 5 to about 8. In some cases, when considering parenteral administration, the pharmaceutical composition is in the form of an aqueous solution that is free of pyrogen and parenterally acceptable. In some cases, the pharmaceutical composition of the present application can be formulated into an appropriately preserved sterile isotonic solution. In some embodiments, preparations with desired molecules of polymeric compounds, beads or liposomes that provide controlled or sustained release of the product can be prepared and then delivered through depot injection. In some cases, the desired molecules can be imported by an implantable drug delivery device.

### Uses, methods

In one aspect, the present application provides a method for promoting the proliferation of immune cells, which includes: allowing the immune cell to express a TMEM59 protein and/or a functional fragment thereof on its cell membrane, wherein the functional fragment includes at least a transmembrane region and a hinge region of the TMEM59 protein. In vitro amplification of CAR-T cells after antigen stimulation can be measured by flow cytometry, and the amplification of CAR-T cells maintained in the absence of restimulation can also be measured (see, for example, Milone et, al., Molecular Therapy 17 (8): 1453-1464 (2009)). In another aspect, immune cells can also be counted after culturing the immune cells under suitable conditions so that they can express the TMEM59 protein and/or the functional fragment thereof (for example, counting with a cell counter).

The immune cells of the present application may include T lymphocytes. For example, T cells may be tumor infiltrating lymphocytes (TILs), autologous T cells, engineered autologous T cells (eACTTMs), allogeneic T cells, heterologous T cells, or any combinations thereof. In some cases, T cells can be obtained from donor subjects. In some other cases, donor subjects may be human patients with cancers or tumors. In other cases, donor subjects may be human patients without cancers or tumors.

In another aspect, the present application provides a method for promoting the production of a memory immune cell. In some cases, the immune cells of the present application can undergo a stable process of in vivo T lymphocyte expansion, and can persist for an extended period of time. In some other cases, the immune cells of the present application may develop into memory immune cells that can be reactivated to inhibit any additional tumor forms or growth. For example, the immune cells of the present application can undergo strong in vivo T lymphocyte expansion to form specific memory T cells at a high level in blood and bone marrow, and persist for an extended period of time to prolong the survival time of chimeric antigen receptor T lymphocytes.

In another aspect, the present application provides a use of the immune cell or the pharmaceutical composition in the preparation of a drug for treating tumors. In some cases, the tumor may include a solid tumor and/or a non-solid tumor. The solid tumor is usually an abnormal mass of tissues that does not contain cysts or fluid areas. The solid tumor may be benign or malignant. The non-solid tumor may be, such as, a hematologic tumor. Hematologic cancers are cancers of blood or bone marrow, for example, leukemia and lymphoma. For example, the tumor may include B lymphocytic leukemia and/or liver cancer.

In another aspect, the present application further provides a method for enhancing the killing ability of an immune cell against a tumor, which includes: allowing the immune cell to express a TMEM59 protein and/or a functional fragment thereof on its cell membrane, wherein the functional fragment includes at least a transmembrane region and a hinge region of the TMEM59 protein. In some cases, the expression includes allowing the immune cell to include a nucleic acid encoding the TMEM59 protein and/or the functional fragment thereof, so as to transduce cells with the nucleic acid disclosed in the present application under suitable conditions. For example, a nucleic acid encoding CAR is used to transduce cells. Further for example, a vector including a nucleic acid encoding CAR is used to transduce cells.

The present application further provides a method for inhibiting the proliferation of tumor cells and/or killing tumor cells, which includes: contacting the immune cells with the tumor cells. Wherein, the method includes an in vitro method or an ex vivo method. In some cases, the immune cells can be contacted with the tumor cells at 1:500 to 500:1 or any number ratio therebetween. In some cases, the number ratio of the immune cells to the tumor cells may be in a range of 1:100 to 100:1 and any ratio therebetween. For example, it may be from 20:1 to 1:20.

Without wishing to be limited by any theory, the following examples are only intended to illustrate the fusion protein, the preparation methods and the uses of the present application, and are not intended to limit the inventive scope of the present application.

### Examples

### Example 1. Determination of chimeric antigen receptor sequences

H63 represents a single-chain variable region of a humanized CD19 monoclonal antibody, whose sequence structure is as set forth in SEQ ID NO: 11; 21D4 represents a single-chain variable region of a full humanized CD19 monoclonal antibody, whose sequence structure is as set forth in SEQ ID NO: 12; the sequence structure of the GPC3 targeting moiety is as set forth in SEQ ID NO: 36; the sequence structure of the CD22 targeting moiety is as set forth in SEQ ID NO: 35; the sequence structure of the green fluorescent protein (GFP) is as set forth in SEQ ID NO: 15.

### 1.1 Determination of chimeric antigen receptor co-expressed TMEM59 sequences

Structure of chimeric antigen receptor co-expressed TMEM59 sequences: CD8 signal peptide-tumor specific antigen-binding single-chain variable region-human CD8 hinge region-human CD8 transmembrane region-human 4-1BB costimulatory domain-CD3zeta signaling domain-IRES sequence or 2A sequence-TMEM59 protein full-length gene sequence. The CD8 signal peptide, human CD8 hinge region, human CD8 transmembrane region, human 4-1BB costimulatory domain, CD3zeta signaling domain and TMEM59 gene full-length sequence are searched from GenBank Database of the U.S. National Library of Medicine (https://www.ncbi.nlm.nih.gov/gene). Moreover, codons are optimized to facilitate the expression in human cells.

Where, the CD8 signal peptide may include a nucleotide sequence as set forth in SEQ ID NO: 16, the human CD8 hinge region may include a nucleotide sequence as set forth in SEQ ID NO: 8, the human CD8 transmembrane region may include a nucleotide sequence as set forth in SEQ ID NO: 7, the human 4-1BB costimulatory domain may include a nucleotide sequence as set forth in SEQ ID NO: 10, the CD3zeta signaling domain may include a nucleotide sequence as set forth in SEQ ID NO: 9, and the TMEM59 gene full-length sequence may include a nucleotide sequence as set forth in SEQ ID NO: 4.

The above gene sequences are ligated sequentially in a sequence of CD8 signal peptide, tumor specific antigen-binding single-chain variable region, human CD8 hinge region, human CD8 transmembrane region, human 4-1BB costimulatory domain, CD3zeta signaling domain, 2A (SEQ ID NO: 17) or IRES (SEQ ID NO: 18), TMEM59 protein full-length gene sequence, and ligated into a lentiviral vector (from Clontech 631988) through whole gene synthesis.

The structures of chimeric antigen receptor co-expressed TMEM59 sequences are as below,

Structure of H63 CAR-2A-TMEM59 sequence: CD8 signal peptide-H63-human CD8 hinge region-human CD8 transmembrane region-human 4-1BB costimulatory domain-CD3zeta signaling domain-2A-TMEM59 protein full-length gene sequence, and the nucleotide sequence is as set forth in SEQ ID NO: 20;

Structure of 21D4 CAR-2A-TMEM59 sequence: CD8 signal peptide-21D4-human CD8 hinge region-human CD8 transmembrane region-human 4-1BB costimulatory domain-CD3zeta signaling domain -2A- TMEM59 protein full-length gene sequence, and the nucleotide sequence is as set forth in SEQ ID NO: 22;

Structure of GPC3 CAR-IRES-TMEM59 sequence: CD8 signal peptide-GPC3-human CD8 hinge region-human CD8 transmembrane region-human 4-1BB costimulatory domain-CD3zeta signaling domain-IRES-TMEM59 protein full-length gene sequence, and the nucleotide sequence is as set forth in SEQ ID NO: 26;

Structure of CD19CD22 CAR-IRES-TMEM59 sequence: CD8 signal peptide-CD19CD22-human CD8 hinge region-human CD8 transmembrane region-human 4-1BB costimulatory domain-CD3zeta signaling domain-IRES-TMEM59 protein full-length gene sequence, and the nucleotide sequence is as set forth in SEQ ID NO: 29.

### 1.2 Determination of TMEM59 modified chimeric antigen receptor sequence

Structure of TMEM59 modified chimeric antigen receptor sequence: CD8 signal peptide-tumor specific antigen-binding single-chain variable region-human TMEM59 hinge region-human TMEM59 transmembrane region-human 4-1BB costimulatory domain-CD3zeta signaling domain gene sequence. The CD8 signal peptide, human CD8 hinge region, human CD8 transmembrane region, human 4-1BB costimulatory domain, CD3zeta signaling domain and human TMEM59 hinge region, human TMEM59 transmembrane region sequence are searched from GenBank Database of the National Library of Medicine (https://www.ncbi.nlm.nih.gov/gene). Moreover, codons are optimized to facilitate the expression in human cells. The above gene sequences are ligated sequentially in a sequence of CD8 signal peptide, tumor specific antigen-binding single-chain variable region, human TMEM59 hinge region, human TMEM59 transmembrane region, human 4-1BB costimulatory domain, CD3zeta signaling domain, and ligated into a lentiviral vector (from Clontech) through whole gene synthesis.

Where, the human TMEM59 hinge region may include a nucleotide sequence as set forth in SEQ ID NO: 6, the human TMEM59 transmembrane region may include a nucleotide sequence as set forth in SEQ ID NO: 5. The structures of the TMEM59 modified chimeric antigen receptor sequences are as below,

Structure of 21D4-59TM CAR-IRES-GFP sequence: CD8 signal peptide-21D4-human TMEM59 hinge region-human TMEM59 transmembrane region-human 4-1BB costimulatory domain-CD3zeta signaling domain-IRES-GFP gene sequence, and the nucleotide sequence is as set forth in SEQ ID NO: 7;

Structure of GPC3-59TM CAR-IRES-GFP sequence: CD8 signal peptide-GPC3-human TMEM59 hinge region-human TMEM59 transmembrane region-human 4-1BB costimulatory domain-CD3zeta signaling domain-IRES-GFP gene sequence, and the nucleotide sequence is as set forth in SEQ ID NO: 14;

Structure of CD19CD22-59TM CAR-IRES-GFP sequence: CD8 signal peptide-CD19CD22-human TMEM59 hinge region-human TMEM59 transmembrane region-human 4-1BB costimulatory domain-CD3zeta signaling domain-IRES-GFP gene sequence, and the nucleotide sequence is as set forth in SEQ ID NO: 32.

Structure of CD19CD22-59TM CAR sequence: CD8 signal peptide-CD19CD22-human TMEM59 hinge region-human TMEM59 transmembrane region-human 4-1BB costimulatory domain-CD3zeta signaling domain, and the nucleotide sequence is as set forth in SEQ ID NO: 31.

### 1.3 Determination of control sequence

The following sequence structures are ligated into a lentiviral vector (from Clontech 631988) through whole gene synthesis.

Structure of H63 CAR sequence: CD8 signal peptide-H63-human CD8 hinge region-human CD8 transmembrane region-human 4-1BB costimulatory domain-CD3zeta signaling domain, and the nucleotide sequence is as set forth in SEQ ID NO: 19;

Structure of 21D4 CAR-2A-GFP sequence: CD8 signal peptide-21D4-human CD8 hinge region-human CD8 transmembrane region-human 4-1BB costimulatory domain-CD3zeta signaling domain-2A-green fluorescent protein, and the nucleotide sequence is as set forth in SEQ ID NO: 23;

Structure of 21D4 CAR-IRES-GFP sequence: CD8 signal peptide-21D4-human CD8 hinge region-human CD8 transmembrane region-human 4-1BB costimulatory domain-CD3zeta signaling domain-IRES-green fluorescent protein, and the nucleotide sequence is as set forth in SEQ ID NO: 24;

Structure of GPC3 CAR sequence: CD8 signal peptide-GPC3-human CD8 hinge region-human CD8 transmembrane region-human 4-1BB costimulatory domain-CD3zeta signaling domain, and the nucleotide sequence is as set forth in SEQ ID NO: 25;

Structure of GPC3 CAR-IRES-GFP sequence: CD8 signal peptide-GPC3-human CD8 hinge region-human CD8 transmembrane region-human 4-1BB costimulatory domain-CD3zeta signaling domain-IRES-green fluorescent protein, and the nucleotide sequence is as set forth in SEQ ID NO: 27;

Structure of CD19CD22 CAR sequence: CD8 signal peptide-CD19CD22-human CD8 hinge region-human CD8 transmembrane region-human 4-1BB costimulatory domain-CD3zeta signaling domain, and the nucleotide sequence is as set forth in SEQ ID NO: 28;

Structure of CD19CD22 CAR-IRES-GFP sequence: CD8 signal peptide-CD19CD22-human CD8 hinge region-human CD8 transmembrane region-human 4-1BB costimulatory domain-CD3zeta signaling domain-IRES-green fluorescent protein, and the nucleotide sequence is as set forth in SEQ

ID NO: 30.

### Example 2. Preparation of over-expressed lentiviruses

### 2.1 Formulation of transfection liquid

293T cells (ATCC CRL-3216) were plated in a T25 culture flask in DMEM culture solution containing 10% fetal bovine serum (Gibco, 10099-141) and 1% penicillin/streptomycin solution (Gibco, 15140-122), and cultured in an incubator to a cell density of up to 80-90% for transfection.

Reaction solution A and reaction solution B were formulated respectively as follows, mixed thoroughly and left to stand.

Reaction solution A was formulated following Table 1:

**Table 1 Reaction system**

| Reagents | Amount |
|---|---|
| Example 1 plasmid | 3.33 µg |
| psPAX2 plasmid (FENGHUI BIO TECH) | 2.50 µg |
| pMD2.G plasmid (FENGHUI BIO TECH) | 1.67 µg |
| Opti-MEM | 100 µl |

Reaction solution B was formulated following Table 2:

**Table 2 Reaction system**

| Reagents | Volume |
|---|---|
| PEIpro transfection reagent | 15 µl |
| Opti-MEM | 100 µl |

Reaction solution B was added dropwise into reaction solution A, mixed uniformly and let stand for 15 minutes.

293T cells were taken out of the incubator, onto the surface of which was uniformly added the mixed reaction solution dropwise, and then cultured in the incubator at 37°C. The culture solution was replaced after 16-24 h.

### 2.2 Concentration of viruses

5 ml supernatant of the transfection reaction solution of plasmid 293T cells cultured in the T25 culture flask was pipetted into a centrifugal tube and centrifuged at 4800 rpm at 4°C on a benchtop centrifuge for 10 minutes. The supernatant was pipetted into a new centrifugal tube, mixed well by Lenti-X Concentrator (TaKaRa, 631231) at a volume ratio of 1:3, and centrifuged at 1500 xg at 4°C on a benchtop centrifuge for 45 minutes. The supernatant was discarded, 250 µl PBS was added, mixed well, subpackaged, and stored at -80°C.

### Example 3. Viral infection-activated T lymphocytes

Human T lymphocytes (Shanghai Sai Li Biological Technology Co. Ltd.) after being activated by CD3 antibodies and CD28 antibodies were cultured in 12-well cell culture plates. The culture solution was aspirated, aand a viral solution containing different chimeric antigen receptor sequences was added and cultured in an incubator at 37°C. After 2 days of culture, the human T lymphocytes were blown up and transferred into T25 culture flasks, and 5 ml of T lymphocyte culture solution was added for suspension culture, so as to get CD19-targeted chimeric antigen receptor co-expressed TMEM59 protein chimeric antigen receptor T lymphocytes: H63 CAR-2A-TMEM59 CAR-T lymphocytes and 21D4-2A-TMEM59 CAR-T lymphocytes; CD19-targeted TMEM59 modified chimeric antigen receptor T lymphocytes, as well as control CAR-T lymphocytes: H63 CAR-T lymphocytes and 21D4-2A-GFP CAR-T lymphocytes.

### Example 4. Memory testing of CD19-targeted chimeric antigen receptor co-expressed TMEM59 protein chimeric antigen receptor T lymphocytes

H63 CAR-T lymphocytes, H63 CAR-2A-TMEM59 CAR-T lymphocytes, 21D4-2A-GFP CAR-T lymphocytes and 21D4-2A-TMEM59 CAR-T lymphocytes obtained from Example 3 were adequately suspended respectively. After counting, a total amount of 1×10⁶ cells were transferred into flow-through tubes. The cells were washed with phosphate buffer salt solution twice and centrifuged at a centrifugal parameter of 400 xg at room temperature for 5 minutes. The supernatant was discarded, remaining 100 µl of liquid, into which was added 1 µg of biotin-protein L (ACROBIOSYSTEMS, RPL-P814R) at 1 µl per tube, and they were incubated at 4°C in the dark conditions for 30 minutes. Phosphate buffer salt solution was used to wash twice, remaining 100 µl of liquid, into which was added 1 µl PE-streptomycin (BD, 554061), 5µl CD45 RA antibody (Biolegend, 304122) and 5µl CD 62L antibody (BD, 565535), and they were incubated at 4°C in the dark conditions for 30 minutes. After washing twice with the phosphate buffer salt solution, 400 µl of phosphate buffer salt solution was added for adequate suspension. A flow cytometry testing was performed by a flow cytometer. The light chain portion of the single-chain variable region of the CAR protein was labelled with protein L, representing positive chimeric antigen receptor T lymphocytes. CD45RA and CD62L were the memory labels on the surface of T lymphocytes, and the increased expression level of CD45RA and CD62L indicated the enhanced memory of T lymphocytes.

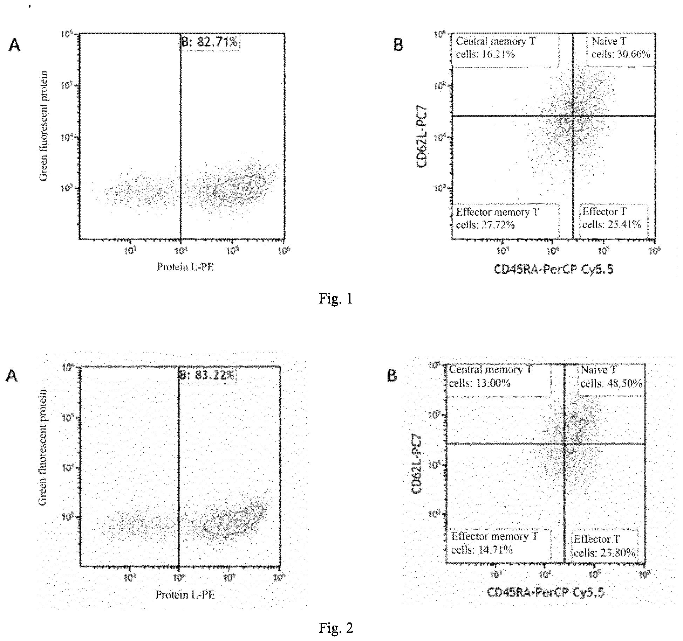

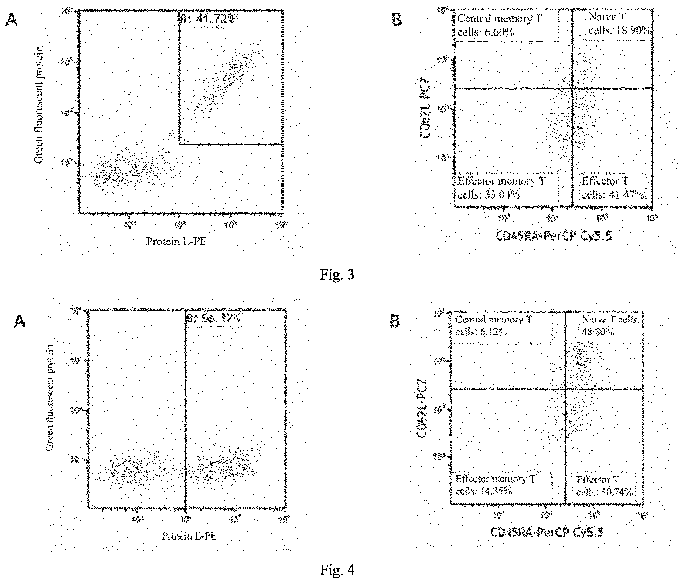

The results were shown in Figs. 1-4 and Table 3 below. Table 3 showed the number of memory cells. Compared to H63 CAR-T lymphocytes (Fig. 1), the proportion of memory cells in H63 CAR-2A-TMEM59 T lymphocytes (Fig. 2) raised from 30.66% (Fig. 1B) up to 48.50% (Fig. 2B). Similarly, compared to 21D4 CAR-2A-GFP T lymphocytes (Fig. 3), the proportion of memory cells in 21D4 CAR-2A-TMEM59 T lymphocytes (Fig. 4) raised from 18.90% (Fig. 3B) up to 48.80% (Fig. 4B). It was indicated that the additional expression of TMEM59 protein by CD19 CAR-T lymphocytes could increase the number of memory cells in CD19 CAR-T lymphocytes.

**Table 3 Number of lymphocytes**

| Samples | CAR positive cells % (protein L-positive) | Naive T lymphocytes % |
|---|---|---|
| H63 CAR-T lymphocytes | 82.71 | 30.66 |
| H63 CAR-2A-TMEM59-T lymphocytes | 83.22 | 48.50 |
| 21D4 CAR-2A-GFP-T lymphocytes | 41.72 | 18.90 |
| 21D4 CAR-2A-TMEM59-T lymphocytes | 56.37 | 48.80 |

### Example 5. Proliferative ability testing of CD19-targeted chimeric antigen receptor co-expressed TMEM59 protein chimeric antigen receptor T lymphocytes

CAR-T cells obtained from Example 3 were cultured in an incubator at 37°C. After 2 days of culture, the cells were blown up and transferred into T25 culture flasks, into which 5 ml of T lymphocyte culture solution was added for suspension culture. On day 4 of culture, 5 ml of T lymphocyte culture solution was supplemented. The cells were blown uniformly with a 1 ml gun and suspended adequately. 20 µl of cell suspension was immediately taken and injected into the sampling holes of the counting plate, and counted by a Cellometer Auto 2000 cell counter. After counting, there was a total of 10 ml cell liquid. 5 ml of cell suspension was discarded, and 5 ml of fresh culture solution was supplemented into the culture solution. The above steps were repeated after 24 hours, and the cells were counted, until day 8.

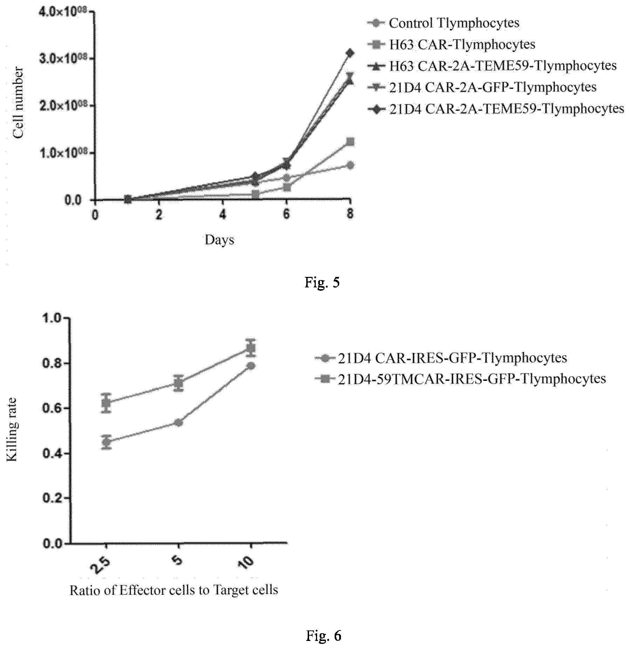

The proliferative number of cells was shown in Fig. 5. Compared to H63 CAR-T lymphocyte, the cell number of H63 CAR-2A-TMEM59-T lymphocytes increased. Similarly, compared to 21D4 CAR-2A-GFP-T lymphocytes, the cell number of 21D4 CAR-2A-TMEM59-T lymphocytes increased. It was indicated that under cultivation conditions, TMEM59 was helpful to improve the proliferative ability of CART lymphocytes.

### Example 6. Memory testing of CD19-targeted TMEM59 modified chimeric antigen receptor T lymphocytes

The memory of CD19-targeted TMEM59 modified chimeric antigen receptor T lymphocytes obtained from Example 3 was tested by a flow cytometer according to the method of Example 4.

The results showed that, compared to that in 21D4 CAR-IRES-GFP-T lymphocytes, the ratio of the number of memory cells in 21D4 59TM CAR-IRES-GFP-T lymphocytes increased, indicating that the TMEM59 modified CD19 CAR-T lymphocytes were able to enhance the number of memory cells in CD19 CAR-T lymphocytes.

### Example 7. Killing ability testing of CD19-targeted TMEM59 modified chimeric antigen receptor T lymphocytes against target cells NALM-6 (Luciferase method)

Lentivirus-infected T lymphocytes were cultured, and plated in 96-well round-bottom plates together with NALM-6 cells (Imanis LIFE SCIENCE, CL151) at ratios of effector cells (T lymphocytes): target cells (NALM-6 cells) of 10:1, 5: 1, 2.5:1, wherein the amount of NALM-6 cells was 1×10⁴/well. The culture system was RPMI-1640 culture solution containing 10% fetal bovine serum, with a total of 200 ul cell suspension per well.

The samples were grouped as shown in Table 4 below, with 3 replications for each group:

**Table 4 Ratio of effector cells to target cells**

| 10:1 | 5:1 | 2.5:1 | 0 | 10:1 | 5:1 | 2.5:1 | 0 |
|---|---|---|---|---|---|---|---|
| 21D4 CAR-IRES-GFP-T lymphocyt es + NALM-6 cells | 21D4 CAR-IRES-GFP-T lymphocyt es + NALM-6 cells | 21D4 CAR-IRES-GFP-T lymphocyt es + NALM-6 cells | NAL M-6 cells | 21D4-59TM CAR-IRES-GFP-T lymphocyt es + NALM-6 cells | 21D4-59TM CAR-IRES-GFP-T lymphocyt es + NALM-6 cells | 21D4-59TM CAR-IRES-GFP-T lymphocyt es + NALM-6 cells | NAL M-6 cells |

96-well culture plates were cultured in an incubator at 37°C for 4 hours. The testing was conducted following the instruction for the luciferase kit (promega, E2920). The 96-well culture plates that had been cultured for 4 hours were taken out, 175 µl of cell mixture was aspirated and added into the wells of clear bottom black plate 96-well culture plates, into which luciferase substrate was added at 25 µl/well, and incubated in dark at room temperature for 10 minutes, and then detected by a microplate reader.

The results were shown in Fig. 6. In this Example, the number of surviving NALM-6 cells was reflected by the activity of luciferase expressed by NALM-6 cells. Both CD19 (21D4)-targeted TMEM59 modified chimeric antigen receptor T lymphocytes (21D4-59TM CAR-IRES-GFP) and CD19 (21D4)-targeted chimeric antigen receptor T lymphocytes (21D4 CAR-IRES-GFP) were able to lyse NALM-6 cells, and the lysis of NALM-6 cells by 21D4-59TM CAR-IRES-GFP-T lymphocytes was slightly higher than that by 21D4 CAR-2A-GFP-T lymphocytes.

### Example 8. Proliferative ability testing of GPC3-targeted chimeric antigen receptor co-expressed TMEM59 protein chimeric antigen receptor T lymphocytes

The proliferative ability of GPC3-targeted chimeric antigen receptor co-expressed TMEM59 protein chimeric antigen receptor T lymphocytes obtained from Example 3 was tested according to the method of Example 5.

The results showed that, the cell number of GPC3-targeted chimeric antigen receptor co-expressed TMEM59 protein chimeric antigen receptor T lymphocytes (GPC3 CAR-IRES-TEME59-T lymphocytes) was increased compared to that of GPC3 CAR-T lymphocytes and GPC3 CAR-IRES-GFP-T lymphocytes.

### Example 9. Memory testing of GPC3-targeted TMEM59 modified chimeric antigen receptor T lymphocytes

The memory of GPC3-targeted TMEM59 modified chimeric antigen receptor T lymphocytes was tested by a flow cytometer according to the method of Example 4.

The results showed that, compared to that in GPC3 CAR-IRES-GFP T lymphocytes, the ratio of the number of memory cells in GPC3-59TM CAR-IRES-GFP-T lymphocytes increased, indicating that the TMEM59 modified GPC3 CAR-T lymphocytes were able to enhance the number of memory cells in GPC3 CAR-T lymphocytes.

### Example 10. Killing ability testing of GPC3-targeted TMEM59 modified chimeric antigen receptor T lymphocytes against target cells Huh7 (Luciferase method)

The killing ability of the GPC3-targeted TMEM59 modified chimeric antigen receptor T lymphocytes obtained from Example 3 against target cells Huh7 was tested according to the method of Example 7. The samples were grouped as shown in Table 5 below, with 3 replications for each group:

**Table 5 Ratio of effector cells to target cells**

| 10:1 | 5:1 | 2.5:1 | 0 | 10:1 | 5:1 | 2.5:1 | 0 |
|---|---|---|---|---|---|---|---|
| GPC3 | GPC3 | GPC3 | Huh | GPC3- | GPC3- | GPC3- | Huh |
| CAR-IRES-GFP-T lymphocyte s + Huh7 cells | CAR-IRES-GFP-T lymphocyte s + Huh7 cell s | CAR-IRES-GFP-T lymphocyte s + Huh7 cells | 7 cells | 59TM CAR-IRES-GFP-T lymphocyte s + Huh7 cells | 59TM CAR-IRES-GFP-T lymphocyte s + Huh7 cells | 59TM CAR-IRES-GFP-T lymphocyte s + Huh7 cells | 7 cells |

The results showed that, both GPC3-targeted TMEM59 modified chimeric antigen receptor T lymphocytes (GPC3-59TM CAR-IRES-GFP) and GPC-targeted chimeric antigen receptor T lymphocytes (GPC3 CAR-2A-GFP) were able to lyse Huh7 cells, and the lysis of Huh7 cells by GPC3-59TM CAR-IRES-GFP-T lymphocytes was higher than that by GPC3 CAR-IRES-GFP-T lymphocytes.

### Example 11. Proliferative ability testing of CD19 CD22-targeted chimeric antigen receptor co-expressed TMEM59 protein chimeric antigen receptor T lymphocytes

The proliferative ability of CD19 CD22-targeted chimeric antigen receptor co-expressed TMEM59 protein chimeric antigen receptor T lymphocytes obtained from Example 3 was tested according to the method of Example 5.

The results showed that, the cell number of CD19 CD22-targeted chimeric antigen receptor co-expressed TMEM59 protein chimeric antigen receptor T lymphocytes (CD19CD22 CAR-IRES-TEME59-T lymphocytes) was increased compared to that of CD19CD22 CAR-T lymphocytes and CD19CD21 CAR-IRES-GFP-T lymphocytes.

### Example 12. Memory testing of CD19 CD22-targeted TMEM59 modified chimeric antigen receptor T lymphocytes

The memory of CD19 CD22-targeted TMEM59 modified chimeric antigen receptor T lymphocytes obtained from Example 3 was tested by a flow cytometer according to the method of Example 4.

The results showed that, compared to that in CD19CD22 CAR-IRES-GFP T lymphocytes, the ratio of the number of memory cells in CD19CD22-59TM CAR-IRES-GFP-T lymphocytes increased, indicating that the TMEM59 modified CD19CD22 CAR-T lymphocytes were able to enhance the number of memory cells in CD19CD22 CAR-T lymphocytes.

### Example 13. Killing ability testing of CD19 CD22-targeted TMEM59 modified chimeric antigen receptor T lymphocytes against target cells NALM-6 (Luciferase method)

The killing ability of the CD19 CD22-targeted TMEM59 modified chimeric antigen receptor T lymphocytes obtained from Example 3 against target cells NALM-6 was tested according to the method of Example 7. The samples were grouped as shown in Table 6 below, with 3 replications for each group:

**Table 6 Ratio of effector cells to target cells**

| 10:1 | 5:1 | 2.5:1 | 0 | 10:1 | 5:1 | 2.5:1 | 0 |
|---|---|---|---|---|---|---|---|
| CD19CD2 2 CAR- IRES- GFP-T lymphocytes + NALM-6 cells | CD19CD2 2 CAR- IRES- GFP-T lymphocytes + NALM-6 cells | CD19CD2 2 CAR- IRES- GFP-T lymphocytes + NALM-6 cells | NAL M-6 cells | CD19CD2 2-59TM CAR- IRES- GFP-T lymphocytes + NALM-6 cells | CD19CD2 2-59TM CAR- IRES- GFP-T lymphocytes + NALM-6 cells + NALM-6 cells | CD19CD2 2-59TM CAR- IRES- GFP-T lymphocytes + NALM-6 cells + NALM-6 cells | NAL M-6 cells |

The results showed that, both CD19 CD22-targeted TMEM59 modified chimeric antigen receptor T lymphocytes (CD19 CD22-59TM CAR-IRES-GFP) and CD19 CD22-targeted chimeric antigen receptor T lymphocytes (CD19 CD22 CAR-2A-GFP) were able to lyse NALM-6 cells, and the lysis of NALM-6 cells by CD19 CD22-59TM CAR-IRES-GFP-T lymphocytes was higher than that by CD19 CD22 CAR-IRES-GFP-T lymphocytes.

### Example 14. Inhibition of tumor growth in mice by chimeric antigen receptor co-expressed TMEM59 and TMEM59 modified chimeric antigen receptor chimeric antigen receptor T lymphocytes

### 14.1 Establishment of tumor-bearing mouse models

Self-constructed target tumor cells expressing luciferase were inoculated into NSG mice for subcutaneous injection into tumors. 2×10⁵ of the above target cells were resuspended in 100 µl of phosphate buffer salt solution for subcutaneous injection.

Alternatively, self-constructed target tumor cells expressing luciferase, Nalm-6 cells, were inoculated into NSG mice for intravenous injection into tumors. 2.5×10⁵ of the above target cells were resuspended in 100 µl of phosphate buffer salt solution for intravenous injection into mouse tail.

### 14.2 In vivo imaging

3 days after tumor formation, Luciferin, a luciferase substrate, was intraperitoneally injected into mice at an injection amount of 3 mg per mouse. After anesthetization, the mice were placed in a small animal in vivo imager for imaging. 7 days after the injection of Nalm cells, T lymphocytes expressing CD19CD22-59TM CAR (whose nucleotide sequence was shown in SEQ ID NO: 31) were washed with PBS and resuspended in serum-free culture solution, and the cell density was adjusted to 2×10⁶/ml. Each mouse was injected with 2×10⁶ cells at the tail vein, with a total of 200 µl. Mice without treatment were set as a control group. After the completion of the experiment, in vivo imaging was performed periodically to collect the effect of tumor elimination. Biofluorescence assays were performed every 3-7 days, and a recurrence model challenge was performed by injecting the CAR-T dosing group with 2.5×10⁵ NALM6 tumor cells on day 60.

### 14.3 Measurement of tumor size

After injection of cells, the weight was measured twice a week, with the day of cell injection as day 0.

### 14.4 Measurement of mouse weight

After injection of cells, the tumor size was measured twice a week, with the day of cell injection as day 0. The tumor size was calculated as below: tumor size (mm³) = 0.5 × long diameter of tumor × short diameter of tumor².

### 14.5 Measurement of tumor weight

At the end of the test, tumor-bearing mice were euthanatized, and the tumor was peeled and weighed.

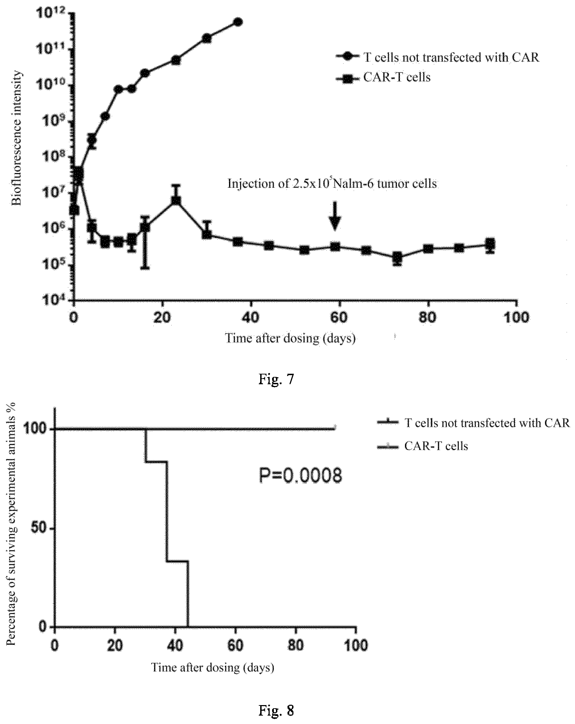

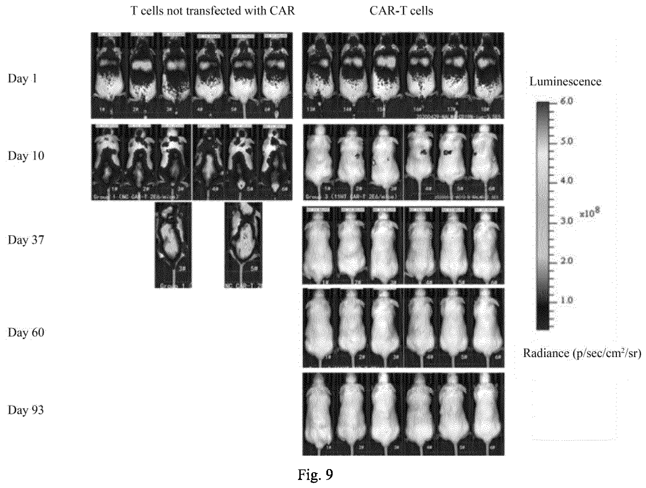

The results showed that, compared to mice in the control group, mice injected with CAR-T cells showed reduced tumor size, reduced tumor weight and slowed weight gain trend. Fig. 7 showed the biofluorescence intensity, reflecting the number of tumor cells. The results showed that, compared to the group of T cells not transfected with CAR and with increasing biofluorescence intensity, the number of tumor cells in the mice in the group of T cells transfected with CD19CD22-59TM CAR was significantly reduced, and the fluorescence intensity was approximately between 10⁵ and 10⁶ and did not increase even after reinjection of tumor cells on day 60. Fig. 8 showed the percentage of surviving experimental animals in the experimental and control groups. The survial rate of mice in the group of T cells not transfected with CAR, served as the control, decreased approximately from day 30 to 0 approximately on day 45. However, the survival rate of mice in the group of T cells transfected with CD19CD22-59TM CAR still remained at 100% after 90 days. Fig. 9 showed the in vivo imaging results of mice. For mice in the group of T cells not transfected with CAR, the light intensity did not decrease on day 10 and day 37, and the number of mice decreased on day 37 because of death. While for mice in the group of T cells transfected with CD19CD22-59TM CAR, the light intensity significantly decreased on day 10, and no light intensity could be detected after day 37, indicating that the tumor had been eliminated.

During the treatment, no animals died, and no obvious drug toxicity was shown, indicating good tolerance.

The foregoing detailed description is provided by way of explanation and examples, and is not intended to limit the scope of the appended claims. Various changes of the embodiments currently enumerated in the present application will be apparent to those of ordinary skills in the art, and are reserved within the scope of the appended claims and their equivalents.

## Claims

1. An immune cell, comprising a TMEM59 protein and/or a functional fragment thereof located on the cell membrane, wherein said functional fragment comprises at least a transmembrane region and a hinge region of said TMEM59 protein.

2. The immune cell according to claim 1, which is modified to comprise said TMEM59 protein and/or the functional fragment thereof on the cell membrane.

3. The immune cell according to any one of claims 1-2, wherein said TMEM59 protein and/or the functional fragment thereof is a human TMEM59 protein and/or a functional fragment thereof.

4. The immune cell according to any one of claims 1-3, wherein said transmembrane region of the TMEM59 protein comprises an amino acid sequence as set forth in SEQ ID NO. 1.

5. The immune cell according to any one of claims 1-4, wherein said hinge region of the TMEM59 protein comprises an amino acid sequence as set forth in SEQ ID NO. 2.

6. The immune cell according to any one of claims 1-5, wherein said TMEM59 protein and/or the functional fragment thereof comprises an amino acid sequence as set forth in SEQ ID NO. 3.

7. The immune cell according to any one of claims 1-6, wherein said immune cell comprises a chimeric antigen receptor (CAR) and/or a coding element thereof.

8. The immune cell according to claim 7, wherein said CAR comprises a transmembrane region.

9. The immune cell according to claim 8, wherein said transmembrane region of the CAR is selected from: a transmembrane region of said TMEM59 protein and a transmembrane region of CD8.

10. The immune cell according to any one of claims 8-9, wherein said transmembrane region of the CAR comprises an amino acid sequence as set forth in any one of SEQ ID NOs. 1 and 37.

11. The immune cell according to any one of claims 7-10, wherein said CAR comprises a hinge region.

12. The immune cell according to claim 11, wherein said hinge region of the CAR is selected from: a hinge region of said TMEM59 protein and a hinge region of CD8.

13. The immune cell according to any one of claims 11-12, wherein said hinge region of the CAR comprises an amino acid sequence as set forth in any one of SEQ ID NOs. 2 and 38.

14. The immune cell according to any one of claims 7-13, wherein said CAR comprises an intracellular domain, and said intracellular domain of the CAR comprises a signaling domain and/or a costimulatory domain.

15. The immune cell according to claim 14, wherein said signaling domain comprises a signaling domain of CD3zeta.

16. The immune cell according to any one of claims 14-15, wherein said costimulatory domain comprises a costimulatory domain of 4-1BB.

17. The immune cell according to any one of claims 7-16, wherein said CAR comprises a targeting moiety.

18. The immune cell according to claim 17, wherein said targeting moiety comprises ScFv.

19. The immune cell according to any one of claims 17-18, wherein said targeting moiety specifically binds to and/or recognizes a tumor antigen.

20. The immune cell according to any one of claims 17-19, wherein said targeting moiety specifically binds to and/or recognizes a target selected from a group consisting of: CD19, CD22 and GPC3.

21. The immune cell according to any one of claims 7-20, comprising said CAR as well as said TMEM59 protein and/or the functional fragment thereof, and wherein said CAR does not comprise said TMEM59 protein and/or the functional fragment thereof.

22. The immune cell according to claim 21, wherein said CAR comprises said targeting moiety, said hinge region of CD8, said transmembrane region of CD8, said signaling domain of CD3zeta and said costimulatory domain of 4-1BB as described in any one of claims 9-21, wherein said TMEM59 protein and/or the functional fragment thereof comprises said transmembrane region and said hinge region of the TMEM59 protein.

23. The immune cell according to any one of claims 21-22, wherein said CAR comprises an amino acid sequence as set forth in any one of SEQ ID NOs. 49, 51, 55 and 56.

24. The immune cell according to any one of claims 7-20, comprising said CAR as well as said TMEM59 protein and/or the functional fragment thereof, and wherein said CAR comprises said TMEM59 protein and/or the functional fragment thereof.

25. The immune cell according to claim 24, wherein said CAR comprises said targeting moiety, said hinge region of the TMEM59 protein, said transmembrane region of the TMEM59 protein, said signaling domain of CD3zeta and said costimulatory domain of 4-1BB as described in any one of claims 9-21, wherein said TMEM59 protein and/or the functional fragment thereof comprises said transmembrane region and said hinge region of the TMEM59 protein.

26. The immune cell according to any one of claims 24-25, wherein said CAR comprises an amino acid sequence as set forth in any one of SEQ ID NOs. 43, 57 and 44.

27. The immune cell according to any one of claims 1-26, wherein said immune cell comprises a T lymphocyte.

28. A pharmaceutical composition, comprising the immune cell according to any one of claims 1-27 and optionally a pharmaceutically acceptable carrier.

29. A use of the immune cell of any one of claims 1-27 or the pharmaceutical composition of claim 28 in the preparation of a drug for treating a tumor.

30. The use according to claim 29, wherein said tumor comprises a solid tumor and/or a non-solid tumor.

31. The use according to any one of claims 29-30, wherein said tumor comprises B lymphocytic leukemia and/or liver cancer.

32. A method for promoting the proliferation of an immune cell, comprising: allowing said immune cell to express a TMEM59 protein and/or a functional fragment thereof on its cell membrane, wherein said functional fragment comprises at least a transmembrane region and a hinge region of said TMEM59 protein.

33. A method for promoting the production of a memory immune cell, comprising: allowing said immune cell to express a TMEM59 protein and/or a functional fragment thereof on its cell membrane, wherein said functional fragment comprises at least a transmembrane region and a hinge region of said TMEM59 protein.

34. A method for enhancing the killing ability of an immune cell against a tumor, comprising: allowing said immune cell to express a TMEM59 protein and/or a functional fragment thereof on its cell membrane, wherein said functional fragment comprises at least a transmembrane region and a hinge region of said TMEM59 protein.

35. The method according to any one of claims 32-34, wherein said expression comprises allowing said immune cell to comprise a nucleic acid encoding said TMEM59 protein and/or the functional fragment thereof.

36. The method according to claim 35, wherein said expression comprises transfecting said immune cell with a plasmid comprising a nucleic acid encoding said TMEM59 protein and/or the functional fragment thereof.

37. The method according to any one of claims 32-36, wherein said immune cell comprises a T lymphocyte.

38. The method according to any one of claims 32-37, wherein said immune cell comprises said nucleic acid encoding said TMEM59 protein and/or the functional fragment thereof.

39. The method according to any one of claims 35-38, wherein said nucleic acid is in a viral vector.

40. A method for inhibiting the proliferation of tumor cells and/or killing tumor cells, comprising: contacting the immune cell of any one of claims 1-27 with said tumor cells.

41. The method according to claim 40, wherein said method comprises an in vitro method or an ex vivo method.

42. The method according to any one of claims 40-41, wherein said tumor comprises a solid tumor and/or a non-solid tumor.

43. The method according to any one of claims 40-42, wherein said tumor comprises B lymphocytic leukemia and/or liver cancer.


Sequence Listing

<110>  SHANGHAI PHARMACEUTICALS HOLDING CO., LTD.

<120>  TMEM59 PROTEIN DIMER OR CHIMERIC EXPRESSION RECEPTOR IMPROVING T CELL
FUNCTION

<130>  0124-PA-003EP

<160>  57

<170>  PatentIn version 3.5

<210>  1
<211>  21
<212>  PRT
<213>  Artificial Sequence

<220>
<223>  amino acid sequnces of human TMEM59 transmembrane region

<400>  1

Gly Trp Ile Leu Thr Thr Thr Leu Val Leu Ser Val Met Val Leu Leu
1               5                   10                  15

Trp Ile Cys Cys Ala
            20

<210>  2
<211>  50
<212>  PRT
<213>  Artificial Sequence

<220>
<223>  amino aicd sequence of human TMEM59 hinge region

<400>  2

Pro His Leu Glu Gln Glu Pro Thr Asn Leu Arg Glu Ser Ser Leu Ser
1               5                   10                  15

Lys Met Ser Ser Asp Leu Gln Met Arg Asn Ser Gln Ala His Arg Asn
            20                  25                  30

Phe Leu Glu Asp Gly Glu Ser Asp Gly Phe Leu Arg Cys Leu Ser Leu
        35                  40                  45

Asn Ser
    50

<210>  3
<211>  324
<212>  PRT
<213>  Artificial Sequence

<220>

<223>  amino acid sequence of human TMEM59 protein and/or its functional
fragments

<400>  3

Met Ala Ala Pro Lys Gly Ser Leu Trp Val Arg Thr Gln Leu Gly Leu
1               5                   10                  15

Pro Pro Leu Leu Leu Leu Thr Met Ala Leu Ala Gly Gly Ser Gly Thr
            20                  25                  30

Ala Ser Ala Glu Ala Phe Asp Ser Val Leu Gly Asp Thr Ala Ser Cys
        35                  40                  45

His Arg Ala Cys Gln Leu Thr Tyr Pro Leu His Thr Tyr Pro Lys Glu
    50                  55                  60

Glu Glu Leu Tyr Ala Cys Gln Arg Gly Cys Arg Leu Phe Ser Ile Cys
65                  70                  75                  80

Gln Phe Val Asp Asp Gly Ile Asp Leu Asn Arg Thr Lys Leu Glu Cys
                85                  90                  95

Glu Ser Ala Cys Thr Glu Ala Tyr Ser Gln Ser Asp Glu Gln Tyr Ala
            100                 105                 110

Cys His Leu Gly Cys Gln Asn Gln Leu Pro Phe Ala Glu Leu Arg Gln
        115                 120                 125

Glu Gln Leu Met Ser Leu Met Pro Lys Met His Leu Leu Phe Pro Leu
    130                 135                 140

Thr Leu Val Arg Ser Phe Trp Ser Asp Met Met Asp Ser Ala Gln Ser
145                 150                 155                 160

Phe Ile Thr Ser Ser Trp Thr Phe Tyr Leu Gln Ala Asp Asp Gly Lys
                165                 170                 175

Ile Val Ile Phe Gln Ser Lys Pro Glu Ile Gln Tyr Ala Pro His Leu
            180                 185                 190

Glu Gln Glu Pro Thr Asn Leu Arg Glu Ser Ser Leu Ser Lys Met Ser
        195                 200                 205

Ser Asp Leu Gln Met Arg Asn Ser Gln Ala His Arg Asn Phe Leu Glu
    210                 215                 220

Asp Gly Glu Ser Asp Gly Phe Leu Arg Cys Leu Ser Leu Asn Ser Gly
225                 230                 235                 240

Trp Ile Leu Thr Thr Thr Leu Val Leu Ser Val Met Val Leu Leu Trp
                245                 250                 255

Ile Cys Cys Ala Thr Val Ala Thr Ala Val Glu Gln Tyr Val Pro Ser
            260                 265                 270

Glu Lys Leu Ser Ile Tyr Gly Asp Leu Glu Phe Met Asn Glu Gln Lys
        275                 280                 285

Leu Asn Arg Tyr Pro Ala Ser Ser Leu Val Val Val Arg Ser Lys Thr
    290                 295                 300

Glu Asp His Glu Glu Ala Gly Pro Leu Pro Thr Lys Val Asn Leu Ala
305                 310                 315                 320

His Ser Glu Ile

<210>  4
<211>  975
<212>  DNA
<213>  Artificial Sequence

<220>
<223>  nucleotide sequence of human TMEM59 protein and/or its functional
fragments

<400>  4
atggcggcgc cgaaggggag cctctgggtg aggacccaac tggggctccc gccgctgctg      60

ctgctgacca tggccttggc cggaggttcg gggaccgctt cggctgaagc atttgactcg     120

gtcttgggtg atacggcgtc ttgccaccgg gcctgtcagt tgacctaccc cttgcacacc     180

taccctaagg aagaggagtt gtacgcatgt cagagaggtt gcaggctgtt ttcaatttgt     240

cagtttgtgg atgatggaat tgacttaaat cgaactaaat tggaatgtga atctgcatgt     300

acagaagcat attcccaatc tgatgagcaa tatgcttgcc atcttggttg ccagaatcag     360

ctgccattcg ctgaactgag acaagaacaa cttatgtccc tgatgccaaa aatgcaccta     420

ctctttcctc taactctggt gaggtcattc tggagtgaca tgatggactc cgcacagagc     480

ttcataacct cttcatggac tttttatctt caagccgatg acggaaaaat agttatattc     540

cagtctaagc cagaaatcca gtacgcacca catttggagc aggagcctac aaatttgaga     600

gaatcatctc taagcaaaat gtcctcagat ctgcaaatga gaaattcaca agcgcacagg     660

aattttcttg aagatggaga aagtgatggc tttttaagat gcctctctct taactctggg     720

tggattttaa ctacaactct tgtcctctcg gtgatggtat tgctttggat ttgttgtgca     780

actgttgcta cagctgtgga gcagtatgtt ccctctgaga agctgagtat ctatggtgac     840

ttggagttta tgaatgaaca aaagctaaac agatatccag cttcttctct tgtggttgtt 900

agatctaaaa ctgaagatca tgaagaagca gggcctctac ctacaaaagt gaatcttgct 960

cattctgaaa tttaa 975

<210> 5
<211> 63
<212> DNA
<213> Artificial Sequence

<220>
<223> nucleotide sequence of human TMEM59 transmembrane region

<400> 5
gggtggattt taactacaac tcttgtcctc tcggtgatgg tattgctttg gatttgttgt 60

gca 63

<210> 6
<211> 150
<212> DNA
<213> Artificial Sequence

<220>
<223> nucleotide sequence of human TMEM59 hinge region

<400> 6
ccacatttgg agcaggagcc tacaaatttg agagaatcat ctctaagcaa aatgtcctca 60

gatctgcaaa tgagaaattc acaagcgcac aggaattttc ttgaagatgg agaaagtgat 120

ggctttttaa gatgcctctc tcttaactct 150

<210> 7
<211> 2796
<212> DNA
<213> Artificial Sequence

<220>
<223> 21D4-59TM CAR-IRES-GFP

<400> 7
atggccttac cagtgaccgc cttgctcctg ccgctggcct tgctgctcca cgccgccagg 60

ccggccatcc agttgaccca gtctccatcc tccctgtctg catctgtagg agacagagtc 120

accatcactt gccgggcaag tcagggcatt agcagtgctt tagcctggta tcagcagaaa 180

ccagggaaag ctcctaagct cctgatctat gatgcctcca gtttggaaag tggggtccca 240

tcaaggttca gcggcagtgg atctgggaca gatttcactc tcaccatcag cagcctgcag 300

cctgaagatt ttgcaactta ttactgtcaa cagtttaata gttacccgta cacttttggc 360

caggggacca agctggagat caaaggtggc ggtggctcgg gcggtggtgg gtcgggtggc 420

ggcggatctg aggtgcagct ggtgcagtct ggagcagagg tgaaaaagcc cggggagtct 480

ctgaagatct cctgtaaggg ttctggatac agctttagca gcagctggat cggctgggtg 540

cgccagatgc ccgggaaagg cctggagtgg atggggatca tctatcctga tgactctgat 600

accagataca gtccgtcctt ccaaggccag gtcaccatct cagccgacaa gtccatcagg 660

accgcctacc tgcagtggag cagcctgaag gcctcggaca ccgccatgta ttactgtgcg 720

agacatgtta ctatgatttg gggagttatt attgacttct ggggccaggg aaccctggtc 780

accgtctcct caccacattt ggagcaggag cctacaaatt tgagagaatc atctctaagc 840

aaaatgtcct cagatctgca aatgagaaat tcacaagcgc acaggaattt tcttgaagat 900

ggagaaagtg atggcttttt aagatgcctc tctcttaact ctgggtggat tttaactaca 960

actcttgtcc tctcggtgat ggtattgctt tggatttgtt gtgcaaaacg gggcagaaag 1020

aaactcctgt atatattcaa acaaccattt atgagaccag tacaaactac tcaagaggaa 1080

gatggctgta gctgccgatt tccagaagaa gaagaaggag gatgtgaact gagagtgaag 1140

ttcagcagga gcgcagacgc ccccgcgtac aagcagggcc agaaccagct ctataacgag 1200

ctcaatctag gacgaagaga ggagtacgat gttttggaca agagacgtgg ccgggaccct 1260

gagatggggg gaaagccgag aaggaagaac cctcaggaag gcctgtacaa tgaactgcag 1320

aaagataaga tggcggaggc ctacagtgag attgggatga aaggcgagcg ccggaggggc 1380

aaggggcacg atggccttta ccagggtctc agtacagcca ccaaggacac ctacgacgcc 1440

cttcacatgc aggccctgcc ccctcgctaa ggatcccgcc cctctccctc ccccccccct 1500

aacgttactg gccgaagccg cttggaataa ggccggtgtg cgtttgtcta tatgttattt 1560

tccaccatat tgccgtcttt tggcaatgtg agggcccgga aacctggccc tgtcttcttg 1620

acgagcattc ctaggggtct ttcccctctc gccaaaggaa tgcaaggtct gttgaatgtc 1680

gtgaaggaag cagttcctct ggaagcttct tgaagacaaa caacgtctgt agcgaccctt 1740

tgcaggcagc ggaacccccc acctggcgac aggtgcctct gcggccaaaa gccacgtgta 1800

taagatacac ctgcaaaggc ggcacaaccc cagtgccacg ttgtgagttg gatagttgtg 1860

gaaagagtca aatggctctc ctcaagcgta ttcaacaagg ggctgaagga tgcccagaag 1920

gtaccccatt gtatgggatc tgatctgggg cctcggtgca catgctttac atgtgtttag 1980

tcgaggttaa aaaaacgtct aggccccccg aaccacgggg acgtggtttt cctttgaaaa 2040

acacgatgat aagcttgcca caacccacaa tctagaatgg tgagcaaggg cgaggagctg 2100

ttcaccgggg tggtgcccat cctggtcgag ctggacggcg acgtaaacgg ccacaagttc 2160

agcgtgtccg gcgagggcga gggcgatgcc acctacggca agctgaccct gaagttcatc 2220

tgcaccaccg gcaagctgcc cgtgccctgg cccaccctcg tgaccaccct gacctacggc 2280

gtgcagtgct tcagccgcta ccccgaccac atgaagcagc acgacttctt caagtccgcc 2340

atgcccgaag gctacgtcca ggagcgcacc atcttcttca aggacgacgg caactacaag 2400

acccgcgccg aggtgaagtt cgagggcgac accctggtga accgcatcga gctgaagggc     2460

atcgacttca aggaggacgg caacatcctg gggcacaagc tggagtacaa ctacaacagc     2520

cacaacgtct atatcatggc cgacaagcag aagaacggca tcaaggtgaa cttcaagatc     2580

cgccacaaca tcgaggacgg cagcgtgcag ctcgccgacc actaccagca gaacaccccc     2640

atcggcgacg gccccgtgct gctgcccgac aaccactacc tgagcaccca gtccgccctg     2700

agcaaagacc ccaacgagaa gcgcgatcac atggtcctgc tggagttcgt gaccgccgcc     2760

gggatcactc tcggcatgga cgagctgtac aagtaa                               2796


<210>  8
<211>  135
<212>  DNA
<213>  Artificial Sequence

<220>
<223>  nucleotide sequence of CD8 hinge region

<400>  8
accacgacgc cagcgccgcg accaccaaca ccggcgccca ccatcgcgtc gcagcccctg       60

tccctgcgcc cagaggcgtg ccggccagcg gcggggggcg cagtgcacac gagggggctg      120

gacttcgcct gtgat                                                       135


<210>  9
<211>  336
<212>  DNA
<213>  Artificial Sequence

<220>
<223>  nucleotide sequence of CD3 zeta signaling domain

<400>  9
agagtgaagt tcagcaggag cgcagacgcc cccgcgtacc agcagggcca gaaccagctc       60

tataacgagc tcaatctagg acgaagagag gagtacgatg ttttggacaa aagaagaggc      120

cgggaccctg agatgggggg aaagccgaga aggaagaacc ctcaggaagg cctgtacaat      180

gaactgcaga aagataagat ggcggaggcc tacagtgaga ttgggatgaa aggcgagcgc      240

cggaggggca aggggcacga tggcctttac cagggtctca gtacagccac caaggacacc      300

tacgacgccc ttcacatgca ggccctgccc cctcgc                                336


<210>  10
<211>  141
<212>  DNA
<213>  Artificial Sequence

<220>
<223>  nucleotide sequence of human 4-1BB costimulatory domain

<400>  10

cgtttctctg ttgttaaacg gggcagaaag aagctcctgt atatattcaa acaaccattt 60

atgagaccag tacaaactac tcaagaggaa gatggctgta gctgccgatt tccagaagaa 120

gaagaaggag gatgtgaact g 141

<210> 11
<211> 735
<212> DNA
<213> Artificial Sequence

<220>
<223> humanized CD19 monoclonal antibody single chain variable region H63

<400> 11
caagtgcagc tcgtccaaag cggcggagga gtggtgcagc ccggaagaag cctcagactc 60

agctgtgctg ccagcggagt cagcctcccc gactacggag tgagctgggt gagacaagcc 120

cccggcaagg gactggaatg ggtcgccgtg atttggggca gcgagaccac ctactacaac 180

tccgctctga agtctagatt cacaatctcc agagataatt ccaaaaatac cctctatctg 240

caaatgaact ctctgagggc cgaggacacc gccgtgtact actgtgctaa gcactactac 300

tatggcggca gcttcgccat ggattactgg ggacaaggca cactggtgac cgtctcctcc 360

ggaagcacca gcggaagcgg aaagcccggc agcggcgagg gctccacaaa aggagacatc 420

cagatgacac aatccccttc ctctctgagc gcttccgtcg gcgacagagt caccatcaca 480

tgcagagcca gccaagacat cagcaagtat ctgaactggt accagcagaa gcccggcaag 540

gccatcaaac tgctgatcta ccacacatct agactgcaca gcggagtgcc ctccagattc 600

tccggcagcg gcagcggcac cgactttaca ctgaccatta gctctctgca gcccgaggac 660

tttgctacct actactgtca gcaaggcgcc acactgccct atacctttgg ccccggcaca 720

aaggtggaca tcaag 735

<210> 12
<211> 792
<212> DNA
<213> Artificial Sequence

<220>
<223> full-humanized CD19 monoclonal antibody single chain variable region
21D4

<400> 12
atggccttac cagtgaccgc cttgctcctg ccgctggcct tgctgctcca cgccgccagg 60

ccggccatcc agttgaccca gtctccatcc tccctgtctg catctgtagg agacagagtc 120

accatcactt gccgggcaag tcagggcatt agcagtgctt tagcctggta tcagcagaaa 180

ccagggaaag ctcctaagct cctgatctat gatgcctcca gtttggaaag tggggtccca 240

tcaaggttca gcggcagtgg atctgggaca gatttcactc tcaccatcag cagcctgcag 300

cctgaagatt ttgcaactta ttactgtcaa cagtttaata gttacccgta cacttttggc 360

caggggacca agctggagat caaaggtggc ggtggctcgg gcggtggtgg gtcgggtggc 420

ggcggatctg aggtgcagct ggtgcagtct ggagcagagg tgaaaaagcc cggggagtct 480

ctgaagatct cctgtaaggg ttctggatac agctttagca gcagctggat cggctgggtg 540

cgccagatgc ccgggaaagg cctggagtgg atggggatca tctatcctga tgactctgat 600

accagataca gtccgtcctt ccaaggccag gtcaccatct cagccgacaa gtccatcagg 660

accgcctacc tgcagtggag cagcctgaag gcctcggaca ccgccatgta ttactgtgcg 720

agacatgtta ctatgatttg gggagttatt attgacttct ggggccaggg aaccctggtc 780

accgtctcct ca 792

<210> 13
<211> 1467
<212> DNA
<213> Artificial Sequence

<220>
<223> GPC3-59TM CAR

<400> 13
atggccttac cagtgaccgc cttgctcctg ccgctggcct tgctgctcca cgccgccagg 60

ccggacgtgg tgatgaccca gagcccttta tctttacccg ttacacccgg tgagcccgct 120

agcatctctt gtagaagcag ccagtcttta gtgcacagca acggcaacac atatttacac 180

tggtatttac agaagcccgg tcagagcccc cagctgctga tctacaaggt gagcaatcgt 240

ttctccggcg tgcccgatag attcagcggc agcggctccg gaaccgactt cactttaaag 300

atcagcagag tggaggccga ggacgtgggt gtgtactact gctcccagaa cacccacgtg 360

ccccctacat tcggtcaagg taccaaactg gagatcaaag gtggcggtgg ctcgggcggt 420

ggtgggtcgg gtggcggcgg atctcaagtt cagctggtgc agagcggcgc cgaggtcaaa 480

aaacccggag ccagcgtgaa ggtgtcttgt aaagccagcg gctacacctt taccgactat 540

gagatgcact gggtgagaca agctcccggt caaggtctgg aatggatggg cgctttagac 600

cccaagactg gtgacacagc ctactcccag aagttcaagg gtcgtgtgac tttaacagcc 660

gacgagtcca ccagcacagc ctacatggaa ctgagctctt taaggagcga ggacaccgcc 720

gtgtattact gcactcgttt ctacagctac acctactggg gccaaggtac tttagtgaca 780

gtgagcagcc cacatttgga gcaggagcct acaaatttga gagaatcatc tctaagcaaa 840

atgtcctcag atctgcaaat gagaaattca caagcgcaca ggaattttct tgaagatgga 900

gaaagtgatg gctttttaag atgcctctct cttaactctg ggtggatttt aactacaact 960

cttgtcctct cggtgatggt attgctttgg atttgttgtg caaaacgggg cagaaagaaa 1020

ctcctgtata tattcaaaca accatttatg agaccagtac aaactactca agaggaagat 1080

ggctgtagct gccgatttcc agaagaagaa gaaggaggat gtgaactgag agtgaagttc 1140

agcaggagcg cagacgcccc cgcgtacaag cagggccaga accagctcta taacgagctc 1200

aatctaggac gaagagagga gtacgatgtt ttggacaaga gacgtggccg ggaccctgag 1260

atggggggaa agccgagaag gaagaaccct caggaaggcc tgtacaatga actgcagaaa 1320

gataagatgg cggaggccta cagtgagatt gggatgaaag gcgagcgccg gaggggcaag 1380

gggcacgatg gcctttacca gggtctcagt acagccacca aggacaccta cgacgccctt 1440

cacatgcagg ccctgccccc tcgctaa 1467

<210> 14
<211> 2785
<212> DNA
<213> Artificial Sequence

<220>
<223> GPC3-59TM CAR-IRES-GFP

<400> 14
atggccttac cagtgaccgc cttgctcctg ccgctggcct tgctgctcca cgccgccagg 60

ccggacgtgg tgatgaccca gagcccttta tctttacccg ttacacccgg tgagcccgct 120

agcatctctt gtagaagcag ccagtcttta gtgcacagca acggcaacac atatttacac 180

tggtatttac agaagcccgg tcagagcccc cagctgctga tctacaaggt gagcaatcgt 240

ttctccggcg tgcccgatag attcagcggc agcggctccg gaaccgactt cactttaaag 300

atcagcagag tggaggccga ggacgtgggt gtgtactact gctcccagaa cacccacgtg 360

ccccctacat tcggtcaagg taccaaactg gagatcaaag gtggcggtgg ctcgggcggt 420

ggtgggtcgg gtggcggcgg atctcaagtt cagctggtgc agagcggcgc cgaggtcaaa 480

aaacccggag ccagcgtgaa ggtgtcttgt aaagccagcg gctacacctt taccgactat 540

gagatgcact gggtgagaca agctcccggt caaggtctgg aatggatggg cgctttagac 600

cccaagactg gtgacacagc ctactcccag aagttcaagg gtcgtgtgac tttaacagcc 660

gacgagtcca ccagcacagc ctacatggaa ctgagctctt taaggagcga ggacaccgcc 720

gtgtattact gcactcgttt ctacagctac acctactggg gccaaggtac tttagtgaca 780

gtgagcagcc cacatttgga gcaggagcct acaaatttga gagaatcatc tctaagcaaa 840

atgtcctcag atctgcaaat gagaaattca caagcgcaca ggaattttct tgaagatgga 900

gaaagtgatg gctttttaag atgcctctct cttaactctg ggtggatttt aactacaact 960

cttgtcctct cggtgatggt attgctttgg atttgttgtg caaaacgggg cagaaagaaa 1020

ctcctgtata tattcaaaca accatttatg agaccagtac aaactactca agaggaagat 1080

ggctgtagct gccgatttcc agaagaagaa gaaggaggat gtgaactgag agtgaagttc 1140

agcaggagcg cagacgcccc cgcgtacaag cagggccaga accagctcta taacgagctc 1200

aatctaggac gaagagagga gtacgatgtt ttggacaaga gacgtggccg ggaccctgag 1260

atggggggaa agccgagaag gaagaaccct caggaaggcc tgtacaatga actgcagaaa 1320

gataagatgg cggaggccta cagtgagatt gggatgaaag gcgagcgccg gaggggcaag 1380

gggcacgatg gcctttacca gggtctcagt acagccacca aggacaccta cgacgccctt 1440

cacatgcagg ccctgccccc tcgctaaccc ctctccctcc ccccccccta acgttactgg 1500

ccgaagccgc ttggaataag gccggtgtgc gtttgtctat atgttatttt ccaccatatt 1560

gccgtctttt ggcaatgtga gggcccggaa acctggccct gtcttcttga cgagcattcc 1620

taggggtctt tcccctctcg ccaaaggaat gcaaggtctg ttgaatgtcg tgaaggaagc 1680

agttcctctg gaagcttctt gaagacaaac aacgtctgta gcgacccttt gcaggcagcg 1740

gaacccccca cctggcgaca ggtgcctctg cggccaaaag ccacgtgtat aagatacacc 1800

tgcaaaggcg gcacaacccc agtgccacgt tgtgagttgg atagttgtgg aaagagtcaa 1860

atggctctcc tcaagcgtat tcaacaaggg gctgaaggat gcccagaagg taccccattg 1920

tatgggatct gatctggggc ctcggtgcac atgctttaca tgtgtttagt cgaggttaaa 1980

aaaacgtcta ggccccccga accacgggga cgtggttttc ctttgaaaaa cacgatgata 2040

agcttgccac aacccacaat ctagaatggt gagcaagggc gaggagctgt tcaccggggt 2100

ggtgcccatc ctggtcgagc tggacggcga cgtaaacggc cacaagttca gcgtgtccgg 2160

cgagggcgag ggcgatgcca cctacggcaa gctgaccctg aagttcatct gcaccaccgg 2220

caagctgccc gtgccctggc ccaccctcgt gaccaccctg acctacggcg tgcagtgctt 2280

cagccgctac cccgaccaca tgaagcagca cgacttcttc aagtccgcca tgcccgaagg 2340

ctacgtccag gagcgcacca tcttcttcaa ggacgacggc aactacaaga cccgcgccga 2400

ggtgaagttc gagggcgaca ccctggtgaa ccgcatcgag ctgaagggca tcgacttcaa 2460

ggaggacggc aacatcctgg ggcacaagct ggagtacaac tacaacagcc acaacgtcta 2520

tatcatggcc gacaagcaga agaacggcat caaggtgaac ttcaagatcc gccacaacat 2580

cgaggacggc agcgtgcagc tcgccgacca ctaccagcag aacaccccca tcggcgacgg 2640

ccccgtgctg ctgcccgaca accactacct gagcacccag tccgccctga gcaaagaccc 2700

caacgagaag cgcgatcaca tggtcctgct ggagttcgtg accgccgccg ggatcactct 2760

cggcatggac gagctgtaca agtaa 2785

<210> 15
<211> 720
<212> DNA
<213> Artificial Sequence

<220>

<223>  GFP targeting moiety

<400>  15
atggtgagca agggcgagga gctgttcacc ggggtggtgc ccatcctggt cgagctggac      60

ggcgacgtaa acggccacaa gttcagcgtg tccggcgagg gcgagggcga tgccacctac     120

ggcaagctga ccctgaagtt catctgcacc accggcaagc tgcccgtgcc ctggcccacc     180

ctcgtgacca ccctgaccta cggcgtgcag tgcttcagcc gctaccccga ccacatgaag     240

cagcacgact tcttcaagtc cgccatgccc gaaggctacg tccaggagcg caccatcttc     300

ttcaaggacg acggcaacta caagacccgc gccgaggtga agttcgaggg cgacaccctg     360

gtgaaccgca tcgagctgaa gggcatcgac ttcaaggagg acggcaacat cctggggcac     420

aagctggagt acaactacaa cagccacaac gtctatatca tggccgacaa gcagaagaac     480

ggcatcaagg tgaacttcaa gatccgccac aacatcgagg acggcagcgt gcagctcgcc     540

gaccactacc agcagaacac ccccatcggc gacggccccg tgctgctgcc cgacaaccac     600

tacctgagca cccagtccgc cctgagcaaa gaccccaacg agaagcgcga tcacatggtc     660

ctgctggagt tcgtgaccgc cgccgggatc actctcggca tggacgagct gtacaagtaa     720


<210>  16
<211>  66
<212>  DNA
<213>  Artificial Sequence

<220>
<223>  nucleotide sequence of CD8 signal peptide

<400>  16
atgctgctgc tcgtgacctc tctgctgctg tgcgaactgc cccaccccgc ctttctgctg      60

attccc                                                                  66


<210>  17
<211>  57
<212>  DNA
<213>  Artificial Sequence

<220>
<223>  2A sequence

<400>  17
gctactaact tcagcctgct gaagcaggct ggagacgtgg aggagaaccc tggacct         57


<210>  18
<211>  574
<212>  DNA
<213>  Artificial Sequence

<220>
<223>  IRES sequence

<400>  18

cccctctccc tccccccccc ctaacgttac tggccgaagc cgcttggaat aaggccggtg 60

tgcgtttgtc tatatgttat tttccaccat attgccgtct tttggcaatg tgagggcccg 120

gaaacctggc cctgtcttct tgacgagcat tcctaggggt ctttcccctc tcgccaaagg 180

aatgcaaggt ctgttgaatg tcgtgaagga agcagttcct ctggaagctt cttgaagaca 240

aacaacgtct gtagcgaccc tttgcaggca gcggaacccc ccacctggcg acaggtgcct 300

ctgcggccaa aagccacgtg tataagatac acctgcaaag gcggcacaac cccagtgcca 360

cgttgtgagt tggatagttg tggaaagagt caaatggctc tcctcaagcg tattcaacaa 420

ggggctgaag gatgcccaga aggtacccca ttgtatggga tctgatctgg ggcctcggtg 480

cacatgcttt acatgtgttt agtcgaggtt aaaaaaacgt ctaggccccc cgaaccacgg 540

ggacgtggtt ttcctttgaa aaacacgatg ataa 574

<210> 19
<211> 1470
<212> DNA
<213> Artificial Sequence

<220>
<223> H63 CAR

<400> 19
atgctgctgc tcgtgacctc tctgctgctg tgcgaactgc cccaccccgc ctttctgctg 60

attccccaag tgcagctcgt ccaaagcggc ggaggagtgg tgcagcccgg aagaagcctc 120

agactcagct gtgctgccag cggagtcagc ctccccgact acggagtgag ctgggtgaga 180

caagcccccg gcaagggact ggaatgggtc gccgtgattt ggggcagcga gaccacctac 240

tacaactccg ctctgaagtc tagattcaca atctccagag ataattccaa aaataccctc 300

tatctgcaaa tgaactctct gagggccgag gacaccgccg tgtactactg tgctaagcac 360

tactactatg gcggcagctt cgccatggat tactggggac aaggcacact ggtgaccgtc 420

tcctccggaa gcaccagcgg aagcggaaag cccggcagcg gcgagggctc cacaaaagga 480

gacatccaga tgacacaatc cccttcctct ctgagcgctt ccgtcggcga cagagtcacc 540

atcacatgca gagccagcca agacatcagc aagtatctga actggtacca gcagaagccc 600

ggcaaggcca tcaaactgct gatctaccac acatctagac tgcacagcgg agtgccctcc 660

agattctccg gcagcggcag cggcaccgac tttacactga ccattagctc tctgcagccc 720

gaggactttg ctacctacta ctgtcagcaa ggcgccacac tgccctatac ctttggcccc 780

ggcacaaagg tggacatcaa gaccacaaca cccgccccta gacctcccac ccccgcccct 840

accatcgcta gccagcctct gtctctgaga cccgaggctt gcagacccgc tgctggagga 900

gctgtgcata caagaggact ggatttcgct tgcgacatct acatctgggc ccctctggct 960

ggcacatgtg gagtgctgct gctgtctctg gtgatcaccc tctactgcaa gagaggaaga 1020

aaaaaactgc tctatatttt taaacagcct tttatgaggc ccgtccagac cacacaagag 1080

gaagacggat gtagctgcag attccccgag gaggaagagg gaggatgcga gctgagagtc 1140

aagttctcta gatccgctga tgcccccgcc tacaaacaag gccagaatca gctctacaac 1200

gagctcaacc tcggcagaag agaggagtac gacgtgctgg acaagagaag aggcagagat 1260

cccgagatgg gcggaaagcc tagaaggaag aaccctcaag agggactgta caacgagctg 1320

cagaaggaca aaatggctga ggcctacagc gaaatcggca tgaaaggcga gaggaggagg 1380

ggcaaaggac acgatggact gtaccaaggc ctctccaccg ccacaaagga cacctacgac 1440

gccctccata tgcaagctct gccccctagg 1470

<210> 20
<211> 2511
<212> DNA
<213> Artificial Sequence

<220>
<223> H63 CAR-2A-TMEM59

<400> 20
atgctgctgc tcgtgacctc tctgctgctg tgcgaactgc cccaccccgc ctttctgctg 60

attccccaag tgcagctcgt ccaaagcggc ggaggagtgg tgcagcccgg aagaagcctc 120

agactcagct gtgctgccag cggagtcagc ctccccgact acggagtgag ctgggtgaga 180

caagcccccg gcaagggact ggaatgggtc gccgtgattt ggggcagcga gaccacctac 240

tacaactccg ctctgaagtc tagattcaca atctccagag ataattccaa aaataccctc 300

tatctgcaaa tgaactctct gagggccgag gacaccgccg tgtactactg tgctaagcac 360

tactactatg gcggcagctt cgccatggat tactggggac aaggcacact ggtgaccgtc 420

tcctccggaa gcaccagcgg aagcggaaag cccggcagcg gcgagggctc cacaaaagga 480

gacatccaga tgacacaatc cccttcctct ctgagcgctt ccgtcggcga cagagtcacc 540

atcacatgca gagccagcca agacatcagc aagtatctga actggtacca gcagaagccc 600

ggcaaggcca tcaaactgct gatctaccac acatctagac tgcacagcgg agtgccctcc 660

agattctccg gcagcggcag cggcaccgac tttacactga ccattagctc tctgcagccc 720

gaggactttg ctacctacta ctgtcagcaa ggcgccacac tgccctatac ctttggcccc 780

ggcacaaagg tggacatcaa gaccacaaca cccgccccta gacctcccac ccccgcccct 840

accatcgcta gccagcctct gtctctgaga cccgaggctt gcagacccgc tgctggagga 900

gctgtgcata caagaggact ggatttcgct tgcgacatct acatctgggc ccctctggct 960

ggcacatgtg gagtgctgct gctgtctctg gtgatcaccc tctactgcaa gagaggaaga 1020

aaaaaactgc tctatatttt taaacagcct tttatgaggc ccgtccagac cacacaagag 1080

gaagacggat gtagctgcag attccccgag gaggaagagg gaggatgcga gctgagagtc 1140

aagttctcta gatccgctga tgcccccgcc tacaaacaag gccagaatca gctctacaac 1200

gagctcaacc tcggcagaag agaggagtac gacgtgctgg acaagagaag aggcagagat 1260

cccgagatgg gcggaaagcc tagaaggaag aaccctcaag agggactgta caacgagctg 1320

cagaaggaca aaatggctga ggcctacagc gaaatcggca tgaaaggcga gaggaggagg 1380

ggcaaaggac acgatggact gtaccaaggc ctctccaccg ccacaaagga cacctacgac 1440

gccctccata tgcaagctct gccccctagg ggaagcggag ctactaactt cagcctgctg 1500

aagcaggctg gagacgtgga ggagaaccct ggacctatgg cggcgccgaa ggggagcctc 1560

tgggtgagga cccaactggg gctcccgccg ctgctgctgc tgaccatggc cttggccgga 1620

ggttcgggga ccgcttcggc tgaagcattt gactcggtct tgggtgatac ggcgtcttgc 1680

caccgggcct gtcagttgac ctaccccttg cacacctacc ctaaggaaga ggagttgtac 1740

gcatgtcaga gaggttgcag gctgttttca atttgtcagt ttgtggatga tggaattgac 1800

ttaaatcgaa ctaaattgga atgtgaatct gcatgtacag aagcatattc ccaatctgat 1860

gagcaatatg cttgccatct tggttgccag aatcagctgc cattcgctga actgagacaa 1920

gaacaactta tgtccctgat gccaaaaatg cacctactct ttcctctaac tctggtgagg 1980

tcattctgga gtgacatgat ggactccgca cagagcttca taacctcttc atggactttt 2040

tatcttcaag ccgatgacgg aaaaatagtt atattccagt ctaagccaga aatccagtac 2100

gcaccacatt tggagcagga gcctacaaat ttgagagaat catctctaag caaaatgtcc 2160

tcagatctgc aaatgagaaa ttcacaagcg cacaggaatt ttcttgaaga tggagaaagt 2220

gatggctttt taagatgcct ctctcttaac tctgggtgga ttttaactac aactcttgtc 2280

ctctcggtga tggtattgct ttggatttgt tgtgcaactg ttgctacagc tgtggagcag 2340

tatgttccct ctgagaagct gagtatctat ggtgacttgg agtttatgaa tgaacaaaag 2400

ctaaacagat atccagcttc ttctcttgtg gttgttagat ctaaaactga agatcatgaa 2460

gaagcagggc ctctacctac aaaagtgaat cttgctcatt ctgaaattta a 2511

<210> 21
<211> 1518
<212> DNA
<213> Artificial Sequence

<220>
<223> 21D4 CAR

<400> 21
atggccttac cagtgaccgc cttgctcctg ccgctggcct tgctgctcca cgccgccagg 60

ccggccatcc agttgaccca gtctccatcc tccctgtctg catctgtagg agacagagtc 120

accatcactt gccgggcaag tcagggcatt agcagtgctt tagcctggta tcagcagaaa 180

ccagggaaag ctcctaagct cctgatctat gatgcctcca gtttggaaag tggggtccca 240

tcaaggttca gcggcagtgg atctgggaca gatttcactc tcaccatcag cagcctgcag 300

cctgaagatt ttgcaactta ttactgtcaa cagtttaata gttacccgta cacttttggc 360

caggggacca agctggagat caaaggtggc ggtggctcgg gcggtggtgg gtcgggtggc 420

ggcggatctg aggtgcagct ggtgcagtct ggagcagagg tgaaaaagcc cggggagtct 480

ctgaagatct cctgtaaggg ttctggatac agctttagca gcagctggat cggctgggtg 540

cgccagatgc ccgggaaagg cctggagtgg atggggatca tctatcctga tgactctgat 600

accagataca gtccgtcctt ccaaggccag gtcaccatct cagccgacaa gtccatcagg 660

accgcctacc tgcagtggag cagcctgaag gcctcggaca ccgccatgta ttactgtgcg 720

agacatgtta ctatgatttg gggagttatt attgacttct ggggccaggg aaccctggtc 780

accgtctcct cattcgtgcc ggtcttcctg ccagcgaagc ccaccacgac gccagcgccg 840

cgaccaccaa caccggcgcc caccatcgcg tcgcagcccc tgtccctgcg cccagaggcg 900

tgccggccag cggcgggggg cgcagtgcac acgagggggc tggacttcgc ctgtgatatc 960

tacatctggg cgcccttggc cgggacttgt ggggtccttc tcctgtcact ggttatcacc 1020

ctttactgca accacaggaa ccgtttctct gttgttaaac ggggcagaaa gaagctcctg 1080

tatatattca aacaaccatt tatgagacca gtacaaacta ctcaagagga agatggctgt 1140

agctgccgat ttccagaaga agaagaagga ggatgtgaac tgagagtgaa gttcagcagg 1200

agcgcagacg cccccgcgta ccagcagggc cagaaccagc tctataacga gctcaatcta 1260

ggacgaagag aggagtacga tgttttggac aagagacgtg gccgggaccc tgagatgggg 1320

ggaaagccga gaaggaagaa ccctcaggaa ggcctgtaca atgaactgca gaaagataag 1380

atggcggagg cctacagtga gattgggatg aaaggcgagc gccggagggg caaggggcac 1440

gatggccttt accagggtct cagtacagcc accaaggaca cctacgacgc ccttcacatg 1500

caggccctgc cccctcgc 1518

<210> 22
<211> 2682
<212> DNA
<213> Artificial Sequence

<220>
<223> 21D4 CAR-2A-TMEM59

<400> 22
atggccttac cagtgaccgc cttgctcctg ccgctggcct tgctgctcca cgccgccagg 60

ccggccatcc agttgaccca gtctccatcc tccctgtctg catctgtagg agacagagtc 120

accatcactt gccgggcaag tcagggcatt agcagtgctt tagcctggta tcagcagaaa 180

ccagggaaag ctcctaagct cctgatctat gatgcctcca gtttggaaag tggggtccca 240

tcaaggttca gcggcagtgg atctgggaca gatttcactc tcaccatcag cagcctgcag 300

cctgaagatt ttgcaactta ttactgtcaa cagtttaata gttacccgta cacttttggc 360

caggggacca agctggagat caaaggtggc ggtggctcgg gcggtggtgg gtcgggtggc 420

ggcggatctg aggtgcagct ggtgcagtct ggagcagagg tgaaaaagcc cggggagtct 480

ctgaagatct cctgtaaggg ttctggatac agctttagca gcagctggat cggctgggtg 540

cgccagatgc ccgggaaagg cctggagtgg atggggatca tctatcctga tgactctgat 600

accagataca gtccgtcctt ccaaggccag gtcaccatct cagccgacaa gtccatcagg 660

accgcctacc tgcagtggag cagcctgaag gcctcggaca ccgccatgta ttactgtgcg 720

agacatgtta ctatgatttg gggagttatt attgacttct ggggccaggg aaccctggtc 780

accgtctcct cattcgtgcc ggtcttcctg ccagcgaagc ccaccacgac gccagcgccg 840

cgaccaccaa caccggcgcc caccatcgcg tcgcagcccc tgtccctgcg cccagaggcg 900

tgccggccag cggcgggggg cgcagtgcac acgagggggc tggacttcgc ctgtgatatc 960

tacatctggg cgcccttggc cgggacttgt ggggtccttc tcctgtcact ggttatcacc 1020

ctttactgca accacaggaa caggagtaag aggagcaggc tcctgcacag tgactacatg 1080

aacatgactc cccgccgccc cgggcccacc cgcaagcatt accagcccta tgccccacca 1140

cgcgacttcg cagcctatcg ctcccgtttc tctgttgtta aacggggcag aaagaagctc 1200

ctgtatatat tcaaacaacc atttatgaga ccagtacaaa ctactcaaga ggaagatggc 1260

tgtagctgcc gatttccaga agaagaagaa ggaggatgtg aactgagagt gaagttcagc 1320

aggagcgcag acgcccccgc gtaccagcag ggccagaacc agctctataa cgagctcaat 1380

ctaggacgaa gagaggagta cgatgttttg gacaagagac gtggccggga ccctgagatg 1440

gggggaaagc cgagaaggaa gaaccctcag gaaggcctgt acaatgaact gcagaaagat 1500

aagatggcgg aggcctacag tgagattggg atgaaaggcg agcgccggag gggcaagggg 1560

cacgatggcc tttaccaggg tctcagtaca gccaccaagg acacctacga cgcccttcac 1620

atgcaggccc tgccccctcg cggaagcgga gctactaact tcagcctgct gaagcaggct 1680

ggagacgtgg aggagaaccc tggacctatg gcggcgccga aggggagcct ctgggtgagg 1740

acccaactgg ggctcccgcc gctgctgctg ctgaccatgg ccttggccgg aggttcgggg 1800

accgcttcgg ctgaagcatt tgactcggtc ttgggtgata cggcgtcttg ccaccgggcc 1860

tgtcagttga cctacccctt gcacacctac cctaaggaag aggagttgta cgcatgtcag 1920

agaggttgca ggctgttttc aatttgtcag tttgtggatg atggaattga cttaaatcga 1980

actaaattgg aatgtgaatc tgcatgtaca gaagcatatt cccaatctga tgagcaatat 2040

gcttgccatc ttggttgcca gaatcagctg ccattcgctg aactgagaca agaacaactt 2100

atgtccctga tgccaaaaat gcacctactc tttcctctaa ctctggtgag gtcattctgg 2160

agtgacatga tggactccgc acagagcttc ataacctctt catggacttt ttatcttcaa 2220

gccgatgacg gaaaaatagt tatattccag tctaagccag aaatccagta cgcaccacat 2280

ttggagcagg agcctacaaa tttgagagaa tcatctctaa gcaaaatgtc ctcagatctg 2340

caaatgagaa attcacaagc gcacaggaat tttcttgaag atggagaaag tgatggcttt 2400

ttaagatgcc tctctcttaa ctctgggtgg attttaacta caactcttgt cctctcggtg 2460

atggtattgc tttggatttg ttgtgcaact gttgctacag ctgtggagca gtatgttccc 2520

tctgagaagc tgagtatcta tggtgacttg gagtttatga atgaacaaaa gctaaacaga 2580

tatccagctt cttctcttgt ggttgttaga tctaaaactg aagatcatga agaagcaggg 2640

cctctaccta caaaagtgaa tcttgctcat tctgaaattt aa 2682

<210> 23
<211> 2304
<212> DNA
<213> Artificial Sequence

<220>
<223> 21D4 CAR-2A-GFP

<400> 23
atggccttac cagtgaccgc cttgctcctg ccgctggcct tgctgctcca cgccgccagg 60

ccggccatcc agttgaccca gtctccatcc tccctgtctg catctgtagg agacagagtc 120

accatcactt gccgggcaag tcagggcatt agcagtgctt tagcctggta tcagcagaaa 180

ccagggaaag ctcctaagct cctgatctat gatgcctcca gtttggaaag tggggtccca 240

tcaaggttca gcggcagtgg atctgggaca gatttcactc tcaccatcag cagcctgcag 300

cctgaagatt ttgcaactta ttactgtcaa cagtttaata gttacccgta cacttttggc 360

caggggacca agctggagat caaaggtggc ggtggctcgg gcggtggtgg gtcgggtggc 420

ggcggatctg aggtgcagct ggtgcagtct ggagcagagg tgaaaaagcc cggggagtct 480

ctgaagatct cctgtaaggg ttctggatac agctttagca gcagctggat cggctgggtg 540

cgccagatgc ccgggaaagg cctggagtgg atggggatca tctatcctga tgactctgat 600

accagataca gtccgtcctt ccaaggccag gtcaccatct cagccgacaa gtccatcagg 660

accgcctacc tgcagtggag cagcctgaag gcctcggaca ccgccatgta ttactgtgcg 720

agacatgtta ctatgatttg gggagttatt attgacttct ggggccaggg aaccctggtc 780

accgtctcct cattcgtgcc ggtcttcctg ccagcgaagc ccaccacgac gccagcgccg 840

cgaccaccaa caccggcgcc caccatcgcg tcgcagcccc tgtccctgcg cccagaggcg 900

tgccggccag cggcgggggg cgcagtgcac acgagggggc tggacttcgc ctgtgatatc 960

tacatctggg cgcccttggc cgggacttgt ggggtccttc tcctgtcact ggttatcacc 1020

ctttactgca accacaggaa ccgtttctct gttgttaaac ggggcagaaa gaagctcctg 1080

tatatattca aacaaccatt tatgagacca gtacaaacta ctcaagagga agatggctgt 1140

agctgccgat ttccagaaga agaagaagga ggatgtgaac tgagagtgaa gttcagcagg 1200

agcgcagacg cccccgcgta ccagcagggc cagaaccagc tctataacga gctcaatcta 1260

ggacgaagag aggagtacga tgttttggac aagagacgtg gccgggaccc tgagatgggg 1320

ggaaagccga gaaggaagaa ccctcaggaa ggcctgtaca atgaactgca gaaagataag 1380

atggcggagg cctacagtga gattgggatg aaaggcgagc gccggagggg caaggggcac 1440

gatggccttt accagggtct cagtacagcc accaaggaca cctacgacgc ccttcacatg 1500

caggccctgc cccctcgcgg aagcggagct actaacttca gcctgctgaa gcaggctgga 1560

gacgtggagg agaaccctgg acctatggtg agcaagggcg aggagctgtt caccggggtg 1620

gtgcccatcc tggtcgagct ggacggcgac gtaaacggcc acaagttcag cgtgtccggc 1680

gagggcgagg gcgatgccac ctacggcaag ctgaccctga agttcatctg caccaccggc 1740

aagctgcccg tgccctggcc caccctcgtg accaccctga cctacggcgt gcagtgcttc 1800

agccgctacc ccgaccacat gaagcagcac gacttcttca agtccgccat gcccgaaggc 1860

tacgtccagg agcgcaccat cttcttcaag gacgacggca actacaagac ccgcgccgag 1920

gtgaagttcg agggcgacac cctggtgaac cgcatcgagc tgaagggcat cgacttcaag 1980

gaggacggca acatcctggg gcacaagctg gagtacaact acaacagcca caacgtctat 2040

atcatggccg acaagcagaa gaacggcatc aaggtgaact tcaagatccg ccacaacatc 2100

gaggacggca gcgtgcagct cgccgaccac taccagcaga acacccccat cggcgacggc 2160

cccgtgctgc tgcccgacaa ccactacctg agcacccagt ccgccctgag caaagacccc 2220

aacgagaagc gcgatcacat ggtcctgctg gagttcgtga ccgccgccgg gatcactctc 2280

ggcatggacg agctgtacaa gtaa 2304

<210> 24
<211> 2970
<212> DNA
<213> Artificial Sequence

<220>
<223> 21D4 CAR-IRES-GFP

<400> 24
atggccttac cagtgaccgc cttgctcctg ccgctggcct tgctgctcca cgccgccagg 60

ccggccatcc agttgaccca gtctccatcc tccctgtctg catctgtagg agacagagtc 120

accatcactt gccgggcaag tcagggcatt agcagtgctt tagcctggta tcagcagaaa 180

ccagggaaag ctcctaagct cctgatctat gatgcctcca gtttggaaag tggggtccca 240

tcaaggttca gcggcagtgg atctgggaca gatttcactc tcaccatcag cagcctgcag 300

cctgaagatt ttgcaactta ttactgtcaa cagtttaata gttacccgta cacttttggc 360

caggggacca agctggagat caaaggtggc ggtggctcgg gcggtggtgg gtcgggtggc 420

ggcggatctg aggtgcagct ggtgcagtct ggagcagagg tgaaaaagcc cggggagtct 480

ctgaagatct cctgtaaggg ttctggatac agctttagca gcagctggat cggctgggtg 540

cgccagatgc ccgggaaagg cctggagtgg atggggatca tctatcctga tgactctgat 600

accagataca gtccgtcctt ccaaggccag gtcaccatct cagccgacaa gtccatcagg 660

accgcctacc tgcagtggag cagcctgaag gcctcggaca ccgccatgta ttactgtgcg 720

agacatgtta ctatgatttg gggagttatt attgacttct ggggccaggg aaccctggtc 780

accgtctcct cattcgtgcc ggtcttcctg ccagcgaagc ccaccacgac gccagcgccg 840

cgaccaccaa caccggcgcc caccatcgcg tcgcagcccc tgtccctgcg cccagaggcg 900

tgccggccag cggcgggggg cgcagtgcac acgagggggc tggacttcgc ctgtgatatc 960

tacatctggg cgcccttggc cgggacttgt ggggtccttc tcctgtcact ggttatcacc 1020

ctttactgca accacaggaa caggagtaag aggagcaggc tcctgcacag tgactacatg 1080

aacatgactc cccgccgccc cgggcccacc cgcaagcatt accagcccta tgccccacca 1140

cgcgacttcg cagcctatcg ctcccgtttc tctgttgtta aacggggcag aaagaagctc 1200

ctgtatatat tcaaacaacc atttatgaga ccagtacaaa ctactcaaga ggaagatggc 1260

tgtagctgcc gatttccaga agaagaagaa ggaggatgtg aactgagagt gaagttcagc 1320

aggagcgcag acgcccccgc gtaccagcag ggccagaacc agctctataa cgagctcaat 1380

ctaggacgaa gagaggagta cgatgttttg gacaagagac gtggccggga ccctgagatg 1440

gggggaaagc cgagaaggaa gaaccctcag gaaggcctgt acaatgaact gcagaaagat 1500

aagatggcgg aggcctacag tgagattggg atgaaaggcg agcgccggag gggcaagggg 1560

cacgatggcc tttaccaggg tctcagtaca gccaccaagg acacctacga cgcccttcac 1620

atgcaggccc tgccccctcg ctaaggatcc cgcccctctc cctccccccc ccctaacgtt 1680

actggccgaa gccgcttgga ataaggccgg tgtgcgtttg tctatatgtt attttccacc 1740

atattgccgt cttttggcaa tgtgagggcc cggaaacctg gccctgtctt cttgacgagc 1800

attcctaggg gtctttcccc tctcgccaaa ggaatgcaag gtctgttgaa tgtcgtgaag 1860

gaagcagttc ctctggaagc ttcttgaaga caaacaacgt ctgtagcgac cctttgcagg 1920

cagcggaacc ccccacctgg cgacaggtgc ctctgcggcc aaaagccacg tgtataagat 1980

acacctgcaa aggcggcaca accccagtgc cacgttgtga gttggatagt tgtggaaaga 2040

gtcaaatggc tctcctcaag cgtattcaac aaggggctga aggatgccca gaaggtaccc 2100

cattgtatgg gatctgatct ggggcctcgg tgcacatgct ttacatgtgt ttagtcgagg 2160

ttaaaaaaac gtctaggccc cccgaaccac ggggacgtgg ttttcctttg aaaaacacga 2220

tgataagctt gccacaaccc acaatctaga atggtgagca agggcgagga gctgttcacc 2280

ggggtggtgc ccatcctggt cgagctggac ggcgacgtaa acggccacaa gttcagcgtg 2340

tccggcgagg gcgagggcga tgccacctac ggcaagctga ccctgaagtt catctgcacc 2400

accggcaagc tgcccgtgcc ctggcccacc ctcgtgacca ccctgaccta cggcgtgcag 2460

tgcttcagcc gctaccccga ccacatgaag cagcacgact tcttcaagtc cgccatgccc 2520

gaaggctacg tccaggagcg caccatcttc ttcaaggacg acggcaacta caagacccgc 2580

gccgaggtga agttcgaggg cgacaccctg gtgaaccgca tcgagctgaa gggcatcgac 2640

ttcaaggagg acggcaacat cctggggcac aagctggagt acaactacaa cagccacaac 2700

gtctatatca tggccgacaa gcagaagaac ggcatcaagg tgaacttcaa gatccgccac 2760

aacatcgagg acggcagcgt gcagctcgcc gaccactacc agcagaacac ccccatcggc 2820

gacggccccg tgctgctgcc cgacaaccac tacctgagca cccagtccgc cctgagcaaa 2880

gaccccaacg agaagcgcga tcacatggtc ctgctggagt tcgtgaccgc cgccgggatc 2940

actctcggca tggacgagct gtacaagtaa 2970

<210> 25
<211> 1518
<212> DNA
<213> Artificial Sequence

<220>
<223> GPC3 CAR

<400> 25
atggccttac cagtgaccgc cttgctcctg ccgctggcct tgctgctcca cgccgccagg 60

ccggacgtgg tgatgaccca gagcccttta tctttacccg ttacacccgg tgagcccgct 120

agcatctctt gtagaagcag ccagtcttta gtgcacagca acggcaacac atatttacac 180

tggtatttac agaagcccgg tcagagcccc cagctgctga tctacaaggt gagcaatcgt 240

ttctccggcg tgcccgatag attcagcggc agcggctccg gaaccgactt cactttaaag 300

atcagcagag tggaggccga ggacgtgggt gtgtactact gctcccagaa cacccacgtg 360

ccccctacat tcggtcaagg taccaaactg gagatcaaag gtggcggtgg ctcgggcggt 420

ggtgggtcgg gtggcggcgg atctcaagtt cagctggtgc agagcggcgc cgaggtcaaa 480

aaacccggag ccagcgtgaa ggtgtcttgt aaagccagcg gctacacctt taccgactat 540

gagatgcact gggtgagaca agctcccggt caaggtctgg aatggatggg cgctttagac 600

cccaagactg gtgacacagc ctactcccag aagttcaagg gtcgtgtgac tttaacagcc 660

gacgagtcca ccagcacagc ctacatggaa ctgagctctt taaggagcga ggacaccgcc 720

gtgtattact gcactcgttt ctacagctac acctactggg gccaaggtac tttagtgaca 780

gtgagcagct tcgtgccggt cttcctgcca gcgaagccca ccacgacgcc agcgccgcga 840

ccaccaacac cggcgcccac catcgcgtcg cagcccctgt ccctgcgccc agaggcgtgc 900

cggccagcgg cggggggcgc agtgcacacg agggggctgg acttcgcctg tgatatctac 960

atctgggcgc ccttggccgg gacttgtggg gtccttctcc tgtcactggt tatcaccctt 1020

tactgcaacc acaggaaccg tttctctgtt gttaaacggg gcagaaagaa gctcctgtat 1080

atattcaaac aaccatttat gagaccagta caaactactc aagaggaaga tggctgtagc 1140

tgccgatttc cagaagaaga agaaggagga tgtgaactga gagtgaagtt cagcaggagc 1200

gcagacgccc ccgcgtacca gcagggccag aaccagctct ataacgagct caatctagga 1260

cgaagagagg agtacgatgt tttggacaaa agaagaggcc gggaccctga gatgggggga 1320

aagccgagaa ggaagaaccc tcaggaaggc ctgtacaatg aactgcagaa agataagatg 1380

gcggaggcct acagtgagat tgggatgaaa ggcgagcgcc ggaggggcaa ggggcacgat 1440

ggcctttacc agggtctcag tacagccacc aaggacacct acgacgccct tcacatgcag 1500

gccctgcccc ctcgctaa 1518

<210> 26
<211> 3099
<212> DNA
<213> Artificial Sequence

<220>
<223> GPC3 CAR-IRES-TMEM59

<400> 26
atggccttac cagtgaccgc cttgctcctg ccgctggcct tgctgctcca cgccgccagg 60

ccggacgtgg tgatgaccca gagcccttta tctttacccg ttacacccgg tgagcccgct 120

agcatctctt gtagaagcag ccagtcttta gtgcacagca acggcaacac atatttacac 180

tggtatttac agaagcccgg tcagagcccc cagctgctga tctacaaggt gagcaatcgt 240

ttctccggcg tgcccgatag attcagcggc agcggctccg gaaccgactt cactttaaag 300

atcagcagag tggaggccga ggacgtgggt gtgtactact gctcccagaa cacccacgtg 360

ccccctacat tcggtcaagg taccaaactg gagatcaaag gtggcggtgg ctcgggcggt 420

ggtgggtcgg gtggcggcgg atctcaagtt cagctggtgc agagcggcgc cgaggtcaaa 480

aaacccggag ccagcgtgaa ggtgtcttgt aaagccagcg gctacacctt taccgactat 540

gagatgcact gggtgagaca agctcccggt caaggtctgg aatggatggg cgctttagac 600

cccaagactg gtgacacagc ctactcccag aagttcaagg gtcgtgtgac tttaacagcc 660

gacgagtcca ccagcacagc ctacatggaa ctgagctctt taaggagcga ggacaccgcc 720

gtgtattact gcactcgttt ctacagctac acctactggg gccaaggtac tttagtgaca 780

gtgagcagct tcgtgccggt cttcctgcca gcgaagccca ccacgacgcc agcgccgcga 840

ccaccaacac cggcgcccac catcgcgtcg cagcccctgt ccctgcgccc agaggcgtgc 900

cggccagcgg cggggggcgc agtgcacacg agggggctgg acttcgcctg tgatatctac 960

atctgggcgc ccttggccgg gacttgtggg gtccttctcc tgtcactggt tatcaccctt 1020

tactgcaacc acaggaaccg tttctctgtt gttaaacggg gcagaaagaa gctcctgtat 1080

atattcaaac aaccatttat gagaccagta caaactactc aagaggaaga tggctgtagc 1140

tgccgatttc cagaagaaga agaaggagga tgtgaactga gagtgaagtt cagcaggagc 1200

gcagacgccc ccgcgtacca gcagggccag aaccagctct ataacgagct caatctagga 1260

cgaagagagg agtacgatgt tttggacaaa agaagaggcc gggaccctga gatgggggga 1320

aagccgagaa ggaagaaccc tcaggaaggc ctgtacaatg aactgcagaa agataagatg 1380

gcggaggcct acagtgagat tgggatgaaa ggcgagcgcc ggaggggcaa ggggcacgat 1440

ggcctttacc agggtctcag tacagccacc aaggacacct acgacgccct tcacatgcag 1500

gccctgcccc ctcgctaagg atcccgcccc tctccctccc ccccccctaa cgttactggc 1560

cgaagccgct tggaataagg ccggtgtgcg tttgtctata tgttattttc caccatattg 1620

ccgtcttttg gcaatgtgag ggcccggaaa cctggccctg tcttcttgac gagcattcct 1680

aggggtcttt cccctctcgc caaaggaatg caaggtctgt tgaatgtcgt gaaggaagca 1740

gttcctctgg aagcttcttg aagacaaaca acgtctgtag cgaccctttg caggcagcgg 1800

aaccccccac ctggcgacag gtgcctctgc ggccaaaagc cacgtgtata agatacacct 1860

gcaaaggcgg cacaacccca gtgccacgtt gtgagttgga tagttgtgga aagagtcaaa 1920

tggctctcct caagcgtatt caacaagggg ctgaaggatg cccagaaggt accccattgt 1980

atgggatctg atctggggcc tcggtgcaca tgctttacat gtgtttagtc gaggttaaaa 2040

aaacgtctag gccccccgaa ccacggggac gtggttttcc tttgaaaaac acgatgataa 2100

gcttgccaca acccacaatc tagaatggcg gcgccgaagg ggagcctctg ggtgaggacc 2160

caactggggc tcccgccgct gctgctgctg accatggcct tggccggagg ttcggggacc 2220

gcttcggctg aagcatttga ctcggtcttg ggtgatacgg cgtcttgcca ccgggcctgt 2280

cagttgacct accccttgca cacctaccct aaggaagagg agttgtacgc atgtcagaga 2340

ggttgcaggc tgttttcaat ttgtcagttt gtggatgatg gaattgactt aaatcgaact 2400

aaattggaat gtgaatctgc atgtacagaa gcatattccc aatctgatga gcaatatgct 2460

tgccatcttg gttgccagaa tcagctgcca ttcgctgaac tgagacaaga acaacttatg 2520

tccctgatgc caaaaatgca cctactcttt cctctaactc tggtgaggtc attctggagt 2580

gacatgatgg actccgcaca gagcttcata acctcttcat ggacttttta tcttcaagcc 2640

gatgacggaa aaatagttat attccagtct aagccagaaa tccagtacgc accacatttg 2700

gagcaggagc ctacaaattt gagagaatca tctctaagca aaatgtcctc agatctgcaa 2760

atgagaaatt cacaagcgca caggaatttt cttgaagatg gagaaagtga tggcttttta 2820

agatgcctct ctcttaactc tgggtggatt ttaactacaa ctcttgtcct ctcggtgatg 2880

gtattgcttt ggatttgttg tgcaactgtt gctacagctg tggagcagta tgttccctct 2940

gagaagctga gtatctatgg tgacttggag tttatgaatg aacaaaagct aaacagatat 3000

ccagcttctt ctcttgtggt tgttagatct aaaactgaag atcatgaaga agcagggcct 3060

ctacctacaa aagtgaatct tgctcattct gaaatttaa 3099

<210> 27
<211> 2844
<212> DNA
<213> Artificial Sequence

<220>
<223> gpc3 car-ires-gfp

<400> 27
atggccttac cagtgaccgc cttgctcctg ccgctggcct tgctgctcca cgccgccagg 60

ccggacgtgg tgatgaccca gagcccttta tctttacccg ttacacccgg tgagcccgct 120

agcatctctt gtagaagcag ccagtcttta gtgcacagca acggcaacac atatttacac 180

tggtatttac agaagcccgg tcagagcccc cagctgctga tctacaaggt gagcaatcgt 240

ttctccggcg tgcccgatag attcagcggc agcggctccg gaaccgactt cactttaaag 300

atcagcagag tggaggccga ggacgtgggt gtgtactact gctcccagaa cacccacgtg 360

ccccctacat tcggtcaagg taccaaactg gagatcaaag gtggcggtgg ctcgggcggt 420

ggtgggtcgg gtggcggcgg atctcaagtt cagctggtgc agagcggcgc cgaggtcaaa 480

aaacccggag ccagcgtgaa ggtgtcttgt aaagccagcg gctacacctt taccgactat 540

gagatgcact gggtgagaca agctcccggt caaggtctgg aatggatggg cgctttagac 600

cccaagactg gtgacacagc ctactcccag aagttcaagg gtcgtgtgac tttaacagcc 660

gacgagtcca ccagcacagc ctacatggaa ctgagctctt taaggagcga ggacaccgcc 720

gtgtattact gcactcgttt ctacagctac acctactggg gccaaggtac tttagtgaca 780

gtgagcagct tcgtgccggt cttcctgcca gcgaagccca ccacgacgcc agcgccgcga 840

ccaccaacac cggcgcccac catcgcgtcg cagcccctgt ccctgcgccc agaggcgtgc 900

cggccagcgg cggggggcgc agtgcacacg agggggctgg acttcgcctg tgatatctac 960

atctgggcgc ccttggccgg gacttgtggg gtccttctcc tgtcactggt tatcaccctt 1020

tactgcaacc acaggaaccg tttctctgtt gttaaacggg gcagaaagaa gctcctgtat 1080

atattcaaac aaccatttat gagaccagta caaactactc aagaggaaga tggctgtagc 1140

tgccgatttc cagaagaaga agaaggagga tgtgaactga gagtgaagtt cagcaggagc 1200

gcagacgccc ccgcgtacca gcagggccag aaccagctct ataacgagct caatctagga 1260

cgaagagagg agtacgatgt tttggacaaa agaagaggcc gggaccctga gatgggggga 1320

aagccgagaa ggaagaaccc tcaggaaggc ctgtacaatg aactgcagaa agataagatg 1380

gcggaggcct acagtgagat tgggatgaaa ggcgagcgcc ggaggggcaa ggggcacgat 1440

ggcctttacc agggtctcag tacagccacc aaggacacct acgacgccct tcacatgcag 1500

gccctgcccc ctcgctaagg atcccgcccc tctccctccc ccccccctaa cgttactggc 1560

cgaagccgct tggaataagg ccggtgtgcg tttgtctata tgttattttc caccatattg 1620

ccgtcttttg gcaatgtgag ggcccggaaa cctggccctg tcttcttgac gagcattcct 1680

aggggtcttt cccctctcgc caaaggaatg caaggtctgt tgaatgtcgt gaaggaagca 1740

gttcctctgg aagcttcttg aagacaaaca acgtctgtag cgaccctttg caggcagcgg 1800

aaccccccac ctggcgacag gtgcctctgc ggccaaaagc cacgtgtata agatacacct 1860

gcaaaggcgg cacaacccca gtgccacgtt gtgagttgga tagttgtgga aagagtcaaa 1920

tggctctcct caagcgtatt caacaagggg ctgaaggatg cccagaaggt accccattgt 1980

atgggatctg atctggggcc tcggtgcaca tgctttacat gtgtttagtc gaggttaaaa 2040

aaacgtctag gccccccgaa ccacggggac gtggttttcc tttgaaaaac acgatgataa 2100

gcttgccaca acccacaatc tagaatggtg agcaagggcg aggagctgtt caccggggtg 2160

gtgcccatcc tggtcgagct ggacggcgac gtaaacggcc acaagttcag cgtgtccggc 2220

gagggcgagg gcgatgccac ctacggcaag ctgaccctga agttcatctg caccaccggc 2280

aagctgcccg tgccctggcc caccctcgtg accaccctga cctacggcgt gcagtgcttc 2340

agccgctacc ccgaccacat gaagcagcac gacttcttca agtccgccat gcccgaaggc 2400

tacgtccagg agcgcaccat cttcttcaag gacgacggca actacaagac ccgcgccgag 2460

gtgaagttcg agggcgacac cctggtgaac cgcatcgagc tgaagggcat cgacttcaag 2520

gaggacggca acatcctggg gcacaagctg gagtacaact acaacagcca caacgtctat 2580

atcatggccg acaagcagaa gaacggcatc aaggtgaact tcaagatccg ccacaacatc 2640

gaggacggca gcgtgcagct cgccgaccac taccagcaga acacccccat cggcgacggc 2700

cccgtgctgc tgcccgacaa ccactacctg agcacccagt ccgccctgag caaagacccc 2760

aacgagaagc gcgatcacat ggtcctgctg gagttcgtga ccgccgccgg gatcactctc 2820

ggcatggacg agctgtacaa gtaa 2844

<210> 28
<211> 2202

<212>  DNA
<213>  Artificial Sequence

<220>
<223>  CD19CD22 CAR

<400>  28
atgctgctgc tcgtgacaag cctgctgctg tgcgagctgc cccaccctgc ctttctgctg      60

atccccgaca tccagatgac ccagaccacc agcagcctga gcgccagcct gggcgataga     120

gtgaccatca gctgcagagc cagccaggac atcagcaagt acctgaactg gtatcagcag     180

aaacccgacg gcaccgtgaa gctgctgatc taccacacca gcagactgca cagcggcgtg     240

cccagcagat tttctggcag cggctccggc accgactaca gcctgaccat ctccaacctg     300

gaacaggaag atatcgctac ctacttctgt cagcaaggca acaccctgcc ctacaccttc     360

ggcggaggca ccaagctgga aatcacaggc ggcggaggat cccaggtgca gctgcagcag     420

tctggacccg gcctcgtgaa gcctagccag accctgtctc tgacctgcgc catcagcggc     480

gatagcgtgt ccagcaatag cgccgcctgg aactggatcc ggcagagccc ttctagaggc     540

ctggaatggc tgggccggac ctactaccgg tccaagtggt acaacgacta cgccgtgtcc     600

gtgaagtccc ggatcaccat caaccccgac accagcaaga accagttctc cctgcagctg     660

aacagcgtga cccccgagga taccgccgtg tactactgcg ccagagaagt gaccggcgac     720

ctggaagatg ccttcgacat ctggggccag ggcacaatgg tcaccgtgtc tagcggcagc     780

acaagcggct ctggcaagcc tggatctggc gagggctcta ccaagggcga tattcagatg     840

acacagagcc cctccagcct gtccgcctct gtgggagaca gagtgacaat cacctgtcgg     900

gcctcccaga ccatctggtc ctatctgaat tggtatcagc agcggcctgg caaggccccc     960

aacctgctga tctatgccgc cagctctctg cagtccggcg tgccatctag attcagcggc    1020

agaggcagcg gcaccgattt caccctgaca attagcagtc tgcaggccga ggacttcgcc    1080

acctactatt gccagcagag ctacagcatc ccccagacct tcggccaggg aacaaaactg    1140

gaaatcaaag ggggaggcgg cagcgaagtg aaactgcagg aatctggccc tggcctggtg    1200

gccccaagcc agtctctgag cgtgacctgt accgtgtctg gcgtgtccct gcccgattac    1260

ggcgtgtcct ggatcagaca gccccccaga aagggactgg aatggctggg agtgatctgg    1320

ggcagcgaga caacctacta caacagcgcc ctgaagtcca ggctgaccat catcaaggac    1380

aactccaaga gccaggtgtt cctgaagatg aattccctgc agaccgacga caccgccatc    1440

tattactgtg ccaagcacta ctactacggc ggcagctacg ccatggacta ctggggacag    1500

ggaacctccg tgaccgtgtc ctcttccgga accacgacgc cagcgccgcg accaccaaca    1560

ccggcgccca ccatcgcgtc gcagcccctg tccctgcgcc cagaggcgtg ccggccagcg    1620

gcggggggcg cagtgcacac gagggggctg gacttcgcct gtgatatcta catctgggcg    1680

cccttggccg ggacttgtgg ggtccttctc ctgtcactgg ttatcaccct ttactgcaaa 1740

cggggcagaa agaaactcct gtatatattc aaacaaccat ttatgagacc agtacaaact 1800

actcaagagg aagatggctg tagctgccga tttccagaag aagaagaagg aggatgtgaa 1860

ctgagagtga agttcagcag gagcgcagac gcccccgcgt acaagcaggg ccagaaccag 1920

ctctataacg agctcaatct aggacgaaga gaggagtacg atgttttgga caagagacgt 1980

ggccgggacc ctgagatggg gggaaagccg agaaggaaga accctcagga aggcctgtac 2040

aatgaactgc agaaagataa gatggcggag gcctacagtg agattgggat gaaaggcgag 2100

cgccggaggg gcaaggggca cgatggcctt taccagggtc tcagtacagc caccaaggac 2160

acctacgacg cccttcacat gcaggccctg ccccctcgct aa 2202

<210> 29
<211> 3783
<212> DNA
<213> Artificial Sequence

<220>
<223> CD19CD22 CAR-IRES-TMEM59

<400> 29
atgctgctgc tcgtgacaag cctgctgctg tgcgagctgc cccaccctgc ctttctgctg 60

atccccgaca tccagatgac ccagaccacc agcagcctga gcgccagcct gggcgataga 120

gtgaccatca gctgcagagc cagccaggac atcagcaagt acctgaactg gtatcagcag 180

aaacccgacg gcaccgtgaa gctgctgatc taccacacca gcagactgca cagcggcgtg 240

cccagcagat tttctggcag cggctccggc accgactaca gcctgaccat ctccaacctg 300

gaacaggaag atatcgctac ctacttctgt cagcaaggca acaccctgcc ctacaccttc 360

ggcggaggca ccaagctgga aatcacaggc ggcggaggat cccaggtgca gctgcagcag 420

tctggacccg gcctcgtgaa gcctagccag accctgtctc tgacctgcgc catcagcggc 480

gatagcgtgt ccagcaatag cgccgcctgg aactggatcc ggcagagccc ttctagaggc 540

ctggaatggc tgggccggac ctactaccgg tccaagtggt acaacgacta cgccgtgtcc 600

gtgaagtccc ggatcaccat caaccccgac accagcaaga accagttctc cctgcagctg 660

aacagcgtga cccccgagga taccgccgtg tactactgcg ccagagaagt gaccggcgac 720

ctggaagatg ccttcgacat ctggggccag ggcacaatgg tcaccgtgtc tagcggcagc 780

acaagcggct ctggcaagcc tggatctggc gagggctcta ccaagggcga tattcagatg 840

acacagagcc cctccagcct gtccgcctct gtgggagaca gagtgacaat cacctgtcgg 900

gcctcccaga ccatctggtc ctatctgaat tggtatcagc agcggcctgg caaggccccc 960

aacctgctga tctatgccgc cagctctctg cagtccggcg tgccatctag attcagcggc 1020

agaggcagcg gcaccgattt caccctgaca attagcagtc tgcaggccga ggacttcgcc 1080

acctactatt gccagcagag ctacagcatc ccccagacct tcggccaggg aacaaaactg 1140

gaaatcaaag ggggaggcgg cagcgaagtg aaactgcagg aatctggccc tggcctggtg 1200

gccccaagcc agtctctgag cgtgacctgt accgtgtctg gcgtgtccct gcccgattac 1260

ggcgtgtcct ggatcagaca gccccccaga aagggactgg aatggctggg agtgatctgg 1320

ggcagcgaga caacctacta caacagcgcc ctgaagtcca ggctgaccat catcaaggac 1380

aactccaaga gccaggtgtt cctgaagatg aattccctgc agaccgacga caccgccatc 1440

tattactgtg ccaagcacta ctactacggc ggcagctacg ccatggacta ctggggacag 1500

ggaacctccg tgaccgtgtc ctcttccgga accacgacgc cagcgccgcg accaccaaca 1560

ccggcgccca ccatcgcgtc gcagcccctg tccctgcgcc cagaggcgtg ccggccagcg 1620

gcggggggcg cagtgcacac gagggggctg gacttcgcct gtgatatcta catctgggcg 1680

cccttggccg ggacttgtgg ggtccttctc ctgtcactgg ttatcaccct ttactgcaaa 1740

cggggcagaa agaaactcct gtatatattc aaacaaccat ttatgagacc agtacaaact 1800

actcaagagg aagatggctg tagctgccga tttccagaag aagaagaagg aggatgtgaa 1860

ctgagagtga agttcagcag gagcgcagac gcccccgcgt acaagcaggg ccagaaccag 1920

ctctataacg agctcaatct aggacgaaga gaggagtacg atgttttgga caagagacgt 1980

ggccgggacc ctgagatggg gggaaagccg agaaggaaga accctcagga aggcctgtac 2040

aatgaactgc agaaagataa gatggcggag gcctacagtg agattgggat gaaaggcgag 2100

cgccggaggg gcaaggggca cgatggcctt taccagggtc tcagtacagc caccaaggac 2160

acctacgacg cccttcacat gcaggccctg ccccctcgct aaggatcccg cccctctccc 2220

tccccccccc ctaacgttac tggccgaagc cgcttggaat aaggccggtg tgcgtttgtc 2280

tatatgttat tttccaccat attgccgtct tttggcaatg tgagggcccg gaaacctggc 2340

cctgtcttct tgacgagcat tcctaggggt ctttcccctc tcgccaaagg aatgcaaggt 2400

ctgttgaatg tcgtgaagga agcagttcct ctggaagctt cttgaagaca aacaacgtct 2460

gtagcgaccc tttgcaggca gcggaacccc ccacctggcg acaggtgcct ctgcggccaa 2520

aagccacgtg tataagatac acctgcaaag gcggcacaac cccagtgcca cgttgtgagt 2580

tggatagttg tggaaagagt caaatggctc tcctcaagcg tattcaacaa ggggctgaag 2640

gatgcccaga aggtacccca ttgtatggga tctgatctgg ggcctcggtg cacatgcttt 2700

acatgtgttt agtcgaggtt aaaaaaacgt ctaggccccc cgaaccacgg ggacgtggtt 2760

ttcctttgaa aaacacgatg ataagcttgc cacaacccac aatctagaat ggcggcgccg 2820

aaggggagcc tctgggtgag gacccaactg gggctcccgc cgctgctgct gctgaccatg 2880

gccttggccg gaggttcggg gaccgcttcg gctgaagcat ttgactcggt cttgggtgat 2940

acggcgtctt gccaccgggc ctgtcagttg acctacccct tgcacaccta ccctaaggaa 3000

gaggagttgt acgcatgtca gagaggttgc aggctgtttt caatttgtca gtttgtggat 3060

gatggaattg acttaaatcg aactaaattg gaatgtgaat ctgcatgtac agaagcatat 3120

tcccaatctg atgagcaata tgcttgccat cttggttgcc agaatcagct gccattcgct 3180

gaactgagac aagaacaact tatgtccctg atgccaaaaa tgcacctact ctttcctcta 3240

actctggtga ggtcattctg gagtgacatg atggactccg cacagagctt cataacctct 3300

tcatggactt tttatcttca agccgatgac ggaaaaatag ttatattcca gtctaagcca 3360

gaaatccagt acgcaccaca tttggagcag gagcctacaa atttgagaga atcatctcta 3420

agcaaaatgt cctcagatct gcaaatgaga aattcacaag cgcacaggaa ttttcttgaa 3480

gatggagaaa gtgatggctt tttaagatgc ctctctctta actctgggtg gattttaact 3540

acaactcttg tcctctcggt gatggtattg ctttggattt gttgtgcaac tgttgctaca 3600

gctgtggagc agtatgttcc ctctgagaag ctgagtatct atggtgactt ggagtttatg 3660

aatgaacaaa agctaaacag atatccagct tcttctcttg tggttgttag atctaaaact 3720

gaagatcatg aagaagcagg gcctctacct acaaaagtga atcttgctca ttctgaaatt 3780

taa 3783

<210> 30
<211> 3528
<212> DNA
<213> Artificial Sequence

<220>
<223> CD19CD22 CAR-IRES-GFP

<400> 30
atgctgctgc tcgtgacaag cctgctgctg tgcgagctgc cccaccctgc ctttctgctg 60

atccccgaca tccagatgac ccagaccacc agcagcctga gcgccagcct gggcgataga 120

gtgaccatca gctgcagagc cagccaggac atcagcaagt acctgaactg gtatcagcag 180

aaacccgacg gcaccgtgaa gctgctgatc taccacacca gcagactgca cagcggcgtg 240

cccagcagat tttctggcag cggctccggc accgactaca gcctgaccat ctccaacctg 300

gaacaggaag atatcgctac ctacttctgt cagcaaggca acaccctgcc ctacaccttc 360

ggcggaggca ccaagctgga aatcacaggc ggcggaggat cccaggtgca gctgcagcag 420

tctggacccg gcctcgtgaa gcctagccag accctgtctc tgacctgcgc catcagcggc 480

gatagcgtgt ccagcaatag cgccgcctgg aactggatcc ggcagagccc ttctagaggc 540

ctggaatggc tgggccggac ctactaccgg tccaagtggt acaacgacta cgccgtgtcc 600

gtgaagtccc ggatcaccat caaccccgac accagcaaga accagttctc cctgcagctg 660

aacagcgtga cccccgagga taccgccgtg tactactgcg ccagagaagt gaccggcgac 720

ctggaagatg ccttcgacat ctggggccag ggcacaatgg tcaccgtgtc tagcggcagc 780

acaagcggct ctggcaagcc tggatctggc gagggctcta ccaagggcga tattcagatg 840

acacagagcc cctccagcct gtccgcctct gtgggagaca gagtgacaat cacctgtcgg 900

gcctcccaga ccatctggtc ctatctgaat tggtatcagc agcggcctgg caaggccccc 960

aacctgctga tctatgccgc cagctctctg cagtccggcg tgccatctag attcagcggc 1020

agaggcagcg gcaccgattt caccctgaca attagcagtc tgcaggccga ggacttcgcc 1080

acctactatt gccagcagag ctacagcatc ccccagacct tcggccaggg aacaaaactg 1140

gaaatcaaag ggggaggcgg cagcgaagtg aaactgcagg aatctggccc tggcctggtg 1200

gccccaagcc agtctctgag cgtgacctgt accgtgtctg gcgtgtccct gcccgattac 1260

ggcgtgtcct ggatcagaca gccccccaga aagggactgg aatggctggg agtgatctgg 1320

ggcagcgaga caacctacta caacagcgcc ctgaagtcca ggctgaccat catcaaggac 1380

aactccaaga gccaggtgtt cctgaagatg aattccctgc agaccgacga caccgccatc 1440

tattactgtg ccaagcacta ctactacggc ggcagctacg ccatggacta ctggggacag 1500

ggaacctccg tgaccgtgtc ctcttccgga accacgacgc cagcgccgcg accaccaaca 1560

ccggcgccca ccatcgcgtc gcagcccctg tccctgcgcc cagaggcgtg ccggccagcg 1620

gcggggggcg cagtgcacac gagggggctg gacttcgcct gtgatatcta catctgggcg 1680

cccttggccg ggacttgtgg ggtccttctc ctgtcactgg ttatcaccct ttactgcaaa 1740

cggggcagaa agaaactcct gtatatattc aaacaaccat ttatgagacc agtacaaact 1800

actcaagagg aagatggctg tagctgccga tttccagaag aagaagaagg aggatgtgaa 1860

ctgagagtga agttcagcag gagcgcagac gcccccgcgt acaagcaggg ccagaaccag 1920

ctctataacg agctcaatct aggacgaaga gaggagtacg atgttttgga caagagacgt 1980

ggccgggacc ctgagatggg gggaaagccg agaaggaaga accctcagga aggcctgtac 2040

aatgaactgc agaaagataa gatggcggag gcctacagtg agattgggat gaaaggcgag 2100

cgccggaggg gcaaggggca cgatggcctt taccagggtc tcagtacagc caccaaggac 2160

acctacgacg cccttcacat gcaggccctg ccccctcgct aaggatcccg cccctctccc 2220

tccccccccc ctaacgttac tggccgaagc cgcttggaat aaggccggtg tgcgtttgtc 2280

tatatgttat tttccaccat attgccgtct tttggcaatg tgagggcccg gaaacctggc 2340

cctgtcttct tgacgagcat tcctaggggt ctttcccctc tcgccaaagg aatgcaaggt 2400

ctgttgaatg tcgtgaagga agcagttcct ctggaagctt cttgaagaca aacaacgtct 2460

gtagcgaccc tttgcaggca gcggaacccc ccacctggcg acaggtgcct ctgcggccaa 2520

aagccacgtg tataagatac acctgcaaag gcggcacaac cccagtgcca cgttgtgagt 2580

tggatagttg tggaaagagt caaatggctc tcctcaagcg tattcaacaa ggggctgaag 2640

gatgcccaga aggtacccca ttgtatggga tctgatctgg ggcctcggtg cacatgcttt 2700

acatgtgttt agtcgaggtt aaaaaaacgt ctaggccccc cgaaccacgg ggacgtggtt 2760

ttcctttgaa aaacacgatg ataagcttgc cacaacccac aatctagaat ggtgagcaag 2820

ggcgaggagc tgttcaccgg ggtggtgccc atcctggtcg agctggacgg cgacgtaaac 2880

ggccacaagt tcagcgtgtc cggcgagggc gagggcgatg ccacctacgg caagctgacc 2940

ctgaagttca tctgcaccac cggcaagctg cccgtgccct ggcccaccct cgtgaccacc 3000

ctgacctacg gcgtgcagtg cttcagccgc taccccgacc acatgaagca gcacgacttc 3060

ttcaagtccg ccatgcccga aggctacgtc caggagcgca ccatcttctt caaggacgac 3120

ggcaactaca agacccgcgc cgaggtgaag ttcgagggcg acaccctggt gaaccgcatc 3180

gagctgaagg gcatcgactt caaggaggac ggcaacatcc tggggcacaa gctggagtac 3240

aactacaaca gccacaacgt ctatatcatg gccgacaagc agaagaacgg catcaaggtg 3300

aacttcaaga tccgccacaa catcgaggac ggcagcgtgc agctcgccga ccactaccag 3360

cagaacaccc ccatcggcga cggccccgtg ctgctgcccg acaaccacta cctgagcacc 3420

cagtccgccc tgagcaaaga ccccaacgag aagcgcgatc acatggtcct gctggagttc 3480

gtgaccgccg ccgggatcac tctcggcatg gacgagctgt acaagtaa 3528

<210> 31
<211> 2202
<212> DNA
<213> Artificial Sequence

<220>
<223> CD19CD22-59TM CAR

<400> 31
atgctgctgc tggtgacatc tctgctcctc tgcgaactcc ctcatcccgc ctttctgctg 60

atccccgaca tccagatgac acagaccaca tcctctctgt ccgcctctct gggagatagg 120

gtgacaatca gctgtagagc ttcccaagac atctccaagt atctgaactg gtaccagcag 180

aaacccgatg gaaccgtcaa actgctgatt taccatacct ctagactgca ctccggagtg 240

ccttccagat ttagcggcag cggcagcggc acagactaca gcctcaccat ctccaatctg 300

gagcaagagg acattgctac ctacttctgt cagcaaggca acacactgcc ttacaccttc 360

ggcggcggca ccaaactgga gatcaccggc ggcggaggaa gccaagtgca gctgcagcag 420

agcggccccg gcctcgtgaa acccagccag accctctctc tgacatgcgc tatttccggc 480

gatagcgtga gctccaactc cgccgcttgg aactggatta ggcagagccc ctctagagga 540

ctggaatggc tgggaaggac ctactataga agcaaatggt acaacgacta cgccgtctcc 600

gtgaagtcca gaatcaccat caatcccgac acatccaaga atcagttctc cctccagctg 660

aacagcgtca cacccgagga caccgccgtg tactactgcg ctagagaggt gaccggcgat 720

ctggaggacg ctttcgacat ctggggccaa ggaacaatgg tcaccgtcag cagcggaagc 780

acaagcggca gcggcaaacc cggaagcgga gagggcagca ccaagggaga cattcaaatg 840

acccagtccc ccagctctct gagcgcttcc gtgggagata gagtgaccat tacatgcaga 900

gccagccaga caatctggtc ctatctgaac tggtatcagc agaggcccgg aaaagccccc 960

aatctgctga tctacgccgc tagctctctg caatccggag tgccctctag attttccgga 1020

aggggaagcg gaaccgactt caccctcacc atttcctctc tgcaagccga ggatttcgct 1080

acatactact gccagcagtc ctattccatt ccccagacct ttggccaagg caccaagctg 1140

gaaattaaag gcggaggagg ctccgaggtc aagctccaag aaagcggacc cggactggtc 1200

gctcctagcc agagcctcag cgtcacatgc accgtgtccg gagtgtccct ccccgactac 1260

ggagtgagct ggattagaca gccccctagg aaaggactgg agtggctggg cgtgatctgg 1320

ggctccgaga ccacctacta caatagcgct ctgaagtcta gactcacaat cattaaggac 1380

aacagcaaat cccaagtgtt cctcaagatg aatagcctcc agaccgacga tacagccatc 1440

tattactgcg ccaagcacta ctactacggc ggcagctacg ccatggacta ttggggacaa 1500

ggcacatccg tcacagtcag cagcccacat ttggagcagg agcctacaaa tttgagagaa 1560

tcatctctaa gcaaaatgtc ctcagatctg caaatgagaa attcacaagc gcacaggaat 1620

tttcttgaag atggagaaag tgatggcttt ttaagatgcc tctctcttaa ctctgggtgg 1680

attttaacta caactcttgt cctctcggtg atggtattgc tttggatttg ttgtgcaaaa 1740

cggggcagaa agaaactcct gtatatattc aaacaaccat ttatgagacc agtacaaact 1800

actcaagagg aagatggctg tagctgccga tttccagaag aagaagaagg aggatgtgaa 1860

ctgagagtga agttcagcag gagcgcagac gcccccgcgt acaagcaggg ccagaaccag 1920

ctctataacg agctcaatct aggacgaaga gaggagtacg atgttttgga caagagacgt 1980

ggccgggacc ctgagatggg gggaaagccg agaaggaaga accctcagga aggcctgtac 2040

aatgaactgc agaaagataa gatggcggag gcctacagtg agattgggat gaaaggcgag 2100

cgccggaggg gcaaggggca cgatggcctt taccagggtc tcagtacagc caccaaggac 2160

acctacgacg cccttcacat gcaggccctg ccccctcgct aa 2202

<210> 32
<211> 3528
<212> DNA
<213> Artificial Sequence

<220>
<223> CD19CD22-59TM CAR-IRES-GFP

<400> 32

atgctgctgc tggtgacatc tctgctcctc tgcgaactcc ctcatcccgc ctttctgctg 60

atccccgaca tccagatgac acagaccaca tcctctctgt ccgcctctct gggagatagg 120

gtgacaatca gctgtagagc ttcccaagac atctccaagt atctgaactg gtaccagcag 180

aaacccgatg gaaccgtcaa actgctgatt taccatacct ctagactgca ctccggagtg 240

ccttccagat ttagcggcag cggcagcggc acagactaca gcctcaccat ctccaatctg 300

gagcaagagg acattgctac ctacttctgt cagcaaggca acacactgcc ttacaccttc 360

ggcggcggca ccaaactgga gatcaccggc ggcggaggaa gccaagtgca gctgcagcag 420

agcggccccg gcctcgtgaa acccagccag accctctctc tgacatgcgc tatttccggc 480

gatagcgtga gctccaactc cgccgcttgg aactggatta ggcagagccc ctctagagga 540

ctggaatggc tgggaaggac ctactataga agcaaatggt acaacgacta cgccgtctcc 600

gtgaagtcca gaatcaccat caatcccgac acatccaaga atcagttctc cctccagctg 660

aacagcgtca cacccgagga caccgccgtg tactactgcg ctagagaggt gaccggcgat 720

ctggaggacg ctttcgacat ctggggccaa ggaacaatgg tcaccgtcag cagcggaagc 780

acaagcggca gcggcaaacc cggaagcgga gagggcagca ccaagggaga cattcaaatg 840

acccagtccc ccagctctct gagcgcttcc gtgggagata gagtgaccat tacatgcaga 900

gccagccaga caatctggtc ctatctgaac tggtatcagc agaggcccgg aaaagccccc 960

aatctgctga tctacgccgc tagctctctg caatccggag tgccctctag attttccgga 1020

aggggaagcg gaaccgactt caccctcacc atttcctctc tgcaagccga ggatttcgct 1080

acatactact gccagcagtc ctattccatt ccccagacct ttggccaagg caccaagctg 1140

gaaattaaag gcggaggagg ctccgaggtc aagctccaag aaagcggacc cggactggtc 1200

gctcctagcc agagcctcag cgtcacatgc accgtgtccg gagtgtccct ccccgactac 1260

ggagtgagct ggattagaca gccccctagg aaaggactgg agtggctggg cgtgatctgg 1320

ggctccgaga ccacctacta caatagcgct ctgaagtcta gactcacaat cattaaggac 1380

aacagcaaat cccaagtgtt cctcaagatg aatagcctcc agaccgacga tacagccatc 1440

tattactgcg ccaagcacta ctactacggc ggcagctacg ccatggacta ttggggacaa 1500

ggcacatccg tcacagtcag cagcccacat ttggagcagg agcctacaaa tttgagagaa 1560

tcatctctaa gcaaaatgtc ctcagatctg caaatgagaa attcacaagc gcacaggaat 1620

tttcttgaag atggagaaag tgatggcttt ttaagatgcc tctctcttaa ctctgggtgg 1680

attttaacta caactcttgt cctctcggtg atggtattgc tttggatttg ttgtgcaaaa 1740

cggggcagaa agaaactcct gtatatattc aaacaaccat ttatgagacc agtacaaact 1800

actcaagagg aagatggctg tagctgccga tttccagaag aagaagaagg aggatgtgaa 1860

ctgagagtga agttcagcag gagcgcagac gcccccgcgt acaagcaggg ccagaaccag 1920

ctctataacg agctcaatct aggacgaaga gaggagtacg atgttttgga caagagacgt 1980

ggccgggacc ctgagatggg gggaaagccg agaaggaaga accctcagga aggcctgtac 2040

aatgaactgc agaaagataa gatggcggag gcctacagtg agattgggat gaaaggcgag 2100

cgccggaggg gcaaggggca cgatggcctt taccagggtc tcagtacagc caccaaggac 2160

acctacgacg cccttcacat gcaggccctg ccccctcgct aaggatcccg cccctctccc 2220

tccccccccc ctaacgttac tggccgaagc cgcttggaat aaggccggtg tgcgtttgtc 2280

tatatgttat tttccaccat attgccgtct tttggcaatg tgagggcccg gaaacctggc 2340

cctgtcttct tgacgagcat tcctaggggt ctttcccctc tcgccaaagg aatgcaaggt 2400

ctgttgaatg tcgtgaagga agcagttcct ctggaagctt cttgaagaca aacaacgtct 2460

gtagcgaccc tttgcaggca gcggaacccc ccacctggcg acaggtgcct ctgcggccaa 2520

aagccacgtg tataagatac acctgcaaag gcggcacaac cccagtgcca cgttgtgagt 2580

tggatagttg tggaaagagt caaatggctc tcctcaagcg tattcaacaa ggggctgaag 2640

gatgcccaga aggtacccca ttgtatggga tctgatctgg ggcctcggtg cacatgcttt 2700

acatgtgttt agtcgaggtt aaaaaaacgt ctaggccccc cgaaccacgg ggacgtggtt 2760

ttcctttgaa aaacacgatg ataagcttgc cacaacccac aatctagaat ggtgagcaag 2820

ggcgaggagc tgttcaccgg ggtggtgccc atcctggtcg agctggacgg cgacgtaaac 2880

ggccacaagt tcagcgtgtc cggcgagggc gagggcgatg ccacctacgg caagctgacc 2940

ctgaagttca tctgcaccac cggcaagctg cccgtgccct ggcccaccct cgtgaccacc 3000

ctgacctacg gcgtgcagtg cttcagccgc taccccgacc acatgaagca gcacgacttc 3060

ttcaagtccg ccatgcccga aggctacgtc caggagcgca ccatcttctt caaggacgac 3120

ggcaactaca agacccgcgc cgaggtgaag ttcgagggcg acaccctggt gaaccgcatc 3180

gagctgaagg gcatcgactt caaggaggac ggcaacatcc tggggcacaa gctggagtac 3240

aactacaaca gccacaacgt ctatatcatg gccgacaagc agaagaacgg catcaaggtg 3300

aacttcaaga tccgccacaa catcgaggac ggcagcgtgc agctcgccga ccactaccag 3360

cagaacaccc ccatcggcga cggccccgtg ctgctgcccg acaaccacta cctgagcacc 3420

cagtccgccc tgagcaaaga ccccaacgag aagcgcgatc acatggtcct gctggagttc 3480

gtgaccgccg ccgggatcac tctcggcatg gacgagctgt acaagtaa 3528

<210> 33
<211> 1470
<212> DNA
<213> Artificial Sequence

<220>
<223> 21D4-59TM CAR

<400>  33
atggccttac cagtgaccgc cttgctcctg ccgctggcct tgctgctcca cgccgccagg      60

ccggccatcc agttgaccca gtctccatcc tccctgtctg catctgtagg agacagagtc     120

accatcactt gccgggcaag tcagggcatt agcagtgctt tagcctggta tcagcagaaa     180

ccagggaaag ctcctaagct cctgatctat gatgcctcca gtttggaaag tggggtccca     240

tcaaggttca gcggcagtgg atctgggaca gatttcactc tcaccatcag cagcctgcag     300

cctgaagatt ttgcaactta ttactgtcaa cagtttaata gttacccgta cacttttggc     360

caggggacca agctggagat caaaggtggc ggtggctcgg gcggtggtgg gtcgggtggc     420

ggcggatctg aggtgcagct ggtgcagtct ggagcagagg tgaaaaagcc cggggagtct     480

ctgaagatct cctgtaaggg ttctggatac agctttagca gcagctggat cggctgggtg     540

cgccagatgc ccgggaaagg cctggagtgg atggggatca tctatcctga tgactctgat     600

accagataca gtccgtcctt ccaaggccag gtcaccatct cagccgacaa gtccatcagg     660

accgcctacc tgcagtggag cagcctgaag gcctcggaca ccgccatgta ttactgtgcg     720

agacatgtta ctatgatttg gggagttatt attgacttct ggggccaggg aaccctggtc     780

accgtctcct caccacattt ggagcaggag cctacaaatt tgagagaatc atctctaagc     840

aaaatgtcct cagatctgca aatgagaaat tcacaagcgc acaggaattt tcttgaagat     900

ggagaaagtg atggcttttt aagatgcctc tctcttaact ctgggtggat tttaactaca     960

actcttgtcc tctcggtgat ggtattgctt tggatttgtt gtgcaaaacg gggcagaaag    1020

aaactcctgt atatattcaa acaaccattt atgagaccag tacaaactac tcaagaggaa    1080

gatggctgta gctgccgatt tccagaagaa gaagaaggag gatgtgaact gagagtgaag    1140

ttcagcagga gcgcagacgc ccccgcgtac aagcagggcc agaaccagct ctataacgag    1200

ctcaatctag gacgaagaga ggagtacgat gttttggaca agagacgtgg ccgggaccct    1260

gagatggggg gaaagccgag aaggaagaac cctcaggaag gcctgtacaa tgaactgcag    1320

aaagataaga tggcggaggc ctacagtgag attgggatga aaggcgagcg ccggaggggc    1380

aaggggcacg atggccttta ccagggtctc agtacagcca ccaaggacac ctacgacgcc    1440

cttcacatgc aggccctgcc ccctcgctaa                                      1470

<210>  34
<211>  252
<212>  DNA
<213>  Artificial Sequence

<220>
<223>  nucleotide sequence of CD8 transmembrane region

<400>  34
ttcgtgccgg tcttcctgcc agcgaagccc accacgacgc cagcgccgcg accaccaaca      60

ccggcgccca ccatcgcgtc gcagcccctg tccctgcgcc cagaggcgtg ccggccagcg 120

gcggggggcg cagtgcacac gagggggctg gacttcgcct gtgatatcta catctgggcg 180

cccttggccg ggacttgtgg ggtccttctc ctgtcactgg ttatcaccct ttactgcaac 240

cacaggaaca gg 252

<210> 35
<211> 747
<212> DNA
<213> Artificial Sequence

<220>
<223> CD22 targeting moiety

<400> 35
caagtgcagc tgcagcagtc cggacccggc ctcgtcaagc cctcccagac cctctctctg 60

acatgtgcca tcagcggcga tagcgtcagc tccaacagcg ctgcttggaa ctggattagg 120

cagagcccta gcagaggact ggagtggctg ggaaggacct actatagaag caagtggtac 180

aacgattacg ccgtgagcgt caagtctaga atcaccatta accccgacac ctccaagaac 240

cagttctctc tgcagctcaa cagcgtgacc cccgaggaca ccgctgtcta ctattgtgcc 300

agagaggtga ccggagatct ggaggatgcc ttcgacatct ggggccaagg caccatggtc 360

accgtgagca gcggctccac atccggctcc ggaaagcccg gcagcggcga aggctccaca 420

aagggcgaca tccagatgac ccaaagccct tcctctctgt ccgctagcgt cggagataga 480

gtcaccatca catgcagagc tagccagacc atttggagct acctcaactg gtaccagcag 540

aggcccggca aggcccctaa tctgctgatc tacgctgcct cctctctgca gagcggagtg 600

cctagcagat tcagcggaag gggcagcgga accgacttta cactgaccat tagcagcctc 660

caagccgaag acttcgccac ctactactgc caacagtcct actccattcc tcagacattt 720

ggccaaggca ccaagctcga gatcaag 747

<210> 36
<211> 789
<212> DNA
<213> Artificial Sequence

<220>
<223> GPC3 targeting moiety

<400> 36
atggccttac cagtgaccgc cttgctcctg ccgctggcct tgctgctcca cgccgccagg 60

ccggacgtgg tgatgaccca gagcccttta tctttacccg ttacacccgg tgagcccgct 120

agcatctctt gtagaagcag ccagtcttta gtgcacagca acggcaacac atatttacac 180

tggtatttac agaagcccgg tcagagcccc cagctgctga tctacaaggt gagcaatcgt 240

ttctccggcg tgcccgatag attcagcggc agcggctccg gaaccgactt cactttaaag 300

atcagcagag tggaggccga ggacgtgggt gtgtactact gctcccagaa cacccacgtg 360

ccccctacat tcggtcaagg taccaaactg gagatcaaag gtggcggtgg ctcgggcggt 420

ggtgggtcgg gtggcggcgg atctcaagtt cagctggtgc agagcggcgc cgaggtcaaa 480

aaacccggag ccagcgtgaa ggtgtcttgt aaagccagcg gctacacctt taccgactat 540

gagatgcact gggtgagaca agctcccggt caaggtctgg aatggatggg cgctttagac 600

cccaagactg gtgacacagc ctactcccag aagttcaagg gtcgtgtgac tttaacagcc 660

gacgagtcca ccagcacagc ctacatggaa ctgagctctt taaggagcga ggacaccgcc 720

gtgtattact gcactcgttt ctacagctac acctactggg gccaaggtac tttagtgaca 780

gtgagcagc 789

<210> 37
<211> 84
<212> PRT
<213> Artificial Sequence

<220>
<223> amino acid sequence of CD8 transmembrane region

<400> 37

Phe Val Pro Val Phe Leu Pro Ala Lys Pro Thr Thr Thr Pro Ala Pro
1               5                   10                  15

Arg Pro Pro Thr Pro Ala Pro Thr Ile Ala Ser Gln Pro Leu Ser Leu
            20                  25                  30

Arg Pro Glu Ala Cys Arg Pro Ala Ala Gly Gly Ala Val His Thr Arg
        35                  40                  45

Gly Leu Asp Phe Ala Cys Asp Ile Tyr Ile Trp Ala Pro Leu Ala Gly
    50                  55                  60

Thr Cys Gly Val Leu Leu Leu Ser Leu Val Ile Thr Leu Tyr Cys Asn
65                  70                  75                  80

His Arg Asn Arg

<210> 38
<211> 45
<212> PRT
<213> Artificial Sequence

<220>
<223> amino acid sequence of CD8 hinge region

<400>  38

Thr Thr Thr Pro Ala Pro Arg Pro Pro Thr Pro Ala Pro Thr Ile Ala
1               5                   10                  15

Ser Gln Pro Leu Ser Leu Arg Pro Glu Ala Cys Arg Pro Ala Ala Gly
            20                  25                  30

Gly Ala Val His Thr Arg Gly Leu Asp Phe Ala Cys Asp
        35                  40                  45

<210>  39
<211>  112
<212>  PRT
<213>  Artificial Sequence

<220>
<223>  amino acid sequence of CD3 zeta signaling domain

<400>  39

Arg Val Lys Phe Ser Arg Ser Ala Asp Ala Pro Ala Tyr Gln Gln Gly
1               5                   10                  15

Gln Asn Gln Leu Tyr Asn Glu Leu Asn Leu Gly Arg Arg Glu Glu Tyr
            20                  25                  30

Asp Val Leu Asp Lys Arg Arg Gly Arg Asp Pro Glu Met Gly Gly Lys
        35                  40                  45

Pro Arg Arg Lys Asn Pro Gln Glu Gly Leu Tyr Asn Glu Leu Gln Lys
    50                  55                  60

Asp Lys Met Ala Glu Ala Tyr Ser Glu Ile Gly Met Lys Gly Glu Arg
65                  70                  75                  80

Arg Arg Gly Lys Gly His Asp Gly Leu Tyr Gln Gly Leu Ser Thr Ala
                85                  90                  95

Thr Lys Asp Thr Tyr Asp Ala Leu His Met Gln Ala Leu Pro Pro Arg
            100                 105                 110

<210>  40
<211>  47
<212>  PRT
<213>  Artificial Sequence

<220>
<223>  amino acid sequence of human 4-1BB costimulatory domain

<400>  40

Arg Phe Ser Val Val Lys Arg Gly Arg Lys Lys Leu Leu Tyr Ile Phe

1               5                   10                  15

Lys Gln Pro Phe Met Arg Pro Val Gln Thr Thr Gln Glu Glu Asp Gly
            20                  25                  30

Cys Ser Cys Arg Phe Pro Glu Glu Glu Glu Gly Gly Cys Glu Leu
        35                  40                  45

<210>  41
<211>  245
<212>  PRT
<213>  Artificial Sequence

<220>
<223>  humanized CD19 monoclonal antibody single chain variable region

<400>  41

Gln Val Gln Leu Val Gln Ser Gly Gly Gly Val Val Gln Pro Gly Arg
1               5                   10                  15

Ser Leu Arg Leu Ser Cys Ala Ala Ser Gly Val Ser Leu Pro Asp Tyr
            20                  25                  30

Gly Val Ser Trp Val Arg Gln Ala Pro Gly Lys Gly Leu Glu Trp Val
        35                  40                  45

Ala Val Ile Trp Gly Ser Glu Thr Thr Tyr Tyr Asn Ser Ala Leu Lys
    50                  55                  60

Ser Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys His Tyr Tyr Tyr Gly Gly Ser Phe Ala Met Asp Tyr Trp Gly Gln
            100                 105                 110

Gly Thr Leu Val Thr Val Ser Ser Gly Ser Thr Ser Gly Ser Gly Lys
        115                 120                 125

Pro Gly Ser Gly Glu Gly Ser Thr Lys Gly Asp Ile Gln Met Thr Gln
    130                 135                 140

Ser Pro Ser Ser Leu Ser Ala Ser Val Gly Asp Arg Val Thr Ile Thr
145                 150                 155                 160

Cys Arg Ala Ser Gln Asp Ile Ser Lys Tyr Leu Asn Trp Tyr Gln Gln
                165                 170                 175

Lys Pro Gly Lys Ala Ile Lys Leu Leu Ile Tyr His Thr Ser Arg Leu
            180                 185                 190

His Ser Gly Val Pro Ser Arg Phe Ser Gly Ser Gly Ser Gly Thr Asp
        195                 200                 205

Phe Thr Leu Thr Ile Ser Ser Leu Gln Pro Glu Asp Phe Ala Thr Tyr
    210                 215                 220

Tyr Cys Gln Gln Gly Ala Thr Leu Pro Tyr Thr Phe Gly Pro Gly Thr
225                 230                 235                 240

Lys Val Asp Ile Lys
                245

<210>  42
<211>  264
<212>  PRT
<213>  Artificial Sequence

<220>
<223>  full-humanized CD19 monoclonal single chain variable region 21D4

<400>  42

Met Ala Leu Pro Val Thr Ala Leu Leu Leu Pro Leu Ala Leu Leu Leu
1               5                   10                  15

His Ala Ala Arg Pro Ala Ile Gln Leu Thr Gln Ser Pro Ser Ser Leu
            20                  25                  30

Ser Ala Ser Val Gly Asp Arg Val Thr Ile Thr Cys Arg Ala Ser Gln
        35                  40                  45

Gly Ile Ser Ser Ala Leu Ala Trp Tyr Gln Gln Lys Pro Gly Lys Ala
    50                  55                  60

Pro Lys Leu Leu Ile Tyr Asp Ala Ser Ser Leu Glu Ser Gly Val Pro
65                  70                  75                  80

Ser Arg Phe Ser Gly Ser Gly Ser Gly Thr Asp Phe Thr Leu Thr Ile
                85                  90                  95

Ser Ser Leu Gln Pro Glu Asp Phe Ala Thr Tyr Tyr Cys Gln Gln Phe
            100                 105                 110

Asn Ser Tyr Pro Tyr Thr Phe Gly Gln Gly Thr Lys Leu Glu Ile Lys
        115                 120                 125

Gly Gly Gly Gly Ser Gly Gly Gly Gly Ser Gly Gly Gly Gly Ser Glu
    130                 135                 140

Val Gln Leu Val Gln Ser Gly Ala Glu Val Lys Lys Pro Gly Glu Ser
145                 150                 155                 160

Leu Lys Ile Ser Cys Lys Gly Ser Gly Tyr Ser Phe Ser Ser Ser Trp
                165                 170                 175

Ile Gly Trp Val Arg Gln Met Pro Gly Lys Gly Leu Glu Trp Met Gly
            180                 185                 190

Ile Ile Tyr Pro Asp Asp Ser Asp Thr Arg Tyr Ser Pro Ser Phe Gln
        195                 200                 205

Gly Gln Val Thr Ile Ser Ala Asp Lys Ser Ile Arg Thr Ala Tyr Leu
    210                 215                 220

Gln Trp Ser Ser Leu Lys Ala Ser Asp Thr Ala Met Tyr Tyr Cys Ala
225                 230                 235                 240

Arg His Val Thr Met Ile Trp Gly Val Ile Ile Asp Phe Trp Gly Gln
                245                 250                 255

Gly Thr Leu Val Thr Val Ser Ser
            260

<210>  43
<211>  488
<212>  PRT
<213>  Artificial Sequence

<220>
<223>  GPC3-59TM CAR

<400>  43

Met Ala Leu Pro Val Thr Ala Leu Leu Leu Pro Leu Ala Leu Leu Leu
1               5                   10                  15

His Ala Ala Arg Pro Asp Val Val Met Thr Gln Ser Pro Leu Ser Leu
            20                  25                  30

Pro Val Thr Pro Gly Glu Pro Ala Ser Ile Ser Cys Arg Ser Ser Gln
        35                  40                  45

Ser Leu Val His Ser Asn Gly Asn Thr Tyr Leu His Trp Tyr Leu Gln
    50                  55                  60

Lys Pro Gly Gln Ser Pro Gln Leu Leu Ile Tyr Lys Val Ser Asn Arg
65                  70                  75                  80

Phe Ser Gly Val Pro Asp Arg Phe Ser Gly Ser Gly Ser Gly Thr Asp
                85                  90                  95

Phe Thr Leu Lys Ile Ser Arg Val Glu Ala Glu Asp Val Gly Val Tyr
            100                 105                 110

Tyr Cys Ser Gln Asn Thr His Val Pro Pro Thr Phe Gly Gln Gly Thr
        115                 120                 125

Lys Leu Glu Ile Lys Gly Gly Gly Gly Ser Gly Gly Gly Gly Ser Gly
    130                 135                 140

Gly Gly Gly Ser Gln Val Gln Leu Val Gln Ser Gly Ala Glu Val Lys
145                 150                 155                 160

Lys Pro Gly Ala Ser Val Lys Val Ser Cys Lys Ala Ser Gly Tyr Thr
                165                 170                 175

Phe Thr Asp Tyr Glu Met His Trp Val Arg Gln Ala Pro Gly Gln Gly
            180                 185                 190

Leu Glu Trp Met Gly Ala Leu Asp Pro Lys Thr Gly Asp Thr Ala Tyr
        195                 200                 205

Ser Gln Lys Phe Lys Gly Arg Val Thr Leu Thr Ala Asp Glu Ser Thr
    210                 215                 220

Ser Thr Ala Tyr Met Glu Leu Ser Ser Leu Arg Ser Glu Asp Thr Ala
225                 230                 235                 240

Val Tyr Tyr Cys Thr Arg Phe Tyr Ser Tyr Thr Tyr Trp Gly Gln Gly
                245                 250                 255

Thr Leu Val Thr Val Ser Ser Pro His Leu Glu Gln Glu Pro Thr Asn
            260                 265                 270

Leu Arg Glu Ser Ser Leu Ser Lys Met Ser Ser Asp Leu Gln Met Arg
        275                 280                 285

Asn Ser Gln Ala His Arg Asn Phe Leu Glu Asp Gly Glu Ser Asp Gly
    290                 295                 300

Phe Leu Arg Cys Leu Ser Leu Asn Ser Gly Trp Ile Leu Thr Thr Thr
305                 310                 315                 320

Leu Val Leu Ser Val Met Val Leu Leu Trp Ile Cys Cys Ala Lys Arg
                325                 330                 335

Gly Arg Lys Lys Leu Leu Tyr Ile Phe Lys Gln Pro Phe Met Arg Pro
            340                 345                 350

Val Gln Thr Thr Gln Glu Glu Asp Gly Cys Ser Cys Arg Phe Pro Glu
        355                 360                 365

Glu Glu Glu Gly Gly Cys Glu Leu Arg Val Lys Phe Ser Arg Ser Ala
    370                 375                 380

Asp Ala Pro Ala Tyr Lys Gln Gly Gln Asn Gln Leu Tyr Asn Glu Leu
385                 390                 395                 400

Asn Leu Gly Arg Arg Glu Glu Tyr Asp Val Leu Asp Lys Arg Arg Gly
                405                 410                 415

Arg Asp Pro Glu Met Gly Gly Lys Pro Arg Arg Lys Asn Pro Gln Glu
            420                 425                 430

Gly Leu Tyr Asn Glu Leu Gln Lys Asp Lys Met Ala Glu Ala Tyr Ser
        435                 440                 445

Glu Ile Gly Met Lys Gly Glu Arg Arg Arg Gly Lys Gly His Asp Gly
    450                 455                 460

Leu Tyr Gln Gly Leu Ser Thr Ala Thr Lys Asp Thr Tyr Asp Ala Leu
465                 470                 475                 480

His Met Gln Ala Leu Pro Pro Arg
                485

<210>  44
<211>  489
<212>  PRT
<213>  Artificial Sequence

<220>
<223>  21D4-59TM CAR

<400>  44

Met Ala Leu Pro Val Thr Ala Leu Leu Leu Pro Leu Ala Leu Leu Leu
1               5                   10                  15

His Ala Ala Arg Pro Ala Ile Gln Leu Thr Gln Ser Pro Ser Ser Leu
            20                  25                  30

Ser Ala Ser Val Gly Asp Arg Val Thr Ile Thr Cys Arg Ala Ser Gln
        35                  40                  45

Gly Ile Ser Ser Ala Leu Ala Trp Tyr Gln Gln Lys Pro Gly Lys Ala
    50                  55                  60

Pro Lys Leu Leu Ile Tyr Asp Ala Ser Ser Leu Glu Ser Gly Val Pro
65                  70                  75                  80

Ser Arg Phe Ser Gly Ser Gly Ser Gly Thr Asp Phe Thr Leu Thr Ile
                85                  90                  95

Ser Ser Leu Gln Pro Glu Asp Phe Ala Thr Tyr Tyr Cys Gln Gln Phe
            100                 105                 110

Asn Ser Tyr Pro Tyr Thr Phe Gly Gln Gly Thr Lys Leu Glu Ile Lys
        115                 120                 125

Gly Gly Gly Gly Ser Gly Gly Gly Gly Ser Gly Gly Gly Gly Ser Glu
    130                 135                 140

Val Gln Leu Val Gln Ser Gly Ala Glu Val Lys Lys Pro Gly Glu Ser
145                 150                 155                 160

Leu Lys Ile Ser Cys Lys Gly Ser Gly Tyr Ser Phe Ser Ser Ser Trp
                165                 170                 175

Ile Gly Trp Val Arg Gln Met Pro Gly Lys Gly Leu Glu Trp Met Gly
            180                 185                 190

Ile Ile Tyr Pro Asp Asp Ser Asp Thr Arg Tyr Ser Pro Ser Phe Gln
        195                 200                 205

Gly Gln Val Thr Ile Ser Ala Asp Lys Ser Ile Arg Thr Ala Tyr Leu
    210                 215                 220

Gln Trp Ser Ser Leu Lys Ala Ser Asp Thr Ala Met Tyr Tyr Cys Ala
225                 230                 235                 240

Arg His Val Thr Met Ile Trp Gly Val Ile Ile Asp Phe Trp Gly Gln
                245                 250                 255

Gly Thr Leu Val Thr Val Ser Ser Pro His Leu Glu Gln Glu Pro Thr
            260                 265                 270

Asn Leu Arg Glu Ser Ser Leu Ser Lys Met Ser Ser Asp Leu Gln Met
        275                 280                 285

Arg Asn Ser Gln Ala His Arg Asn Phe Leu Glu Asp Gly Glu Ser Asp
    290                 295                 300

Gly Phe Leu Arg Cys Leu Ser Leu Asn Ser Gly Trp Ile Leu Thr Thr
305                 310                 315                 320

Thr Leu Val Leu Ser Val Met Val Leu Leu Trp Ile Cys Cys Ala Lys
                325                 330                 335

Arg Gly Arg Lys Lys Leu Leu Tyr Ile Phe Lys Gln Pro Phe Met Arg
            340                 345                 350

Pro Val Gln Thr Thr Gln Glu Glu Asp Gly Cys Ser Cys Arg Phe Pro
        355                 360                 365

Glu Glu Glu Glu Gly Gly Cys Glu Leu Arg Val Lys Phe Ser Arg Ser
    370                 375                 380

Ala Asp Ala Pro Ala Tyr Lys Gln Gly Gln Asn Gln Leu Tyr Asn Glu
385                 390                 395                 400

Leu Asn Leu Gly Arg Arg Glu Glu Tyr Asp Val Leu Asp Lys Arg Arg
                405                 410                 415

Gly Arg Asp Pro Glu Met Gly Gly Lys Pro Arg Arg Lys Asn Pro Gln
            420                 425                 430

Glu Gly Leu Tyr Asn Glu Leu Gln Lys Asp Lys Met Ala Glu Ala Tyr
        435                 440                 445

Ser Glu Ile Gly Met Lys Gly Glu Arg Arg Arg Gly Lys Gly His Asp
    450                 455                 460

Gly Leu Tyr Gln Gly Leu Ser Thr Ala Thr Lys Asp Thr Tyr Asp Ala
465                 470                 475                 480

Leu His Met Gln Ala Leu Pro Pro Arg
                485

<210>  45
<211>  239
<212>  PRT
<213>  Artificial Sequence

<220>
<223>  GFP targeting moiety

<400>  45

Met Val Ser Lys Gly Glu Glu Leu Phe Thr Gly Val Val Pro Ile Leu
1               5                   10                  15

Val Glu Leu Asp Gly Asp Val Asn Gly His Lys Phe Ser Val Ser Gly
            20                  25                  30

Glu Gly Glu Gly Asp Ala Thr Tyr Gly Lys Leu Thr Leu Lys Phe Ile
        35                  40                  45

Cys Thr Thr Gly Lys Leu Pro Val Pro Trp Pro Thr Leu Val Thr Thr
    50                  55                  60

Leu Thr Tyr Gly Val Gln Cys Phe Ser Arg Tyr Pro Asp His Met Lys
65                  70                  75                  80

Gln His Asp Phe Phe Lys Ser Ala Met Pro Glu Gly Tyr Val Gln Glu
                85                  90                  95

Arg Thr Ile Phe Phe Lys Asp Asp Gly Asn Tyr Lys Thr Arg Ala Glu
            100                 105                 110

Val Lys Phe Glu Gly Asp Thr Leu Val Asn Arg Ile Glu Leu Lys Gly
        115                 120                 125

Ile Asp Phe Lys Glu Asp Gly Asn Ile Leu Gly His Lys Leu Glu Tyr
    130                 135                 140

Asn Tyr Asn Ser His Asn Val Tyr Ile Met Ala Asp Lys Gln Lys Asn
145                 150                 155                 160

Gly Ile Lys Val Asn Phe Lys Ile Arg His Asn Ile Glu Asp Gly Ser
                165                 170                 175

Val Gln Leu Ala Asp His Tyr Gln Gln Asn Thr Pro Ile Gly Asp Gly
            180                 185                 190

Pro Val Leu Leu Pro Asp Asn His Tyr Leu Ser Thr Gln Ser Ala Leu
        195                 200                 205

Ser Lys Asp Pro Asn Glu Lys Arg Asp His Met Val Leu Leu Glu Phe
    210                 215                 220

Val Thr Ala Ala Gly Ile Thr Leu Gly Met Asp Glu Leu Tyr Lys
225                 230                 235

<210>  46
<211>  22
<212>  PRT

<213>  Artificial Sequence

<220>
<223>  amino acid sequence of CD8 signal peptide

<400>  46

Met Leu Leu Leu Val Thr Ser Leu Leu Leu Cys Glu Leu Pro His Pro
1               5                   10                  15

Ala Phe Leu Leu Ile Pro
            20

<210>  47
<211>  19
<212>  PRT
<213>  Artificial Sequence

<220>
<223>  2A sequence

<400>  47

Ala Thr Asn Phe Ser Leu Leu Lys Gln Ala Gly Asp Val Glu Glu Asn
1               5                   10                  15

Pro Gly Pro

<210>  48
<211>  249
<212>  PRT
<213>  Artificial Sequence

<220>
<223>  CD22 targeting moiety

<400>  48

Gln Val Gln Leu Gln Gln Ser Gly Pro Gly Leu Val Lys Pro Ser Gln
1               5                   10                  15

Thr Leu Ser Leu Thr Cys Ala Ile Ser Gly Asp Ser Val Ser Ser Asn
            20                  25                  30

Ser Ala Ala Trp Asn Trp Ile Arg Gln Ser Pro Ser Arg Gly Leu Glu
        35                  40                  45

Trp Leu Gly Arg Thr Tyr Tyr Arg Ser Lys Trp Tyr Asn Asp Tyr Ala
    50                  55                  60

Val Ser Val Lys Ser Arg Ile Thr Ile Asn Pro Asp Thr Ser Lys Asn
65                  70                  75                  80

Gln Phe Ser Leu Gln Leu Asn Ser Val Thr Pro Glu Asp Thr Ala Val
                85                  90                  95

Tyr Tyr Cys Ala Arg Glu Val Thr Gly Asp Leu Glu Asp Ala Phe Asp
            100                 105                 110

Ile Trp Gly Gln Gly Thr Met Val Thr Val Ser Ser Gly Ser Thr Ser
        115                 120                 125

Gly Ser Gly Lys Pro Gly Ser Gly Glu Gly Ser Thr Lys Gly Asp Ile
    130                 135                 140

Gln Met Thr Gln Ser Pro Ser Ser Leu Ser Ala Ser Val Gly Asp Arg
145                 150                 155                 160

Val Thr Ile Thr Cys Arg Ala Ser Gln Thr Ile Trp Ser Tyr Leu Asn
                165                 170                 175

Trp Tyr Gln Gln Arg Pro Gly Lys Ala Pro Asn Leu Leu Ile Tyr Ala
            180                 185                 190

Ala Ser Ser Leu Gln Ser Gly Val Pro Ser Arg Phe Ser Gly Arg Gly
        195                 200                 205

Ser Gly Thr Asp Phe Thr Leu Thr Ile Ser Ser Leu Gln Ala Glu Asp
    210                 215                 220

Phe Ala Thr Tyr Tyr Cys Gln Gln Ser Tyr Ser Ile Pro Gln Thr Phe
225                 230                 235                 240

Gly Gln Gly Thr Lys Leu Glu Ile Lys
                245

<210>  49
<211>  490
<212>  PRT
<213>  Artificial Sequence

<220>
<223>  H63 CAR

<400>  49

Met Leu Leu Leu Val Thr Ser Leu Leu Leu Cys Glu Leu Pro His Pro
1               5                   10                  15

Ala Phe Leu Leu Ile Pro Gln Val Gln Leu Val Gln Ser Gly Gly Gly
            20                  25                  30

Val Val Gln Pro Gly Arg Ser Leu Arg Leu Ser Cys Ala Ala Ser Gly

        35                  40                  45

Val Ser Leu Pro Asp Tyr Gly Val Ser Trp Val Arg Gln Ala Pro Gly
    50                  55                  60

Lys Gly Leu Glu Trp Val Ala Val Ile Trp Gly Ser Glu Thr Thr Tyr
65                  70                  75                  80

Tyr Asn Ser Ala Leu Lys Ser Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Lys His Tyr Tyr Tyr Gly Gly Ser Phe Ala
        115                 120                 125

Met Asp Tyr Trp Gly Gln Gly Thr Leu Val Thr Val Ser Ser Gly Ser
    130                 135                 140

Thr Ser Gly Ser Gly Lys Pro Gly Ser Gly Glu Gly Ser Thr Lys Gly
145                 150                 155                 160

Asp Ile Gln Met Thr Gln Ser Pro Ser Ser Leu Ser Ala Ser Val Gly
                165                 170                 175

Asp Arg Val Thr Ile Thr Cys Arg Ala Ser Gln Asp Ile Ser Lys Tyr
            180                 185                 190

Leu Asn Trp Tyr Gln Gln Lys Pro Gly Lys Ala Ile Lys Leu Leu Ile
        195                 200                 205

Tyr His Thr Ser Arg Leu His Ser Gly Val Pro Ser Arg Phe Ser Gly
    210                 215                 220

Ser Gly Ser Gly Thr Asp Phe Thr Leu Thr Ile Ser Ser Leu Gln Pro
225                 230                 235                 240

Glu Asp Phe Ala Thr Tyr Tyr Cys Gln Gln Gly Ala Thr Leu Pro Tyr
                245                 250                 255

Thr Phe Gly Pro Gly Thr Lys Val Asp Ile Lys Thr Thr Thr Pro Ala
            260                 265                 270

Pro Arg Pro Pro Thr Pro Ala Pro Thr Ile Ala Ser Gln Pro Leu Ser
        275                 280                 285

Leu Arg Pro Glu Ala Cys Arg Pro Ala Ala Gly Gly Ala Val His Thr
    290                 295                 300

Arg Gly Leu Asp Phe Ala Cys Asp Ile Tyr Ile Trp Ala Pro Leu Ala
305                 310                 315                 320

Gly Thr Cys Gly Val Leu Leu Leu Ser Leu Val Ile Thr Leu Tyr Cys
                325                 330                 335

Lys Arg Gly Arg Lys Lys Leu Leu Tyr Ile Phe Lys Gln Pro Phe Met
            340                 345                 350

Arg Pro Val Gln Thr Thr Gln Glu Glu Asp Gly Cys Ser Cys Arg Phe
        355                 360                 365

Pro Glu Glu Glu Glu Gly Gly Cys Glu Leu Arg Val Lys Phe Ser Arg
    370                 375                 380

Ser Ala Asp Ala Pro Ala Tyr Lys Gln Gly Gln Asn Gln Leu Tyr Asn
385                 390                 395                 400

Glu Leu Asn Leu Gly Arg Arg Glu Glu Tyr Asp Val Leu Asp Lys Arg
                405                 410                 415

Arg Gly Arg Asp Pro Glu Met Gly Gly Lys Pro Arg Arg Lys Asn Pro
            420                 425                 430

Gln Glu Gly Leu Tyr Asn Glu Leu Gln Lys Asp Lys Met Ala Glu Ala
        435                 440                 445

Tyr Ser Glu Ile Gly Met Lys Gly Glu Arg Arg Arg Gly Lys Gly His
    450                 455                 460

Asp Gly Leu Tyr Gln Gly Leu Ser Thr Ala Thr Lys Asp Thr Tyr Asp
465                 470                 475                 480

Ala Leu His Met Gln Ala Leu Pro Pro Arg
                485                 490

<210>  50
<211>  836
<212>  PRT
<213>  Artificial Sequence

<220>
<223>  H63 CAR-2A-TMEM59

<400>  50

Met Leu Leu Leu Val Thr Ser Leu Leu Leu Cys Glu Leu Pro His Pro

1               5                   10                  15

Ala Phe Leu Leu Ile Pro Gln Val Gln Leu Val Gln Ser Gly Gly Gly
            20                  25                  30

Val Val Gln Pro Gly Arg Ser Leu Arg Leu Ser Cys Ala Ala Ser Gly
        35                  40                  45

Val Ser Leu Pro Asp Tyr Gly Val Ser Trp Val Arg Gln Ala Pro Gly
    50                  55                  60

Lys Gly Leu Glu Trp Val Ala Val Ile Trp Gly Ser Glu Thr Thr Tyr
65                  70                  75                  80

Tyr Asn Ser Ala Leu Lys Ser Arg Phe Thr Ile Ser Arg Asp Asn Ser
                85                  90                  95

Lys Asn Thr Leu Tyr Leu Gln Met Asn Ser Leu Arg Ala Glu Asp Thr
            100                 105                 110

Ala Val Tyr Tyr Cys Ala Lys His Tyr Tyr Tyr Gly Gly Ser Phe Ala
        115                 120                 125

Met Asp Tyr Trp Gly Gln Gly Thr Leu Val Thr Val Ser Ser Gly Ser
    130                 135                 140

Thr Ser Gly Ser Gly Lys Pro Gly Ser Gly Glu Gly Ser Thr Lys Gly
145                 150                 155                 160

Asp Ile Gln Met Thr Gln Ser Pro Ser Ser Leu Ser Ala Ser Val Gly
                165                 170                 175

Asp Arg Val Thr Ile Thr Cys Arg Ala Ser Gln Asp Ile Ser Lys Tyr
            180                 185                 190

Leu Asn Trp Tyr Gln Gln Lys Pro Gly Lys Ala Ile Lys Leu Leu Ile
        195                 200                 205

Tyr His Thr Ser Arg Leu His Ser Gly Val Pro Ser Arg Phe Ser Gly
    210                 215                 220

Ser Gly Ser Gly Thr Asp Phe Thr Leu Thr Ile Ser Ser Leu Gln Pro
225                 230                 235                 240

Glu Asp Phe Ala Thr Tyr Tyr Cys Gln Gln Gly Ala Thr Leu Pro Tyr
                245                 250                 255

Thr Phe Gly Pro Gly Thr Lys Val Asp Ile Lys Thr Thr Thr Pro Ala
            260                 265                 270

Pro Arg Pro Pro Thr Pro Ala Pro Thr Ile Ala Ser Gln Pro Leu Ser
        275                 280                 285

Leu Arg Pro Glu Ala Cys Arg Pro Ala Ala Gly Gly Ala Val His Thr
    290                 295                 300

Arg Gly Leu Asp Phe Ala Cys Asp Ile Tyr Ile Trp Ala Pro Leu Ala
305                 310                 315                 320

Gly Thr Cys Gly Val Leu Leu Leu Ser Leu Val Ile Thr Leu Tyr Cys
                325                 330                 335

Lys Arg Gly Arg Lys Lys Leu Leu Tyr Ile Phe Lys Gln Pro Phe Met
            340                 345                 350

Arg Pro Val Gln Thr Thr Gln Glu Glu Asp Gly Cys Ser Cys Arg Phe
        355                 360                 365

Pro Glu Glu Glu Glu Gly Gly Cys Glu Leu Arg Val Lys Phe Ser Arg
    370                 375                 380

Ser Ala Asp Ala Pro Ala Tyr Lys Gln Gly Gln Asn Gln Leu Tyr Asn
385                 390                 395                 400

Glu Leu Asn Leu Gly Arg Arg Glu Glu Tyr Asp Val Leu Asp Lys Arg
                405                 410                 415

Arg Gly Arg Asp Pro Glu Met Gly Gly Lys Pro Arg Arg Lys Asn Pro
            420                 425                 430

Gln Glu Gly Leu Tyr Asn Glu Leu Gln Lys Asp Lys Met Ala Glu Ala
        435                 440                 445

Tyr Ser Glu Ile Gly Met Lys Gly Glu Arg Arg Arg Gly Lys Gly His
    450                 455                 460

Asp Gly Leu Tyr Gln Gly Leu Ser Thr Ala Thr Lys Asp Thr Tyr Asp
465                 470                 475                 480

Ala Leu His Met Gln Ala Leu Pro Pro Arg Gly Ser Gly Ala Thr Asn
                485                 490                 495

Phe Ser Leu Leu Lys Gln Ala Gly Asp Val Glu Glu Asn Pro Gly Pro
            500                 505                 510

Met Ala Ala Pro Lys Gly Ser Leu Trp Val Arg Thr Gln Leu Gly Leu
        515                 520                 525

Pro Pro Leu Leu Leu Leu Thr Met Ala Leu Ala Gly Gly Ser Gly Thr
    530                 535                 540

Ala Ser Ala Glu Ala Phe Asp Ser Val Leu Gly Asp Thr Ala Ser Cys
545                 550                 555                 560

His Arg Ala Cys Gln Leu Thr Tyr Pro Leu His Thr Tyr Pro Lys Glu
                565                 570                 575

Glu Glu Leu Tyr Ala Cys Gln Arg Gly Cys Arg Leu Phe Ser Ile Cys
            580                 585                 590

Gln Phe Val Asp Asp Gly Ile Asp Leu Asn Arg Thr Lys Leu Glu Cys
        595                 600                 605

Glu Ser Ala Cys Thr Glu Ala Tyr Ser Gln Ser Asp Glu Gln Tyr Ala
    610                 615                 620

Cys His Leu Gly Cys Gln Asn Gln Leu Pro Phe Ala Glu Leu Arg Gln
625                 630                 635                 640

Glu Gln Leu Met Ser Leu Met Pro Lys Met His Leu Leu Phe Pro Leu
                645                 650                 655

Thr Leu Val Arg Ser Phe Trp Ser Asp Met Met Asp Ser Ala Gln Ser
            660                 665                 670

Phe Ile Thr Ser Ser Trp Thr Phe Tyr Leu Gln Ala Asp Asp Gly Lys
        675                 680                 685

Ile Val Ile Phe Gln Ser Lys Pro Glu Ile Gln Tyr Ala Pro His Leu
    690                 695                 700

Glu Gln Glu Pro Thr Asn Leu Arg Glu Ser Ser Leu Ser Lys Met Ser
705                 710                 715                 720

Ser Asp Leu Gln Met Arg Asn Ser Gln Ala His Arg Asn Phe Leu Glu
                725                 730                 735

Asp Gly Glu Ser Asp Gly Phe Leu Arg Cys Leu Ser Leu Asn Ser Gly
            740                 745                 750

Trp Ile Leu Thr Thr Thr Leu Val Leu Ser Val Met Val Leu Leu Trp
        755                 760                 765

Ile Cys Cys Ala Thr Val Ala Thr Ala Val Glu Gln Tyr Val Pro Ser
    770                 775                 780

Glu Lys Leu Ser Ile Tyr Gly Asp Leu Glu Phe Met Asn Glu Gln Lys
785                 790                 795                 800

Leu Asn Arg Tyr Pro Ala Ser Ser Leu Val Val Val Arg Ser Lys Thr
                805                 810                 815

Glu Asp His Glu Glu Ala Gly Pro Leu Pro Thr Lys Val Asn Leu Ala
            820                 825                 830

His Ser Glu Ile
        835

<210>  51
<211>  506
<212>  PRT
<213>  Artificial Sequence

<220>
<223>  21D4 CAR

<400>  51

Met Ala Leu Pro Val Thr Ala Leu Leu Leu Pro Leu Ala Leu Leu Leu
1               5                   10                  15

His Ala Ala Arg Pro Ala Ile Gln Leu Thr Gln Ser Pro Ser Ser Leu
            20                  25                  30

Ser Ala Ser Val Gly Asp Arg Val Thr Ile Thr Cys Arg Ala Ser Gln
        35                  40                  45

Gly Ile Ser Ser Ala Leu Ala Trp Tyr Gln Gln Lys Pro Gly Lys Ala
    50                  55                  60

Pro Lys Leu Leu Ile Tyr Asp Ala Ser Ser Leu Glu Ser Gly Val Pro
65                  70                  75                  80

Ser Arg Phe Ser Gly Ser Gly Ser Gly Thr Asp Phe Thr Leu Thr Ile
                85                  90                  95

Ser Ser Leu Gln Pro Glu Asp Phe Ala Thr Tyr Tyr Cys Gln Gln Phe
            100                 105                 110

Asn Ser Tyr Pro Tyr Thr Phe Gly Gln Gly Thr Lys Leu Glu Ile Lys
        115                 120                 125

Gly Gly Gly Gly Ser Gly Gly Gly Gly Ser Gly Gly Gly Gly Ser Glu
    130                 135                 140

Val Gln Leu Val Gln Ser Gly Ala Glu Val Lys Lys Pro Gly Glu Ser
145                 150                 155                 160

Leu Lys Ile Ser Cys Lys Gly Ser Gly Tyr Ser Phe Ser Ser Ser Trp
                165                 170                 175

Ile Gly Trp Val Arg Gln Met Pro Gly Lys Gly Leu Glu Trp Met Gly
            180                 185                 190

Ile Ile Tyr Pro Asp Asp Ser Asp Thr Arg Tyr Ser Pro Ser Phe Gln
        195                 200                 205

Gly Gln Val Thr Ile Ser Ala Asp Lys Ser Ile Arg Thr Ala Tyr Leu
    210                 215                 220

Gln Trp Ser Ser Leu Lys Ala Ser Asp Thr Ala Met Tyr Tyr Cys Ala
225                 230                 235                 240

Arg His Val Thr Met Ile Trp Gly Val Ile Ile Asp Phe Trp Gly Gln
                245                 250                 255

Gly Thr Leu Val Thr Val Ser Ser Phe Val Pro Val Phe Leu Pro Ala
            260                 265                 270

Lys Pro Thr Thr Thr Pro Ala Pro Arg Pro Pro Thr Pro Ala Pro Thr
        275                 280                 285

Ile Ala Ser Gln Pro Leu Ser Leu Arg Pro Glu Ala Cys Arg Pro Ala
    290                 295                 300

Ala Gly Gly Ala Val His Thr Arg Gly Leu Asp Phe Ala Cys Asp Ile
305                 310                 315                 320

Tyr Ile Trp Ala Pro Leu Ala Gly Thr Cys Gly Val Leu Leu Leu Ser
                325                 330                 335

Leu Val Ile Thr Leu Tyr Cys Asn His Arg Asn Arg Phe Ser Val Val
            340                 345                 350

Lys Arg Gly Arg Lys Lys Leu Leu Tyr Ile Phe Lys Gln Pro Phe Met
        355                 360                 365

Arg Pro Val Gln Thr Thr Gln Glu Glu Asp Gly Cys Ser Cys Arg Phe
    370                 375                 380

Pro Glu Glu Glu Glu Gly Gly Cys Glu Leu Arg Val Lys Phe Ser Arg
385                 390                 395                 400

Ser Ala Asp Ala Pro Ala Tyr Gln Gln Gly Gln Asn Gln Leu Tyr Asn
                405                 410                 415

Glu Leu Asn Leu Gly Arg Arg Glu Glu Tyr Asp Val Leu Asp Lys Arg
            420                 425                 430

Arg Gly Arg Asp Pro Glu Met Gly Gly Lys Pro Arg Arg Lys Asn Pro
        435                 440                 445

Gln Glu Gly Leu Tyr Asn Glu Leu Gln Lys Asp Lys Met Ala Glu Ala
    450                 455                 460

Tyr Ser Glu Ile Gly Met Lys Gly Glu Arg Arg Arg Gly Lys Gly His
465                 470                 475                 480

Asp Gly Leu Tyr Gln Gly Leu Ser Thr Ala Thr Lys Asp Thr Tyr Asp
                485                 490                 495

Ala Leu His Met Gln Ala Leu Pro Pro Arg
            500                 505

<210>  52
<211>  893
<212>  PRT
<213>  Artificial Sequence

<220>
<223>  21D4 CAR-2A-TMEM59

<400>  52

Met Ala Leu Pro Val Thr Ala Leu Leu Leu Pro Leu Ala Leu Leu Leu
1               5                   10                  15

His Ala Ala Arg Pro Ala Ile Gln Leu Thr Gln Ser Pro Ser Ser Leu
            20                  25                  30

Ser Ala Ser Val Gly Asp Arg Val Thr Ile Thr Cys Arg Ala Ser Gln
        35                  40                  45

Gly Ile Ser Ser Ala Leu Ala Trp Tyr Gln Gln Lys Pro Gly Lys Ala
    50                  55                  60

Pro Lys Leu Leu Ile Tyr Asp Ala Ser Ser Leu Glu Ser Gly Val Pro
65                  70                  75                  80

Ser Arg Phe Ser Gly Ser Gly Ser Gly Thr Asp Phe Thr Leu Thr Ile
                85                  90                  95

Ser Ser Leu Gln Pro Glu Asp Phe Ala Thr Tyr Tyr Cys Gln Gln Phe
            100                 105                 110

Asn Ser Tyr Pro Tyr Thr Phe Gly Gln Gly Thr Lys Leu Glu Ile Lys
        115                 120                 125

Gly Gly Gly Gly Ser Gly Gly Gly Gly Ser Gly Gly Gly Gly Ser Glu
    130                 135                 140

Val Gln Leu Val Gln Ser Gly Ala Glu Val Lys Lys Pro Gly Glu Ser
145                 150                 155                 160

Leu Lys Ile Ser Cys Lys Gly Ser Gly Tyr Ser Phe Ser Ser Ser Trp
                165                 170                 175

Ile Gly Trp Val Arg Gln Met Pro Gly Lys Gly Leu Glu Trp Met Gly
            180                 185                 190

Ile Ile Tyr Pro Asp Asp Ser Asp Thr Arg Tyr Ser Pro Ser Phe Gln
        195                 200                 205

Gly Gln Val Thr Ile Ser Ala Asp Lys Ser Ile Arg Thr Ala Tyr Leu
    210                 215                 220

Gln Trp Ser Ser Leu Lys Ala Ser Asp Thr Ala Met Tyr Tyr Cys Ala
225                 230                 235                 240

Arg His Val Thr Met Ile Trp Gly Val Ile Ile Asp Phe Trp Gly Gln
                245                 250                 255

Gly Thr Leu Val Thr Val Ser Ser Phe Val Pro Val Phe Leu Pro Ala
            260                 265                 270

Lys Pro Thr Thr Thr Pro Ala Pro Arg Pro Pro Thr Pro Ala Pro Thr
        275                 280                 285

Ile Ala Ser Gln Pro Leu Ser Leu Arg Pro Glu Ala Cys Arg Pro Ala
    290                 295                 300

Ala Gly Gly Ala Val His Thr Arg Gly Leu Asp Phe Ala Cys Asp Ile
305                 310                 315                 320

Tyr Ile Trp Ala Pro Leu Ala Gly Thr Cys Gly Val Leu Leu Leu Ser
                325                 330                 335

Leu Val Ile Thr Leu Tyr Cys Asn His Arg Asn Arg Ser Lys Arg Ser
        340                 345                 350

Arg Leu Leu His Ser Asp Tyr Met Asn Met Thr Pro Arg Arg Pro Gly
    355                 360                 365

Pro Thr Arg Lys His Tyr Gln Pro Tyr Ala Pro Pro Arg Asp Phe Ala
    370                 375                 380

Ala Tyr Arg Ser Arg Phe Ser Val Val Lys Arg Gly Arg Lys Lys Leu
385                 390                 395                 400

Leu Tyr Ile Phe Lys Gln Pro Phe Met Arg Pro Val Gln Thr Thr Gln
                405                 410                 415

Glu Glu Asp Gly Cys Ser Cys Arg Phe Pro Glu Glu Glu Glu Gly Gly
            420                 425                 430

Cys Glu Leu Arg Val Lys Phe Ser Arg Ser Ala Asp Ala Pro Ala Tyr
        435                 440                 445

Gln Gln Gly Gln Asn Gln Leu Tyr Asn Glu Leu Asn Leu Gly Arg Arg
    450                 455                 460

Glu Glu Tyr Asp Val Leu Asp Lys Arg Arg Gly Arg Asp Pro Glu Met
465                 470                 475                 480

Gly Gly Lys Pro Arg Arg Lys Asn Pro Gln Glu Gly Leu Tyr Asn Glu
                485                 490                 495

Leu Gln Lys Asp Lys Met Ala Glu Ala Tyr Ser Glu Ile Gly Met Lys
            500                 505                 510

Gly Glu Arg Arg Arg Gly Lys Gly His Asp Gly Leu Tyr Gln Gly Leu
        515                 520                 525

Ser Thr Ala Thr Lys Asp Thr Tyr Asp Ala Leu His Met Gln Ala Leu
    530                 535                 540

Pro Pro Arg Gly Ser Gly Ala Thr Asn Phe Ser Leu Leu Lys Gln Ala
545                 550                 555                 560

Gly Asp Val Glu Glu Asn Pro Gly Pro Met Ala Ala Pro Lys Gly Ser
                565                 570                 575

Leu Trp Val Arg Thr Gln Leu Gly Leu Pro Pro Leu Leu Leu Leu Thr

            580                 585                 590

Met Ala Leu Ala Gly Gly Ser Gly Thr Ala Ser Ala Glu Ala Phe Asp
        595                 600                 605

Ser Val Leu Gly Asp Thr Ala Ser Cys His Arg Ala Cys Gln Leu Thr
    610                 615                 620

Tyr Pro Leu His Thr Tyr Pro Lys Glu Glu Glu Leu Tyr Ala Cys Gln
625                 630                 635                 640

Arg Gly Cys Arg Leu Phe Ser Ile Cys Gln Phe Val Asp Asp Gly Ile
                645                 650                 655

Asp Leu Asn Arg Thr Lys Leu Glu Cys Glu Ser Ala Cys Thr Glu Ala
            660                 665                 670

Tyr Ser Gln Ser Asp Glu Gln Tyr Ala Cys His Leu Gly Cys Gln Asn
        675                 680                 685

Gln Leu Pro Phe Ala Glu Leu Arg Gln Glu Gln Leu Met Ser Leu Met
    690                 695                 700

Pro Lys Met His Leu Leu Phe Pro Leu Thr Leu Val Arg Ser Phe Trp
705                 710                 715                 720

Ser Asp Met Met Asp Ser Ala Gln Ser Phe Ile Thr Ser Ser Trp Thr
                725                 730                 735

Phe Tyr Leu Gln Ala Asp Asp Gly Lys Ile Val Ile Phe Gln Ser Lys
            740                 745                 750

Pro Glu Ile Gln Tyr Ala Pro His Leu Glu Gln Glu Pro Thr Asn Leu
        755                 760                 765

Arg Glu Ser Ser Leu Ser Lys Met Ser Ser Asp Leu Gln Met Arg Asn
    770                 775                 780

Ser Gln Ala His Arg Asn Phe Leu Glu Asp Gly Glu Ser Asp Gly Phe
785                 790                 795                 800

Leu Arg Cys Leu Ser Leu Asn Ser Gly Trp Ile Leu Thr Thr Thr Leu
                805                 810                 815

Val Leu Ser Val Met Val Leu Leu Trp Ile Cys Cys Ala Thr Val Ala
            820                 825                 830

Thr Ala Val Glu Gln Tyr Val Pro Ser Glu Lys Leu Ser Ile Tyr Gly
        835                 840                 845

Asp Leu Glu Phe Met Asn Glu Gln Lys Leu Asn Arg Tyr Pro Ala Ser
    850                 855                 860

Ser Leu Val Val Val Arg Ser Lys Thr Glu Asp His Glu Glu Ala Gly
865                 870                 875                 880

Pro Leu Pro Thr Lys Val Asn Leu Ala His Ser Glu Ile
                885                 890

<210>  53
<211>  767
<212>  PRT
<213>  Artificial Sequence

<220>
<223>  21D4 CAR-2A-GFP

<400>  53

Met Ala Leu Pro Val Thr Ala Leu Leu Leu Pro Leu Ala Leu Leu Leu
1               5                   10                  15

His Ala Ala Arg Pro Ala Ile Gln Leu Thr Gln Ser Pro Ser Ser Leu
            20                  25                  30

Ser Ala Ser Val Gly Asp Arg Val Thr Ile Thr Cys Arg Ala Ser Gln
        35                  40                  45

Gly Ile Ser Ser Ala Leu Ala Trp Tyr Gln Gln Lys Pro Gly Lys Ala
    50                  55                  60

Pro Lys Leu Leu Ile Tyr Asp Ala Ser Ser Leu Glu Ser Gly Val Pro
65                  70                  75                  80

Ser Arg Phe Ser Gly Ser Gly Ser Gly Thr Asp Phe Thr Leu Thr Ile
                85                  90                  95

Ser Ser Leu Gln Pro Glu Asp Phe Ala Thr Tyr Tyr Cys Gln Gln Phe
            100                 105                 110

Asn Ser Tyr Pro Tyr Thr Phe Gly Gln Gly Thr Lys Leu Glu Ile Lys
        115                 120                 125

Gly Gly Gly Gly Ser Gly Gly Gly Gly Ser Gly Gly Gly Gly Ser Glu
    130                 135                 140

Val Gln Leu Val Gln Ser Gly Ala Glu Val Lys Lys Pro Gly Glu Ser

145                 150                 155                 160

Leu Lys Ile Ser Cys Lys Gly Ser Gly Tyr Ser Phe Ser Ser Ser Trp
                165                 170                 175

Ile Gly Trp Val Arg Gln Met Pro Gly Lys Gly Leu Glu Trp Met Gly
            180                 185                 190

Ile Ile Tyr Pro Asp Asp Ser Asp Thr Arg Tyr Ser Pro Ser Phe Gln
        195                 200                 205

Gly Gln Val Thr Ile Ser Ala Asp Lys Ser Ile Arg Thr Ala Tyr Leu
    210                 215                 220

Gln Trp Ser Ser Leu Lys Ala Ser Asp Thr Ala Met Tyr Tyr Cys Ala
225                 230                 235                 240

Arg His Val Thr Met Ile Trp Gly Val Ile Ile Asp Phe Trp Gly Gln
                245                 250                 255

Gly Thr Leu Val Thr Val Ser Ser Phe Val Pro Val Phe Leu Pro Ala
            260                 265                 270

Lys Pro Thr Thr Thr Pro Ala Pro Arg Pro Pro Thr Pro Ala Pro Thr
        275                 280                 285

Ile Ala Ser Gln Pro Leu Ser Leu Arg Pro Glu Ala Cys Arg Pro Ala
    290                 295                 300

Ala Gly Gly Ala Val His Thr Arg Gly Leu Asp Phe Ala Cys Asp Ile
305                 310                 315                 320

Tyr Ile Trp Ala Pro Leu Ala Gly Thr Cys Gly Val Leu Leu Leu Ser
                325                 330                 335

Leu Val Ile Thr Leu Tyr Cys Asn His Arg Asn Arg Phe Ser Val Val
            340                 345                 350

Lys Arg Gly Arg Lys Lys Leu Leu Tyr Ile Phe Lys Gln Pro Phe Met
        355                 360                 365

Arg Pro Val Gln Thr Thr Gln Glu Glu Asp Gly Cys Ser Cys Arg Phe
    370                 375                 380

Pro Glu Glu Glu Glu Gly Gly Cys Glu Leu Arg Val Lys Phe Ser Arg
385                 390                 395                 400

Ser Ala Asp Ala Pro Ala Tyr Gln Gln Gly Gln Asn Gln Leu Tyr Asn
                405                 410                 415

Glu Leu Asn Leu Gly Arg Arg Glu Glu Tyr Asp Val Leu Asp Lys Arg
            420                 425                 430

Arg Gly Arg Asp Pro Glu Met Gly Gly Lys Pro Arg Arg Lys Asn Pro
        435                 440                 445

Gln Glu Gly Leu Tyr Asn Glu Leu Gln Lys Asp Lys Met Ala Glu Ala
    450                 455                 460

Tyr Ser Glu Ile Gly Met Lys Gly Glu Arg Arg Arg Gly Lys Gly His
465                 470                 475                 480

Asp Gly Leu Tyr Gln Gly Leu Ser Thr Ala Thr Lys Asp Thr Tyr Asp
                485                 490                 495

Ala Leu His Met Gln Ala Leu Pro Pro Arg Gly Ser Gly Ala Thr Asn
            500                 505                 510

Phe Ser Leu Leu Lys Gln Ala Gly Asp Val Glu Glu Asn Pro Gly Pro
        515                 520                 525

Met Val Ser Lys Gly Glu Glu Leu Phe Thr Gly Val Val Pro Ile Leu
    530                 535                 540

Val Glu Leu Asp Gly Asp Val Asn Gly His Lys Phe Ser Val Ser Gly
545                 550                 555                 560

Glu Gly Glu Gly Asp Ala Thr Tyr Gly Lys Leu Thr Leu Lys Phe Ile
                565                 570                 575

Cys Thr Thr Gly Lys Leu Pro Val Pro Trp Pro Thr Leu Val Thr Thr
            580                 585                 590

Leu Thr Tyr Gly Val Gln Cys Phe Ser Arg Tyr Pro Asp His Met Lys
        595                 600                 605

Gln His Asp Phe Phe Lys Ser Ala Met Pro Glu Gly Tyr Val Gln Glu
    610                 615                 620

Arg Thr Ile Phe Phe Lys Asp Asp Gly Asn Tyr Lys Thr Arg Ala Glu
625                 630                 635                 640

Val Lys Phe Glu Gly Asp Thr Leu Val Asn Arg Ile Glu Leu Lys Gly
                645                 650                 655

Ile Asp Phe Lys Glu Asp Gly Asn Ile Leu Gly His Lys Leu Glu Tyr
            660                 665                 670

Asn Tyr Asn Ser His Asn Val Tyr Ile Met Ala Asp Lys Gln Lys Asn
        675                 680                 685

Gly Ile Lys Val Asn Phe Lys Ile Arg His Asn Ile Glu Asp Gly Ser
    690                 695                 700

Val Gln Leu Ala Asp His Tyr Gln Gln Asn Thr Pro Ile Gly Asp Gly
705                 710                 715                 720

Pro Val Leu Leu Pro Asp Asn His Tyr Leu Ser Thr Gln Ser Ala Leu
                725                 730                 735

Ser Lys Asp Pro Asn Glu Lys Arg Asp His Met Val Leu Leu Glu Phe
            740                 745                 750

Val Thr Ala Ala Gly Ile Thr Leu Gly Met Asp Glu Leu Tyr Lys
        755                 760                 765

<210>  54
<211>  263
<212>  PRT
<213>  Artificial Sequence

<220>
<223>  GPC3 targeting moiety

<400>  54

Met Ala Leu Pro Val Thr Ala Leu Leu Leu Pro Leu Ala Leu Leu Leu
1               5                   10                  15

His Ala Ala Arg Pro Asp Val Val Met Thr Gln Ser Pro Leu Ser Leu
            20                  25                  30

Pro Val Thr Pro Gly Glu Pro Ala Ser Ile Ser Cys Arg Ser Ser Gln
        35                  40                  45

Ser Leu Val His Ser Asn Gly Asn Thr Tyr Leu His Trp Tyr Leu Gln
    50                  55                  60

Lys Pro Gly Gln Ser Pro Gln Leu Leu Ile Tyr Lys Val Ser Asn Arg
65                  70                  75                  80

Phe Ser Gly Val Pro Asp Arg Phe Ser Gly Ser Gly Ser Gly Thr Asp
                85                  90                  95

Phe Thr Leu Lys Ile Ser Arg Val Glu Ala Glu Asp Val Gly Val Tyr
            100                 105                 110

Tyr Cys Ser Gln Asn Thr His Val Pro Pro Thr Phe Gly Gln Gly Thr
        115                 120                 125

Lys Leu Glu Ile Lys Gly Gly Gly Gly Ser Gly Gly Gly Gly Ser Gly
    130                 135                 140

Gly Gly Gly Ser Gln Val Gln Leu Val Gln Ser Gly Ala Glu Val Lys
145                 150                 155                 160

Lys Pro Gly Ala Ser Val Lys Val Ser Cys Lys Ala Ser Gly Tyr Thr
                165                 170                 175

Phe Thr Asp Tyr Glu Met His Trp Val Arg Gln Ala Pro Gly Gln Gly
            180                 185                 190

Leu Glu Trp Met Gly Ala Leu Asp Pro Lys Thr Gly Asp Thr Ala Tyr
        195                 200                 205

Ser Gln Lys Phe Lys Gly Arg Val Thr Leu Thr Ala Asp Glu Ser Thr
    210                 215                 220

Ser Thr Ala Tyr Met Glu Leu Ser Ser Leu Arg Ser Glu Asp Thr Ala
225                 230                 235                 240

Val Tyr Tyr Cys Thr Arg Phe Tyr Ser Tyr Thr Tyr Trp Gly Gln Gly
                245                 250                 255

Thr Leu Val Thr Val Ser Ser
            260

<210>  55
<211>  505
<212>  PRT
<213>  Artificial Sequence

<220>
<223>  GPC3 CAR

<400>  55

Met Ala Leu Pro Val Thr Ala Leu Leu Leu Pro Leu Ala Leu Leu Leu
1               5                   10                  15

His Ala Ala Arg Pro Asp Val Val Met Thr Gln Ser Pro Leu Ser Leu
            20                  25                  30

Pro Val Thr Pro Gly Glu Pro Ala Ser Ile Ser Cys Arg Ser Ser Gln

35                  40                  45

Ser Leu Val His Ser Asn Gly Asn Thr Tyr Leu His Trp Tyr Leu Gln
    50                  55                  60

Lys Pro Gly Gln Ser Pro Gln Leu Leu Ile Tyr Lys Val Ser Asn Arg
65                  70                  75                  80

Phe Ser Gly Val Pro Asp Arg Phe Ser Gly Ser Gly Ser Gly Thr Asp
                85                  90                  95

Phe Thr Leu Lys Ile Ser Arg Val Glu Ala Glu Asp Val Gly Val Tyr
            100                 105                 110

Tyr Cys Ser Gln Asn Thr His Val Pro Pro Thr Phe Gly Gln Gly Thr
        115                 120                 125

Lys Leu Glu Ile Lys Gly Gly Gly Gly Ser Gly Gly Gly Gly Ser Gly
    130                 135                 140

Gly Gly Gly Ser Gln Val Gln Leu Val Gln Ser Gly Ala Glu Val Lys
145                 150                 155                 160

Lys Pro Gly Ala Ser Val Lys Val Ser Cys Lys Ala Ser Gly Tyr Thr
                165                 170                 175

Phe Thr Asp Tyr Glu Met His Trp Val Arg Gln Ala Pro Gly Gln Gly
            180                 185                 190

Leu Glu Trp Met Gly Ala Leu Asp Pro Lys Thr Gly Asp Thr Ala Tyr
        195                 200                 205

Ser Gln Lys Phe Lys Gly Arg Val Thr Leu Thr Ala Asp Glu Ser Thr
    210                 215                 220

Ser Thr Ala Tyr Met Glu Leu Ser Ser Leu Arg Ser Glu Asp Thr Ala
225                 230                 235                 240

Val Tyr Tyr Cys Thr Arg Phe Tyr Ser Tyr Thr Tyr Trp Gly Gln Gly
                245                 250                 255

Thr Leu Val Thr Val Ser Ser Phe Val Pro Val Phe Leu Pro Ala Lys
            260                 265                 270

Pro Thr Thr Thr Pro Ala Pro Arg Pro Pro Thr Pro Ala Pro Thr Ile
        275                 280                 285

Ala Ser Gln Pro Leu Ser Leu Arg Pro Glu Ala Cys Arg Pro Ala Ala
    290                 295                 300

Gly Gly Ala Val His Thr Arg Gly Leu Asp Phe Ala Cys Asp Ile Tyr
305                 310                 315                 320

Ile Trp Ala Pro Leu Ala Gly Thr Cys Gly Val Leu Leu Leu Ser Leu
                325                 330                 335

Val Ile Thr Leu Tyr Cys Asn His Arg Asn Arg Phe Ser Val Val Lys
            340                 345                 350

Arg Gly Arg Lys Lys Leu Leu Tyr Ile Phe Lys Gln Pro Phe Met Arg
        355                 360                 365

Pro Val Gln Thr Thr Gln Glu Glu Asp Gly Cys Ser Cys Arg Phe Pro
    370                 375                 380

Glu Glu Glu Glu Gly Gly Cys Glu Leu Arg Val Lys Phe Ser Arg Ser
385                 390                 395                 400

Ala Asp Ala Pro Ala Tyr Gln Gln Gly Gln Asn Gln Leu Tyr Asn Glu
                405                 410                 415

Leu Asn Leu Gly Arg Arg Glu Glu Tyr Asp Val Leu Asp Lys Arg Arg
            420                 425                 430

Gly Arg Asp Pro Glu Met Gly Gly Lys Pro Arg Arg Lys Asn Pro Gln
        435                 440                 445

Glu Gly Leu Tyr Asn Glu Leu Gln Lys Asp Lys Met Ala Glu Ala Tyr
    450                 455                 460

Ser Glu Ile Gly Met Lys Gly Glu Arg Arg Arg Gly Lys Gly His Asp
465                 470                 475                 480

Gly Leu Tyr Gln Gly Leu Ser Thr Ala Thr Lys Asp Thr Tyr Asp Ala
                485                 490                 495

Leu His Met Gln Ala Leu Pro Pro Arg
            500                 505

<210>  56
<211>  733
<212>  PRT
<213>  Artificial Sequence

<220>
<223>  CD19CD22 CAR

<400>  56

Met Leu Leu Leu Val Thr Ser Leu Leu Leu Cys Glu Leu Pro His Pro
1               5                   10                  15

Ala Phe Leu Leu Ile Pro Asp Ile Gln Met Thr Gln Thr Thr Ser Ser
            20                  25                  30

Leu Ser Ala Ser Leu Gly Asp Arg Val Thr Ile Ser Cys Arg Ala Ser
        35                  40                  45

Gln Asp Ile Ser Lys Tyr Leu Asn Trp Tyr Gln Gln Lys Pro Asp Gly
    50                  55                  60

Thr Val Lys Leu Leu Ile Tyr His Thr Ser Arg Leu His Ser Gly Val
65                  70                  75                  80

Pro Ser Arg Phe Ser Gly Ser Gly Ser Gly Thr Asp Tyr Ser Leu Thr
                85                  90                  95

Ile Ser Asn Leu Glu Gln Glu Asp Ile Ala Thr Tyr Phe Cys Gln Gln
            100                 105                 110

Gly Asn Thr Leu Pro Tyr Thr Phe Gly Gly Gly Thr Lys Leu Glu Ile
        115                 120                 125

Thr Gly Gly Gly Gly Ser Gln Val Gln Leu Gln Gln Ser Gly Pro Gly
    130                 135                 140

Leu Val Lys Pro Ser Gln Thr Leu Ser Leu Thr Cys Ala Ile Ser Gly
145                 150                 155                 160

Asp Ser Val Ser Ser Asn Ser Ala Ala Trp Asn Trp Ile Arg Gln Ser
                165                 170                 175

Pro Ser Arg Gly Leu Glu Trp Leu Gly Arg Thr Tyr Tyr Arg Ser Lys
            180                 185                 190

Trp Tyr Asn Asp Tyr Ala Val Ser Val Lys Ser Arg Ile Thr Ile Asn
        195                 200                 205

Pro Asp Thr Ser Lys Asn Gln Phe Ser Leu Gln Leu Asn Ser Val Thr
    210                 215                 220

Pro Glu Asp Thr Ala Val Tyr Tyr Cys Ala Arg Glu Val Thr Gly Asp
225                 230                 235                 240

Leu Glu Asp Ala Phe Asp Ile Trp Gly Gln Gly Thr Met Val Thr Val
                245                 250                 255

Ser Ser Gly Ser Thr Ser Gly Ser Gly Lys Pro Gly Ser Gly Glu Gly
            260                 265                 270

Ser Thr Lys Gly Asp Ile Gln Met Thr Gln Ser Pro Ser Ser Leu Ser
        275                 280                 285

Ala Ser Val Gly Asp Arg Val Thr Ile Thr Cys Arg Ala Ser Gln Thr
    290                 295                 300

Ile Trp Ser Tyr Leu Asn Trp Tyr Gln Gln Arg Pro Gly Lys Ala Pro
305                 310                 315                 320

Asn Leu Leu Ile Tyr Ala Ala Ser Ser Leu Gln Ser Gly Val Pro Ser
                325                 330                 335

Arg Phe Ser Gly Arg Gly Ser Gly Thr Asp Phe Thr Leu Thr Ile Ser
            340                 345                 350

Ser Leu Gln Ala Glu Asp Phe Ala Thr Tyr Tyr Cys Gln Gln Ser Tyr
        355                 360                 365

Ser Ile Pro Gln Thr Phe Gly Gln Gly Thr Lys Leu Glu Ile Lys Gly
    370                 375                 380

Gly Gly Gly Ser Glu Val Lys Leu Gln Glu Ser Gly Pro Gly Leu Val
385                 390                 395                 400

Ala Pro Ser Gln Ser Leu Ser Val Thr Cys Thr Val Ser Gly Val Ser
                405                 410                 415

Leu Pro Asp Tyr Gly Val Ser Trp Ile Arg Gln Pro Pro Arg Lys Gly
            420                 425                 430

Leu Glu Trp Leu Gly Val Ile Trp Gly Ser Glu Thr Thr Tyr Tyr Asn
        435                 440                 445

Ser Ala Leu Lys Ser Arg Leu Thr Ile Ile Lys Asp Asn Ser Lys Ser
    450                 455                 460

Gln Val Phe Leu Lys Met Asn Ser Leu Gln Thr Asp Asp Thr Ala Ile
465                 470                 475                 480

Tyr Tyr Cys Ala Lys His Tyr Tyr Tyr Gly Gly Ser Tyr Ala Met Asp
                485                 490                 495

Tyr Trp Gly Gln Gly Thr Ser Val Thr Val Ser Ser Ser Gly Thr Thr
        500                 505                 510

Thr Pro Ala Pro Arg Pro Pro Thr Pro Ala Pro Thr Ile Ala Ser Gln
    515                 520                 525

Pro Leu Ser Leu Arg Pro Glu Ala Cys Arg Pro Ala Ala Gly Gly Ala
    530                 535                 540

Val His Thr Arg Gly Leu Asp Phe Ala Cys Asp Ile Tyr Ile Trp Ala
545                 550                 555                 560

Pro Leu Ala Gly Thr Cys Gly Val Leu Leu Leu Ser Leu Val Ile Thr
                565                 570                 575

Leu Tyr Cys Lys Arg Gly Arg Lys Lys Leu Leu Tyr Ile Phe Lys Gln
            580                 585                 590

Pro Phe Met Arg Pro Val Gln Thr Thr Gln Glu Glu Asp Gly Cys Ser
        595                 600                 605

Cys Arg Phe Pro Glu Glu Glu Glu Gly Gly Cys Glu Leu Arg Val Lys
    610                 615                 620

Phe Ser Arg Ser Ala Asp Ala Pro Ala Tyr Lys Gln Gly Gln Asn Gln
625                 630                 635                 640

Leu Tyr Asn Glu Leu Asn Leu Gly Arg Arg Glu Glu Tyr Asp Val Leu
                645                 650                 655

Asp Lys Arg Arg Gly Arg Asp Pro Glu Met Gly Gly Lys Pro Arg Arg
            660                 665                 670

Lys Asn Pro Gln Glu Gly Leu Tyr Asn Glu Leu Gln Lys Asp Lys Met
        675                 680                 685

Ala Glu Ala Tyr Ser Glu Ile Gly Met Lys Gly Glu Arg Arg Arg Gly
    690                 695                 700

Lys Gly His Asp Gly Leu Tyr Gln Gly Leu Ser Thr Ala Thr Lys Asp
705                 710                 715                 720

Thr Tyr Asp Ala Leu His Met Gln Ala Leu Pro Pro Arg
                725                 730

<210>  57
<211>  733

<212>  PRT
<213>  Artificial Sequence

<220>
<223>  CD19CD22-59TM CAR

<400>  57

Met Leu Leu Leu Val Thr Ser Leu Leu Leu Cys Glu Leu Pro His Pro
1               5                   10                  15

Ala Phe Leu Leu Ile Pro Asp Ile Gln Met Thr Gln Thr Thr Ser Ser
            20                  25                  30

Leu Ser Ala Ser Leu Gly Asp Arg Val Thr Ile Ser Cys Arg Ala Ser
        35                  40                  45

Gln Asp Ile Ser Lys Tyr Leu Asn Trp Tyr Gln Gln Lys Pro Asp Gly
    50                  55                  60

Thr Val Lys Leu Leu Ile Tyr His Thr Ser Arg Leu His Ser Gly Val
65                  70                  75                  80

Pro Ser Arg Phe Ser Gly Ser Gly Ser Gly Thr Asp Tyr Ser Leu Thr
                85                  90                  95

Ile Ser Asn Leu Glu Gln Glu Asp Ile Ala Thr Tyr Phe Cys Gln Gln
            100                 105                 110

Gly Asn Thr Leu Pro Tyr Thr Phe Gly Gly Gly Thr Lys Leu Glu Ile
        115                 120                 125

Thr Gly Gly Gly Gly Ser Gln Val Gln Leu Gln Gln Ser Gly Pro Gly
    130                 135                 140

Leu Val Lys Pro Ser Gln Thr Leu Ser Leu Thr Cys Ala Ile Ser Gly
145                 150                 155                 160

Asp Ser Val Ser Ser Asn Ser Ala Ala Trp Asn Trp Ile Arg Gln Ser
                165                 170                 175

Pro Ser Arg Gly Leu Glu Trp Leu Gly Arg Thr Tyr Tyr Arg Ser Lys
            180                 185                 190

Trp Tyr Asn Asp Tyr Ala Val Ser Val Lys Ser Arg Ile Thr Ile Asn
        195                 200                 205

Pro Asp Thr Ser Lys Asn Gln Phe Ser Leu Gln Leu Asn Ser Val Thr
    210                 215                 220

Pro Glu Asp Thr Ala Val Tyr Tyr Cys Ala Arg Glu Val Thr Gly Asp
225                 230                 235                 240

Leu Glu Asp Ala Phe Asp Ile Trp Gly Gln Gly Thr Met Val Thr Val
                245                 250                 255

Ser Ser Gly Ser Thr Ser Gly Ser Gly Lys Pro Gly Ser Gly Glu Gly
            260                 265                 270

Ser Thr Lys Gly Asp Ile Gln Met Thr Gln Ser Pro Ser Ser Leu Ser
        275                 280                 285

Ala Ser Val Gly Asp Arg Val Thr Ile Thr Cys Arg Ala Ser Gln Thr
    290                 295                 300

Ile Trp Ser Tyr Leu Asn Trp Tyr Gln Gln Arg Pro Gly Lys Ala Pro
305                 310                 315                 320

Asn Leu Leu Ile Tyr Ala Ala Ser Ser Leu Gln Ser Gly Val Pro Ser
                325                 330                 335

Arg Phe Ser Gly Arg Gly Ser Gly Thr Asp Phe Thr Leu Thr Ile Ser
            340                 345                 350

Ser Leu Gln Ala Glu Asp Phe Ala Thr Tyr Tyr Cys Gln Gln Ser Tyr
        355                 360                 365

Ser Ile Pro Gln Thr Phe Gly Gln Gly Thr Lys Leu Glu Ile Lys Gly
    370                 375                 380

Gly Gly Gly Ser Glu Val Lys Leu Gln Glu Ser Gly Pro Gly Leu Val
385                 390                 395                 400

Ala Pro Ser Gln Ser Leu Ser Val Thr Cys Thr Val Ser Gly Val Ser
                405                 410                 415

Leu Pro Asp Tyr Gly Val Ser Trp Ile Arg Gln Pro Pro Arg Lys Gly
            420                 425                 430

Leu Glu Trp Leu Gly Val Ile Trp Gly Ser Glu Thr Thr Tyr Tyr Asn
        435                 440                 445

Ser Ala Leu Lys Ser Arg Leu Thr Ile Ile Lys Asp Asn Ser Lys Ser
    450                 455                 460

Gln Val Phe Leu Lys Met Asn Ser Leu Gln Thr Asp Asp Thr Ala Ile
465                 470                 475                 480

Tyr Tyr Cys Ala Lys His Tyr Tyr Tyr Gly Gly Ser Tyr Ala Met Asp
                485                 490                 495

Tyr Trp Gly Gln Gly Thr Ser Val Thr Val Ser Ser Pro His Leu Glu
            500                 505                 510

Gln Glu Pro Thr Asn Leu Arg Glu Ser Ser Leu Ser Lys Met Ser Ser
        515                 520                 525

Asp Leu Gln Met Arg Asn Ser Gln Ala His Arg Asn Phe Leu Glu Asp
    530                 535                 540

Gly Glu Ser Asp Gly Phe Leu Arg Cys Leu Ser Leu Asn Ser Gly Trp
545                 550                 555                 560

Ile Leu Thr Thr Thr Leu Val Leu Ser Val Met Val Leu Leu Trp Ile
                565                 570                 575

Cys Cys Ala Lys Arg Gly Arg Lys Lys Leu Leu Tyr Ile Phe Lys Gln
            580                 585                 590

Pro Phe Met Arg Pro Val Gln Thr Thr Gln Glu Glu Asp Gly Cys Ser
        595                 600                 605

Cys Arg Phe Pro Glu Glu Glu Glu Gly Gly Cys Glu Leu Arg Val Lys
    610                 615                 620

Phe Ser Arg Ser Ala Asp Ala Pro Ala Tyr Lys Gln Gly Gln Asn Gln
625                 630                 635                 640

Leu Tyr Asn Glu Leu Asn Leu Gly Arg Arg Glu Glu Tyr Asp Val Leu
                645                 650                 655

Asp Lys Arg Arg Gly Arg Asp Pro Glu Met Gly Gly Lys Pro Arg Arg
            660                 665                 670

Lys Asn Pro Gln Glu Gly Leu Tyr Asn Glu Leu Gln Lys Asp Lys Met
        675                 680                 685

Ala Glu Ala Tyr Ser Glu Ile Gly Met Lys Gly Glu Arg Arg Arg Gly
    690                 695                 700

Lys Gly His Asp Gly Leu Tyr Gln Gly Leu Ser Thr Ala Thr Lys Asp
705                 710                 715                 720

Thr Tyr Asp Ala Leu His Met Gln Ala Leu Pro Pro Arg

725                                        730